(19) Europäisches Patentamt
European Patent Office
Office européen des brevets

(11) EP 0 888 292 B1

(12) # FASCICULE DE BREVET EUROPEEN

(45) Date de publication et mention de la délivrance du brevet:
**31.10.2001 Bulletin 2001/44**

(21) Numéro de dépôt: **97915519.9**

(22) Date de dépôt: **20.03.1997**

(51) Int Cl.[7]: **C07C 281/12**, C07D 233/52, C07C 337/08, C07D 239/18, C07D 317/70, A61K 31/19, A61K 31/415, A61K 31/505, A61K 31/36

(86) Numéro de dépôt international:
**PCT/FR97/00487**

(87) Numéro de publication internationale:
**WO 97/34865 (25.09.1997 Gazette 1997/41)**

(54) **COMPOSES TRICYCLIQUES AYANT UNE ACTIVITE VIS-A-VIS DES INTEGRINES NOTAMMENT VIS-A-VIS DE L'INTEGRINE ALPHA vBETA3, LEUR PROCEDE DE PREPARATION ET LES INTERMEDIAIRES DE CE PROCEDE, LEUR APPLICATION A TITRE DE MEDICAMENTS ET LES COMPOSITIONS PHARMACEUTIQUES LES RENFERMANT**

TRIZYCLISCHE VERBINDUNGEN MIT WIRKUNG AUF INTEGRINE, BESONDERS AUF DAS ALPHAvBETA3-INTEGRIN, VERFAHREN ZUR IHRER HERSTELLUNG UND ZWISCHENPRODUKTE DES VERFAHRENS, IHRE VERWENDUNG ALS ARZNEIMITTEL UND SIE ENTHALTENDE PHARMAZEUTISCHE ZUSAMMENSETZUNGEN

TRICYCLIC COMPOUNDS HAVING ACTIVITY SPECIFIC FOR INTEGRINS, PARTICULARLY ALPHAvBETA3 INTEGRINS, METHOD FOR PREPARING SAME, INTERMEDIATES THEREFOR, USE OF SAID COMPOUNDS AS DRUGS, AND PHARMACEUTICAL COMPOSITIONS CONTAINING SAME

(84) Etats contractants désignés:
**AT BE CH DE DK ES FI FR GB GR IE IT LI LU NL PT SE**

(30) Priorité: **20.03.1996 FR 9603437**

(43) Date de publication de la demande:
**07.01.1999 Bulletin 1999/01**

(73) Titulaires:
- **Aventis Pharma S.A.**
  **92160 Antony (FR)**
- **GENENTECH, INC.**
  **South San Francisco, CA 94080-4990 (US)**

(72) Inventeurs:
- **BERNARD, Serge**
  **F-60330 Le Plessis-Belleville (FR)**
- **CARNIATO, Denis**
  **F-91460 Marcoussis (FR)**
- **GOURVEST, Jean-François**
  **F-77410 Claye-Souilly (FR)**
- **TEUTSCH, Jean-Georges**
  **F-93500 Pantin (FR)**
- **KNOLLE, Jochen**
  **D-65830 Kriftel (DE)**
- **STILZ, Hans-Ulrich**
  **D-65929 Frankfurt am Main (DE)**
- **WEHNER, Volkmar**
  **D-97657 Sandberg 2 (DE)**
- **BODARY, Sarah, C.**
  **San Bruno, CA 94066 (US)**
- **GADEK, Thomas, R.**
  **Oakland, CA 94611 (US)**
- **MCDOWELL, Robert, S.**
  **San Francisco, CA 94114 (US)**
- **PITTI, Robert, M.**
  **El Cerrito, CA 94530 (US)**

(56) Documents cités:
**WO-A-96/06087**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 888 292 B1

## Description

La présente invention concerne de nouveaux composés tricycliques, leur procédé de préparation et les intermédiaires de ce procédé, leur application à titre de médicaments et les compositions pharmaceutiques les renfermant.

WO96/06087 décrit des dérivés bicycliques (benzazépine et benzodiazépine) qui présentent une activité antagoniste de la Vitronectine et peuvent ainsi être utilisés dans le traitement de l'ostéoporose.

La présente invention a pour objet les composés de formule générale (I) :

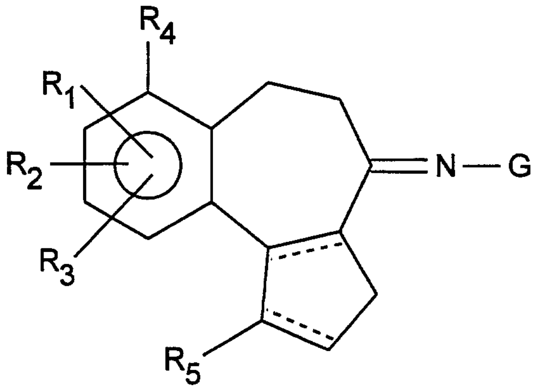

(I)

dans laquelle $R_1$ représente un groupement $-C{\equiv}C-[A]-[B]-COR_6$, $-CH=CH-[A]-[B]-COR_6$, $-(CH_2)_2-[A]-[B]-COR_6$, $-O-[A]-[B]-COR_6$, $-CH_2CO-[A]-[B]-COR_6$, -[A]- représentant

- soit un radical hydrocarboné bivalent dérivé d'une structure linéaire ou ramifiée, saturé ou insaturé, comportant de 1 à 12 atomes de carbone et de 1 à 6 hétéroatomes choisis parmi les atomes d'oxygène, d'azote ou de soufre,
- soit un radical bivalent dérivé d'un hydrocarbure acyclique, linéaire ou ramifié, saturé ou insaturé, comportant de 1 à 12 atomes de carbone,

[B] représentant un radical phényle, un radical CH(Z), ou une simple liaison,
Z représente un atome d'hydrogène, un groupement $(D)_{0-6}$-NRaRb, $(D)_{0-6}$-NH-$SO_2$-Rc, $(D)_{0-6}$-NH-$CO_2$-Rc, $(D)_{0-6}$-NH-CO-Rc, $(D)_{0-6}$-NH-$SO_2$-NH-Rc, $(D)_{0-6}$-NH-CO-NH-Rc, $(D)_{0-6}$-$CO_2$-Rc, $(D)_{0-6}$-$SO_2$-Rc, $(D)_{0-6}$-CO-Rc ou $(D)_{0-6}$-Rc dans lesquels $(D)_{0-6}$ est un radical bivalent dérivé d'un hydrocarbure acyclique, linéaire ou ramifié, saturé ou insaturé, comportant de 0 à 6 atomes de carbone,
Ra, Rb et Rc représentent un atome d'hydrogène, un radical $(CH_2)_{0-3}$-Ar dans lequel Ar représente un groupement aryle carbocyclique renfermant de 6 à 18 atomes de carbone, un radical $(CH_2)_{0-3}$-Het dans lequel Het représente un radical dérivé d'un hétérocycle aromatique ou non aromatique, saturé ou non saturé, comportant de 1 à 9 atomes de carbone et de 1 à 5 hétéroatomes choisis parmi les atomes d'oxygène, d'azote ou de soufre, un radical $(CH_2)_{0-3}$-Alk dans lequel Alk représente un radical dérivé d'un hydrocarbure, non aromatique, linéaire, ramifié ou cyclique, saturé ou insaturé, et comportant de 1 à 12 atomes de carbone, les radicaux Het, Ar et Alk pouvant être non substitués ou substitués,
ou encore, Ra et Rb représentent ensemble avec l'atome d'azote auquel ils sont liés un hétérocycle azoté, aromatique ou non aromatique, saturé ou insaturé, renfermant éventuellement un ou plusieurs hétéroatomes choisis parmi les atomes d'oxygène, d'azote ou de soufre, ce radical pouvant être substitué ou non substitué,

- $R_6$ représente un radical hydroxyle, un radical O-Alk, O-Ar, $NH_2$, NH-Alk, $N(Alk)_2$ ou le reste d'un amino acide L ou D, Alk et Ar étant tels que définis précédemment et pouvant être substitués ou non substitués,
- $R_2$ et $R_3$ identiques ou différents représentent <u>ou bien</u> un atome d'hydrogène, un radical hydroxyle, un radical O-Alk ou un radical O-$(CH_2)_{0-3}$-Ar, Alk et Ar étant tels que définis précédemment, <u>ou bien</u> $R_2$ et $R_3$ forment ensemble un cycle du type -O-$(CRdRe)_n$-O-, n étant un entier de 1 à 5, Rd et Re indépendamment l'un de l'autre représente un atome d'hydrogène, un radical alkyle renfermant de 1 à 6 atomes de carbone, ou un radical phényle,
- $R_4$ représente un atome d'hydrogène, un atome d'halogène, un groupement hydroxyle, amino, nitro, cyano, $CF_3$, acyle ou acyloxy renfermant de 1 à 12 atomes de carbone alkyle, alkényle, alkynyle, alkylthio, alkoxy, alkylamino, dialkylamino, dialkylaminoalkyle, dialkylaminoalkyloxy, dans lesquels le terme alkyle renferme de 1 à 6 atomes de carbone,

- $R_5$ représente un atome d'hydrogène, un radical hydroxyle, un atome d'halogène, un radical O-Alk ou un radical O-$(CH_2)_{0\text{-}3}$-Ar, Alk et Ar étant tels que définis précédemment,
- G représente,
  **soit** un radical de formule G1

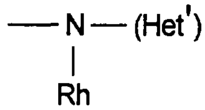

dans lequel Rh est un atome d'hydrogène ou un groupement (Alk) tel que défini précédemment et (Het') est un hétérocycle de formule générale :

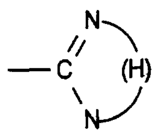

dans lequel (H) forme, avec le motif N=C-NH-, le reste d'un hétérocycle aromatique ou non aromatique, mono ou bicyclique, saturé ou non saturé, comportant de 1 à 9 atomes de carbone et de 2 à 5 hétéroatomes choisis parmi les atomes d'oxygène, d'azote et de soufre, ce radical pouvant être substitué ou non substitué,
- **soit** un radical NRaRb (radical G2), Ra et Rb étant tels que définis plus haut,
- **soit** un radical (Het) (radical G3) tel que défini plus haut,
- **soit** un radical -NRh-C(=X)-NHRc (radical G4), dans lequel X est un atome de soufre, d'oxygène ou NH, Rh et Rc sont tels que définis précédemment,
- **soit** un radical -NRh-$SO_2$Rc, (radical G5), dans lequel Rh et Rc sont tels que définis précédemment,
  les traits en pointillés représentent une éventuelle seconde liaison, ainsi que les sels d'addition avec les acides et les bases et les esters,
  $R_1$, $R_2$ et $R_3$ pouvant être en position 8, 9 ou 10 du tricycle, les substituants éventuels des radicaux (Alk), (Ar), (Het), (Het') ou NRaRb formant un hétérocycles pouvant être
- halogène
- alkyle, alkényle, alkynyle renfermant de 1 à 12 atomes de carbone,
- oxo, cyano, nitro, formyl, carboxy et carboxyalkyle renfermant de 1 à 6 atomes de carbone, carboxamide,
- alkoxy renfermant de 1 à 12 atomes de carbone,
- alkylthio renfermant de 1 à 12 atomes de carbone,
- amino, alkylamino renfermant de 1 à 12 atomes de carbone, dialkylamino renfermant de 2 à 24 atomes de carbone,
- aminoalkyle renfermant de 1 à 12 atomes de carbone,
- dialkylaminoalkyle renfermant de 3 à 25 atomes de carbone,
- dialkylaminoalkyloxy renfermant de 3 à 25 atomes de carbone,
- hydroxyle éventuellement acylé renfermant de 1 à 12 atomes de carbone,
- acyle renfermant de 1 à 12 atomes de carbone ou benzoyle éventuellement substitués par un atome de chlore, d'iode ou de fluor,
- aryle,carbocyclique ou hétérocyclique, ou aralkyle éventuellement substitués par halogène, alkyle, alkoxy, alkylthio, aminoalkyle ou dialkylamino indiqués ci-dessus.

Par composé de formule (I) on désigne tous les isomères géométriques et les stéréoisomères possibles pris individuellement ou en mélange.

Par groupe -[A]- représentant un radical bivalent dérivé d'une structure linéaire ou ramifiée, saturé ou insaturé, comportant de 1 à 12 atomes de carbone et de 1 à 6 hétéroatomes choisis parmi les atomes d'oxygène, d'azote et de soufre, on désigne notamment, les radicaux dérivés des alcanes dont certains carbones sont remplacés par des atomes d'oxygène, de soufre ou par des groupement C=O, SO, $SO_2$, NH, N(Alk), NH-CO, N(Alk)-CO, CO-NH, CO-N(Alk), $SO_2$-NH, $SO_2$-N(Alk), (Alk) étant tel que défini plus haut. Il peut alors s'agir des radicaux suivants -$CH_2$-$CH_2$-O-$CH_2$-

$CH_2$-, -$CH_2$-$CH_2$-N($CH_3$)-$CH_2$-$CH_2$-, $CH_2$-$CH_2$-C(O)-$CH_2$-$CH_2$, $CH_2$-C(O)-C$(Me)_2$-$CH_2$.

Lorsque - [A] - représente un radical bivalent dérivé d'un hydrocarbure acyclique, linéaire ou ramifié, saturé ou insaturé, comportant de 1 à 12 atomes de carbone, on désigne notamment les radicaux alkylènes de formule -$(CH_2)_n$-, dans laquelle n représente un entier compris entre 1 et 12, tels que -$CH_2$-, -$CH_2CH_2$-, -$CH_2CH_2CH_2$- ou -$CH_2CH_2CH_2CH_2$-, ou bien les radicaux alkénylène ou alkynylène tels que -CH=CH-$CH_2$- ou -C≡C-$CH_2$-.

Lorsque ces radicaux bivalents sont ramifiés, il peut s'agir de radicaux tels que -CH($CH_3$)-, -C$(Me)_2$, -$CH_2$-C$(Me)_2$-, -CH(Et)-, -CH(C≡CH)- ou -C(C≡CH)(Et)-.

Lorsque [B] représente un radical bivalent -Ph-, le groupement $COR_6$ peut être en position ortho, méta ou para. Il se trouve de préférence en position para.

**[0009]** Lorsque $(D)_{0-6}$ est un radical bivalent dérivé d'un hydrocarbure acyclique, linéaire ou ramifié, saturé ou insaturé, comportant de 0 à 6 atomes de carbone, $(D)_{0-6}$ est choisi parmi les valeurs de [A] citées plus haut. On entend par $(D)_0$ l'absence de ce radical ce qui revient à avoir une simple liaison covalente. (D) sera de préférence une simple liaison ou un groupement $(CH_2)_n$, n étant un entier choisi parmi 1, 2 ou 3.

Lorsque Ra, Rb et Rc représentent un groupement $(CH_2)_{0-3}$-Ar, $(CH_2)_{0-3}$-Het, $(CH_2)_{0-3}$-Alk, $(CH_2)_{0-3}$ représente soit une simple liaison dans le cas de $(CH_2)_0$, soit les radicaux -$CH_2$-, -$(CH_2)_2$- ou -$(CH_2)_3$-.

Par le terme (Ar) représentant un groupement aryle carbocyclique renfermant de 6 à 18 atomes de carbone, on entend un radical dérivé d'un hydrocarbure cyclique aromatique tel que le radical phényle, naphtyle, phénanthrènyl ou bien un radical dérivé d'un hydrocarbure bicyclique ou tricyclique condensé comportant un cycle benzénique tel que indanyle, indényle, dihydronaphtyle, tétrahydronaphtyle ou fluorènyl. La jonction s'effectue au niveau du cycle benzénique. Il s'agit de préférence du phényle.

Par le terme (Het) représentant un radical dérivé d'un hétérocycle aromatique ou non aromatique, saturé ou non saturé, comportant de 1 à 9 atomes de carbone et de 1 à 5 hétéroatomes choisis parmi les atomes d'oxygène, d'azote et de soufre, on désigne notamment :

- les radicaux monocyclique hétérocycliques, par exemple les radicaux thiényle, furyle, pyrannyle, pyrrolyle, imidazolyle, pyrazolyle, pyridyle, pyrazinyle, pyrimidinyle, pyridazinyle, thiazolyle, oxazolyle, furazannyle, pyrrolinyle, imidazolinyle, pyrazolinyle, thiazolinyle, triazolyle, tétrazolyle,
- les cycles condensés hétérocycliques, par exemple le benzofurannyle, le benzothiényle, le benzimidazolyle, le benzothiazolyle, le naphto[2,3-b]thiényle, le thianthrényle, l'isobenzofurannyle, le chroményle, le xanthényle, le phénoxathiinyle, l'indolizinyle, l'isoindolyle, le 3H-indolyle, l'indolyle, l'indazolyle, le purinyle, le quinolizinyle, l'isoquinolyle, le quinolyle, le phtalazinyle, le naphtyridinyle, le quinoxalinyle, le quinazolinyle, le cinnolinyle, le ptéridinyle, le carbazolyle, le béta-carbolinyle, l'acridinyle, le phénazinyle, le phénothiazinyle, le phénoxazinyle, l'indolinyle, l'isoindolinyle, l'imidazopyridyle, l'imidazopyrimidinyle ou encore les systèmes polycycliques condensés constitués de monocycliques hétérocycliques tels que définis ci-dessus comme par exemple le furo[2,3-b]pyrrole ou le thiéno[2,3-b]furanne,
- ou les hétérocycles saturés tels que pyrrolidine, pipéridine, morpholine.

Ce terme (Het) englobe par ailleurs les valeurs de (Het') telles que définies précédemment.

Par le terme (Alk) représentant un radical dérivé d'un hydrocarbure, non aromatique, linéaire, ramifié ou cyclique, saturé ou insaturé, on désigne dans le cas des hydrocarbures acycliques les radicaux alkyles tels que méthyle, éthyle, propyle, isopropyle, butyle, isobutyle, tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle, nonyle, 2,4-diméthylheptyle ou n-décyle, les radicaux alkényles tels que vinyle, propényle, isopropényle, allyle, 2-méthylallyle, butényle ou isobutényle, ou les radicaux alkynyles tels que éthynyle, propynyle, propargyle, butynyle ou isobutynyle, et dans le cas des radicaux cycliques, les radicaux cycloalkyles, tels que cyclopropyle, cyclobutyle, cyclopentyle, cyclohexyle ou adamantyle.

Lorsque Ra et Rb représentent ensemble avec l'atome d'azote auquel ils sont liés un hétérocycle azoté, il s'agit notamment des hétérocycles saturés suivants morpholine, pipéridine, pipérazine, pyrrolidine, ou des hétérocycles insaturés tels que pyrimidine, pyridine ou pyrazine.

Lorsque $R_2$, $R_3$, $R_4$ et $R_5$ représentent un radical O-(Alk) renfermant de 1 à 12 atomes de carbone, il s'agit de préférence des radicaux méthoxy, éthoxy, propyloxy, isopropyloxy, butyloxy, allenyloxy ou propargyloxy. Lorsque $R_2$, $R_3$, $R_4$ et $R_5$ représentent un radical O-$(CH_2)_{O-3}$-Ar on entend de préférence les radicaux phényléthoxy et phénylpropyloxy.

Lorsque $R_2$ et $R_3$ forment ensemble un cycle du type -O-$(CRdRe)_n$-O-, n étant un entier de 1 à 5, il s'agit notamment des radicaux -O-$CH_2$-O, O-C($Me_2$)-O, O-C($Ph_2$)-O. $R_2$ et $R_3$ sont impérativement en position ortho l'un de l'autre.

Lorsque $R_6$ représente un radical O-Alk ou O-Ar, Alk et Ar étant substitués ou non substitués, il s'agit notamment des radicaux suivants : ($C_1$-$C_8$) alkoxy, ($C_1$-$C_{14}$)-aryl ($C_1$-$C_8$)-alkoxy, ($C_6$-$C_{14}$) aryloxy, ($C_1$-$C_8$) alkylcarboxyloxy,

($C_1$-$C_8$) dialkylaminocarbonylméthoxy, ($C_6$-$C_{14}$) aryl ($C_1$-$C_8$) dialkylaminocarbonylméthoxy.

Lorsque $R_6$ représente un radical NH-alk, NH(alk)$_2$ ou NH-Ar, il s'agit notamment des radicaux ($C_1$-$C_8$) alkylamino, di($C_1$-$C_8$) alkylamino, ($C_6$-$C_{14}$) aryl ($C_2$-$C_8$) alkylamino, $C_6$-$C_{14}$) arylamino.

Lorsque $R_6$ représente le reste d'un amino acide il peut s'agir d'amino acide L ou D.

Les amino acides L ou D peuvent être naturels ou non naturels. De préférence il s'agit des α-amino acides. Par exemple, ceux décrits dans Houben-Weyl, Methoden der organischen Chemie, Band XV/1 et 2, Georg Thieme Verlag, Stuttgart, 1974 :

Aad, Abu, γAbu, Abz, 2ABz, εAca, Ach, Acp, Adpd, Ahb, Aib, βAib, Ala, βAla, Δala, Alg, All, Ama, Amt, Ape, Apm, Apr, Arg, Asn, Asp, Asu, Aze, Azi, Bai, Bph, Can, Cit, Cys, (Cys)$_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, Δlys, Met, Mim, Min, nArg, Nle, Nva, Oly, Orn, Pan, Pec, Pen, Phe, Phg, Pic, Pro, Δpro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, tert-butylglycine (Tbg), Neopentylglycine (Npg), Cyclohexylglycine (Chg), Cyclohexylalanine (Cha), 2-Thienylalanine (Thia), acide 2,2-diphénylaminoacétique, acide 2-(p-tolyl) 2-phénylamino acétique, acide 2-(p-chlorophényl) amino acétique,

ou encore

l'acide 2-pyrrolidine acétique, l'acide 1,2,3,4-tétrahydroisoquinoline 3-acétique, l'acide décahydroisoquinoline 3-acétique, l'acide octahydroisoindol 2-acétique, l'acide décahydroquinoline 2-acétique, l'acide octahydrocyclopenta [b] pyrrol 2-carboxylique, l'acide 2-azabicyclo [2,2,2] octan-3-carboxylique, l'acide 2-azabicyclo [2,2,1] heptan-3-carboxylique, l'acide 2-azabicyclo [3,1,0] hexan-3-carboxylique, l'acide 2-azaspiro [4,4] nonan-3-carboxylique, l'acide 2-azaspiro [4,5] decan-3-carboxylique, l'acide spiro (bicyclo [2,2,1] heptan)-2,3-pyrrolidin-5-carboxylique, l'acide spiro (bicyclo [2,2,2] octan-2,3-pyrrolidin-5-carboxylique, l'acide 2-azatricyclo [4,3,0,1$^{6,9}$] decan-3-carboxylique, l'acide décahydrocyclohepta [b] pyrrol-2-carboxylique, l'acide decahydrocycloocta [c] pyrrol-2-carboxylique, l'acide octahydrocyclopenta [c] pyrrol-2-carboxylique, l'acide octahydroisoindol-1-carboxylique, l'acide 2,3,3a,4,6a-hexahydrocyclopenta [b] pyrrol-2-carboxylique, l'acide 2,3,3a,4,5,7a-hexahydroindol-2-carboxylique, l'acide tétrahydrothiazol-4-carboxylique, l'acide isoxazolidin-3-carboxylique, l'acide pyrazolidin-3-carboxylique, l'acide hydroxypyrrolidin-2-carboxylique, qui le cas échéant, peuvent être substitués (voir les formules suivantes) :

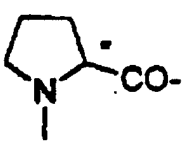 ; 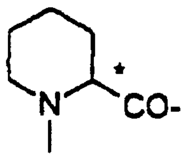 ; 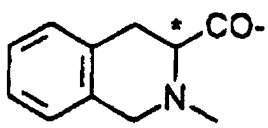 ; 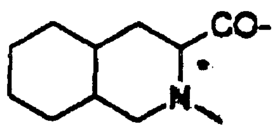 ;

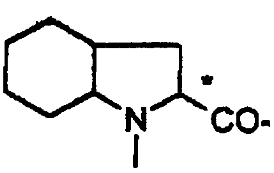 ; 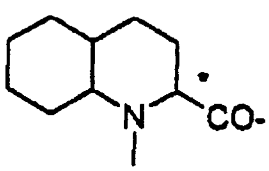 ; 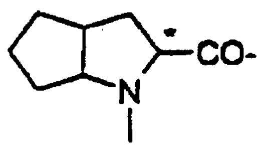 ;

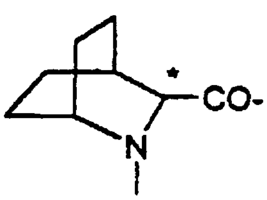 ; 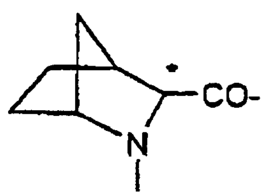 ; 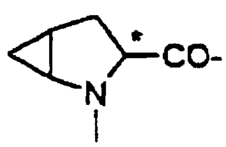 ;

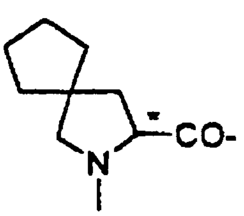 ; 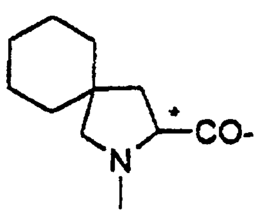 ; 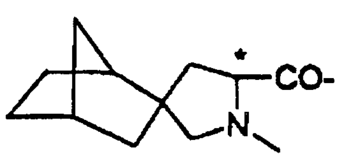 ;

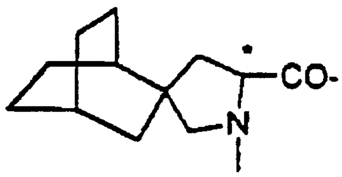 ; 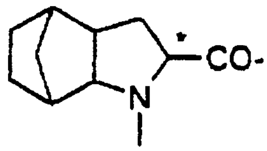 ; 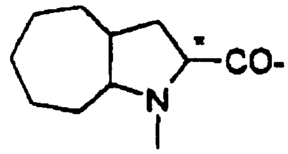 ;

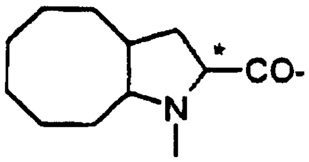 ; 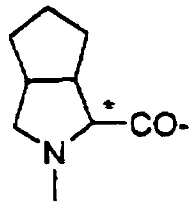 ; 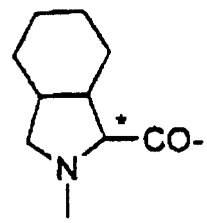 ;

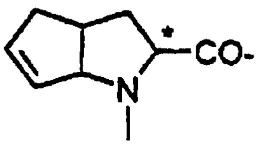 ; 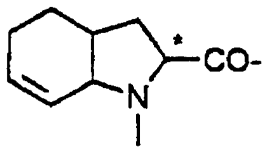 ; 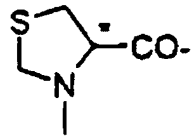 ; 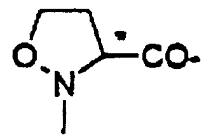 ;

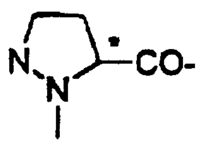 ; 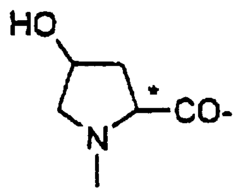 .

Les restes hétérocycles tels que décrits plus haut sont connus par exemple dans les brevets ou demandes de brevets suivants :

US-A-4.344.949 ; US-A-4.374.847 ; US-A-4.350.704 ; EP-A-29.488 ; EP-A-31.741 ; EP-A-46.953 ; EP-A-49.605 ; EP-A-49.658 ; EP-A-50.800 ; EP-A-51.020 ; EP-A-52.870 ; EP-A-79.022 ; EP-A-84.164 ; EP-A-89.637 ; EP-A-90.341 ; EP-A-90.362 ; EP-A-105.102 ; EP-A-109.020 ; EP-A-111.873 ; EP-A-271.865 et EP-A-344.682.

De plus les amino acides peuvent être sous forme d'ester ou d'amide, comme par exemple, ester méthylique, ester éthylique, ester isopropylique, ester isobutylique, ester tert-butylique, ester benzylique, ethylamide, semicarbazide ou ω-amino ($C_2$-$C_8$)-alkylamide.

Enfin, les groupes fonctionnels de ces amino acides peuvent être protégés. Les groupements protecteurs appropriés tels que les groupements protecteurs des uréthannes, les groupements protecteurs des carboxyle ou les groupements protecteurs des chaînes latérales sont décrites par Hubbuch, Kontakte (Merck) 1979, n° 3, p. 14-23 et par Büllesbach, Kontakte (Merck) 1980, n° 1, p. 23-35.

On peut citer par exemple Aloc, Pyoc, Fmoc, Tcboc, Z, Boc, Ddz, Bpoc, Adoc, Msc, Moc, $Z(NO_2)$, $Z(Hal_n)$, Bobz, Iboc, Adpoc, Mboc, Acm, tertbutyl, Obzl, Onbzl, Ombzl, Bzl, Mob, Pic, Trt.

Lorsque G est un radical de formule G1

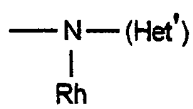

et (Het') est un hétérocycle de formule générale :

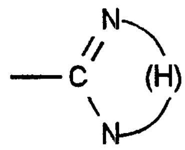

dans lequel (H) forme, avec le motif N=C-NH-, un hétérocycle aromatique ou non aromatique, mono ou bicyclique, saturé ou non saturé, comportant de 1 à 9 atomes de carbone et de 2 à 5 hétéroatomes choisis parmi les atomes d'oxygène, d'azote et de soufre, ce radical pouvant être substitué ou non substitué, G1 représente notamment des hétérocycles suivants :

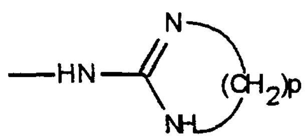

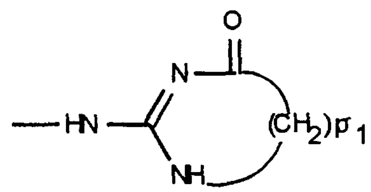

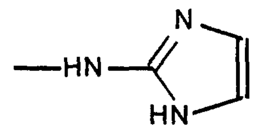

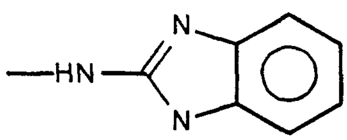

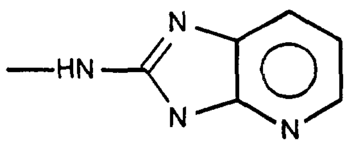

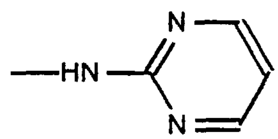

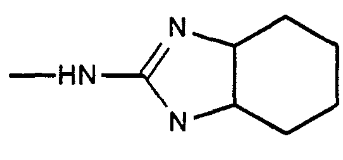

dans lesquels p représente un entier de 1 à 4.

- Lorsque G est un radical -NRaRb (nommé G2), Ra et Rb, peuvent être un atome d'hydrogène, un radical $(CH_2)_{0-3}$-Ar, $(CH_2)_{0-3}$-Het ou $(CH_2)_{0-3}$-Alk. Les groupements Ar, Het et Alk pouvant également être substitués par les groupements tels que définis plus bas.

G2 peut être notamment un groupement $NH_2$, NH-Alk tel que NHMe, NHEt, $N(Alk)_2$ tel que $NMe_2$, $NEt_2$, NMeEt, NH-$(CH_2)_{0-1}$-Ar tel que NHPh, $NHCH_2Ph$ ou bien $NHCH_2Het$ tel que $NHCH_2$-pyrrol-2-yle.

Lorsque Ra est un atome d'hydrogène ou un groupement (Alk) et lorsque Rb est un groupement (Het') on retrouve les valeurs de G1.

Lorsque Ra et Rb forment ensemble avec l'atome d'azote auquel ils sont liés un hétérocycle azoté, il s'agit notamment des groupements hétérocycliques tels que décrits plus haut, ceux-ci pouvant être substitués ou non substitués.

- Lorsque G est un radical (Het) (radical G3) ce radical pouvant être substitué ou non substitué, il s'agit notamment

des hétérocycles listés plus haut et en particulier les hétérocycles de formule générale (Het') telle que définie plus haut. Lorsque cet hétérocycle est relié au niveau de son atome d'azote, on retrouve les valeurs de G2 dans lesquelles Ra et Rb forment avec l'atome d'azote qui les porte un hétérocycle.

- Lorsque G est un radical -NRh-C(=X)-NHRc (radical G4), ou $NRhSO_2Rc$ (radical G5), dans lesquels X est un atome de soufre, d'oxygène ou NH, Rh et Rc sont tels que définis précédemment. Il s'agit notamment des groupements -NH-C(=NH)-$NH_2$, -NH-C(=O)-$NH_2$ ou -NH-C(=S)-$NH_2$, -NH-C(=NH)-$NHCH_2$-Ar tel que -NH-C(=NH)-$NHCH_2$Ph, -NH-C(=NH)-$NHCH_2$-Het, -NH-C(=NH)-$NHCH_2$-Het', -NH-C(=NH)-NH-Alk tel que -NH-C(=NH)-$NHCH_3$, ou -NH-$SO_2$Ph, les groupements Ar, Het, Het' ou Alk étant substitués ou non substitués.

Les substituants éventuels des radicaux (Alk), (Ar), (Het), (Het') ou NRaRb formant un hétérocycle, sont de préférence les radicaux suivants :

- halogène : fluor, chlore, brome, iode,
- alkyle, alkényle, alkynyle renfermant de 1 à 12 atomes de carbone tel que méthyle, éthyle, propyle, isopropyle, butyle, isobutyle, tert-butyle, vinyl ou allenyl. Ces radicaux étant eux mêmes éventuellement substitués par un ou plusieurs atome d'halogène, par exemple le fluor tel que le trifluorométhyle.
- oxo, cyano, nitro, formyl, carboxy et carboxyalkyl renfermant de 1 à 6 atomes de carbone, carboxamide,
- alkoxy renfermant de 1 à 12 atomes de carbone tel que méthoxy, éthoxy, propyloxy, isopropyloxy, butyloxy,
- alkylthio renfermant de 1 à 12 atomes de carbone tel que méthylthio, éthylthio, propylthio, isopropylthio, butylthio,
- amino, alkylamino renfermant de 1 à 12 atomes de carbone tel que méthylamino ou éthylamino, dialkylamino renfermant de 2 à 24 atomes de carbone tel que diméthylamino, diéthylamino, méthyléthylamino, chacun de ces radicaux dialkylamino étant éventuellement sous forme oxydée,
- aminoalkyle renfermant de 1 à 12 atomes de carbone tel que aminométhyle ou aminoéthyle,
- dialkylaminoalkyle renfermant de 3 à 25 atomes de carbone tel que diméthylamino méthyle ou éthyle,
- dialkylaminoalkyloxy renfermant de 3 à 25 atomes de carbone tel que diméthylaminoéthyloxy,
- hydroxyle éventuellement acylé renfermant de 1 à 12 atomes de carbone, par exemple acétoxy,
- acyle renfermant de 1 à 12 atomes de carbone tels que formyle, acétyle, propionyle, butyryle, isobutyryle, valéryle, isovaléryle, succinyle, pivaloyle benzoyle éventuellement substitué par exemple par un atome de chlore, d'iode ou de fluor. On peut citer les radicaux chloroacétyle, dichloroacétyle, trichloroacétyle, bromoacétyle ou trifluoroacétyle,
- aryle carbocyclique ou hétérocyclique tel que phényle, furyle, thiènyle, pyridinyle ou aralkyle tel que benzyle, ces radicaux étant eux-mêmes éventuellement substitués par des radicaux halogène, alkyle, alkoxy, alkylthio, amino alkyle ou dialkylamino indiqués ci-dessus.

Bien entendu, un ou plusieurs substituants, identiques ou différents, peuvent être présents. Dans le cas de (Het) les substituants peuvent être au niveau du groupement NH ou de l'atome de carbone.

Ces substituants illustrent également la définition de $R_4$.

Il est bien entendu que lorsque $R_1$, $R_2$, $R_3$, $R_4$, $R_5$, $R_6$, $R_a$, $R_b$, $R_c$ renferment un groupement alkyle, aryle ou hétérocycle tels que définis ci-dessus, ils peuvent être identiques ou différents indépendamment les uns des autres.

L'invention s'étend naturellement aux sels des composés de formule (I), comme par exemple les sels formés, lorsque les composés de formule (I) comportent une fonction amino ou amino guanidine, avec les acides chlorhydrique, bromhydrique, nitrique, sulfurique, phosphorique, acétique, trifluoroacétique, formique, propionique, benzoïque, maléique, fumarique, succinique, tartrique, citrique, oxalique, glyoxylique, aspartique, alcanesulfoniques tels que les acides méthane ou éthanesulfonique, arènesulfoniques, tels que les acides benzène ou paratoluène sulfonique et arylcarboxylique, ou lorsque les composés de formule (I) comportent une fonction acide, avec les sels des métaux alcalins ou alcalino terreux ou d'ammonium éventuellement substitué.

L'invention s'étend également aux esters des composés de formule (I).

Dans un premier groupe préféré, l'invention a pour objet les composés de formule générale (I) telle que définie précédemment, répondant à la formule générale (I') :

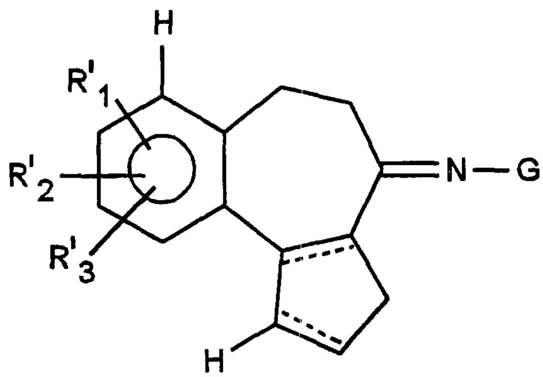

(I')

dans laquelle $R'_1$ représente un groupement
-C≡C-[A']-[B']-$COR'_6$, -CH=CH-[A']-[B']-$COR'_6$,
-$(CH_2)_2$-[A']-[B']-$COR'_6$, -O-[A']-[B']-$COR'_6$,
-$CH_2$CO-[A']-[B]-$COR'_6$, - [A']- représentant un radical bivalent alkylène, alkénylène ou alkynylène renfermant de 1 à 6 atomes de carbone, [B'] représentant un radical CH(Z') ou une simple liaison,
Z' représente un atome d'hydrogène, un groupement
$(CH_2)_{0-6}$-NRaRb, $(CH_2)_{0-6}$-NH-$SO_2$-Rc, $(CH_2)_{0-6}$-NH-$CO_2$-Rc, $(CH_2)_{0-6}$-NH-CO-Rc, $(CH_2)_{0-6}$-NH-$SO_2$-NH-Rc, $(CH_2)_{0-6}$-NH-CO-NH-Rc, $(CH_2)_{0-6}$-$CO_2$-Rc, $(CH_2)_{0-6}$-$SO_2$-Rc, $(CH_2)_{0-6}$-CO-Rc ou $(CH_2)_{0-6}$-Rc, Ra, Rb et Rc étant tels que définis précédemment, $R'_6$ représente un radical OH, amino ou alkoxy renfermant de 1 à 8 atomes de carbone, éventuellement substitué par un ou plusieurs radicaux choisis parmi les radicaux hydroxy, amino, phényle, alkylamino ou dialkylamino, $R'_2$ et $R'_3$ représentent un atome d'hydrogène ou un radical méthoxy, et G est tel que défini précédemment, les traits en pointillés représentent une éventuelle seconde liaison, ainsi que les sels d'addition avec les acides et les bases et les esters.

Dans un deuxième groupe préféré l'invention a pour objet les composés de formule générale (I) telle que définie précédemment dans laquelle $R_6$ représente un groupement -OH, -$OCH_3$, -$OCH_2CH_3$,-O-$(CH_2)_2$-OH,

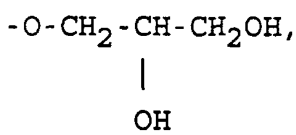

-O-$(CH_2)_2$-$NH_2$, -O-$(CH_2)_2$-N-$(CH_3)_2$, -$NH_2$ ou -O-($CH_2$)-phényle, ainsi que les sels d'addition avec les acides et les bases et les esters.

Dans un troisième groupe préféré, l'invention a pour objet les composés de formule générale (I) telle que définie précédemment dans laquelle $R_1$ représente un groupement O-$(CH_2)_{0-6}$CH(Z')-COOH ou -$(CH_2)_{0-7}$-CH(Z')-COOH, ainsi que les sels d'addition avec les acides et les bases et les esters.

Dans un quatrième groupe préféré, l'invention a pour objet les composés de formule générale (I) telle que définie précédemment, dans laquelle (Z') est un atome d'hydrogène, ainsi que les sels d'addition avec les acides et les bases et les esters.

Dans un cinquième groupe préféré, l'invention a pour objet les composés de formule générale (I) telle que définie précédemment, dans laquelle (Z') est le groupement $(CH_2)_{0-6}$-NH-$CO_2$-Rc ou $(CH_2)_{0-6}$-NHRb, Rb et Rc étant tels que définis à précédemment, ainsi que les sels d'addition avec les acides et les bases et les esters.

Dans un sixième groupe préféré, l'invention a pour objet les composés de formule générale (I) telle que définie précédemment, dans laquelle Rb et Rc représentent le groupement $(CH_2)_{0-3}$-Ar, Ar étant tel que défini précédemment et pouvant être substitué ou non substitué, ainsi que les sels d'addition avec les acides et les bases et les esters.

Dans un septième groupe préféré, l'invention a pour objet les composés de formule générale (I) telle que définie précédemment, dans laquelle G est un groupement G4 de formule -NH-C(=NH)-NHRc, Rc étant tel que défini précédemment, ainsi que les sels d'addition avec les acides et les bases et les esters.

Dans un huitième groupe préféré, l'invention a pour objet les composés de formule générale (I) telle que définie précédemment, dans laquelle G est un groupement G4 de formule NH-C(=NH)-$NH_2$, ainsi que les sels d'addition avec les acides et les bases et les esters.

Dans un neuvième groupe préféré, l'invention a pour objet les composés de formule générale (I) telle que définie précédemment, dans laquelle G est un groupement -NH-(Het') tel que défini précédemment et notamment,

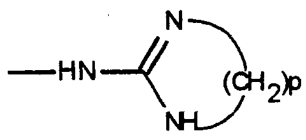
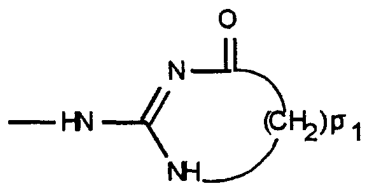
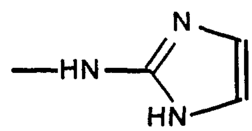

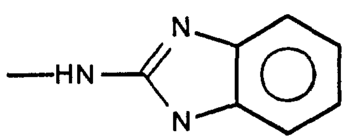
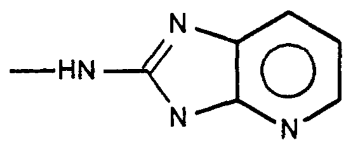
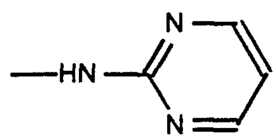

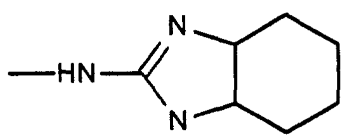

p étant un entier égal à 2, 3 ou 4, ces hétérocycles étant substitués ou non substitués, ainsi que les sels d'addition avec les acides et les bases et les esters.

Dans un dixième groupe préféré, l'invention a pour objet les composés de formule générale (I) telle que définie précédemment, dans laquelle G est le groupement

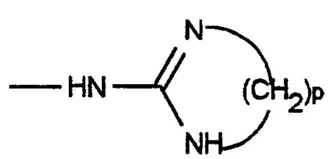

p étant un entier égal à 2, 3 ou 4, ainsi que les sels d'addition avec les acides et les bases et les esters.

Dans un onzième groupe préféré, l'invention a pour objet les composés de formule (I) telle que définie précédemment dont les noms suivent:

- Acide 4-((4-((aminoiminométhyl)hydrazono)-9,10-diméthoxy-1, 2,3,4,5,6-hexahydro-8-benz(e)azulèn-yl)oxy)-butanoique,
- Acide 5-((4-((aminoiminométhyl)hydrazono)-9,10-diméthoxy-1, 2,3,4,5,6-hexahydro-8-benz(e)azulèn-yl)oxy)-pentanoique,
- Acide 5-((4-((aminoiminométhyl)hydrazono)-8,10-diméthoxy-1, 2,3,4,5,6-hexahydro-9-benz(e)azulèn-yl)oxy)-pentanoique,
- Acide 6-((4-((aminoiminométhyl)hydrazono)-9,10-diméthoxy-1, 2,3,4,5,6-hexahydro-8-benz(e)azulènyl)oxy)-hexanoique,

- Acide 7-((4-((aminoiminométhyl)hydrazono)-9,10-diméthoxy-1, 2,3,4,5,6-hexahydro-8-benz(e)azulènyl)oxy)-heptanoique,
- Acide 5-((9,10-diméthoxy-1,2,3,4,5,6-hexahydro-4-((4,5-dihydro-1H-imidazol-2-yl)hydrazono)-8-benz(e)azulènyl)oxy)pentanoique,
- chlorhydrate de 5-((4-((aminoiminométhyl)hydrazono)-9,10-diméthoxy-1, 2,3,4,5,6-hexahydro-8-benz(e)azulènyl) oxy)pentanoate d'éthyle,
- Acide 4-((4-((aminoiminométhyl)hydrazono)-8,9-diméthoxy-1, 2,3,4,5,6-hexahydro-10-benz(e)azulènyl)oxy)-butanoique,
- Acide 5-((4-((aminoiminométhyl)hydrazono)-8,9-diméthoxy-1, 2,3,4,5,6-hexahydro-10-benz(e)azulènyl)oxy)-pentanoique,
- Acide 5-((4-(((amino)carbonyl)hydrazono)-9,10-diméthoxy-1, 2,3,4,5,6-hexahydro-8-benz(e)azulènyl)oxy)-pentanoique,
- Acide 5-((4-(((amino)thiocarbonyl)hydrazono)-9,10-diméthoxy-1,2,3,4,5,6-hexahydro-8-benz(e)azulènyl)oxy)-pentanoique,
- Acide 4-((4-((aminoiminométhyl)hydrazono)-8,10-diméthoxy-1, 2,3,4,5,6-hexahydro-9-benz(e)azulèn-yl)oxy)-butanoique,
- Acide 6-((4-((4,5-dihydro-1H-imidazol-2-yl)hydrazono)-9,10-diméthoxy-1, 2,3,4,5,6-hexahydro-8-benz(e)azulênyl)oxy)hexanoique,
- Acide 5-((4-((aminoiminométhyl)hydrazono)-9,10-diméthoxy-1, 2,3,4,5,6-hexahydro-8-benz(e)azulènyl)oxy)-3,3-diméthyl-4-oxo-pentanoique,
- Acide 5-((4-((4,5-dihydro-1H-imidazol-2-yl)hydrazono)-9,10-diméthoxy-1, 2,3,4,5,6-hexahydro-8-benz(e)azulènyl)oxy)-3,3-diméthyl-4-oxo-pentanoique,
- Chlorhydrate de l'acide 5-((4-((aminoiminométhyl)hydrazono)-9,10-diméthoxy-1, 2,3,4,5,6-hexahydro-8-benz(e) azulènyl)oxy)-pentanoique,
- Acide 4-((4-((4,5-dihydro-1H-imidazol-2-yl)hydrazono)-9,10-diméthoxy-1, 2,3,4,5,6-hexahydro-8-benz(e)azulènyl)oxy)butanoique,
- Acide 5-((8((aminoiminométhyl)hydrazono)-6,7,8,9,10,11-hexahydro-azulèno(5,6-d)-1,3-benzodioxol-4-yl)oxy)-pentanoique,
- Acide 5-((8((aminoiminométhyl)hydrazono)-2,2-diphényl-6,7,8,9,10,11-hexahydro-azulèno(4,5-e)-(1,3)-benzodioxol-4-yl)oxy)-pentanoique,
- Acide 4-((9,10-diméthoxy-4-((1,4,5,6-tétrahydro-2-pyrimidinyl)hydrazono)-1,2,3,4,5,6-hexahydro-8-benz(e)azulènyl) oxy)-butanoique,
- Acide 2-((4-((4,5-dihydro-1H-imidazol-2-yl)hydrazono)-9,10-diméthoxy-1,2,3,4,5,6-hexahydro-8-benz(e)azulènyl)oxy)-éthanoique,
- Acide 3-((4-((4,5-dihydro-1H-imidazol-2-yl)hydrazono)-9,10-diméthoxy-1,2,3,4,5,6-hexahydro-8-benz(e)azulènyl)oxy)-propanoique,
- Acide 4-((4-((4,5-dihydro-1H-imidazol-2-yl)hydrazono)1,2,3,4,5,6-hexahydro-8-benz(e)azulènyl)oxy)-butanoique,
- Acide 4-((4-((4,5-dihydro-1H-imidazol-2-yl)hydrazono)1,2,3,4,5,6-hexahydro-8-benz(e)azulènyl)oxy)-butanoique,
- O-[4[(4,5-dihydro-1H-imidazol-2-yl)hydrazono]-9,10-diméthoxy-1,2,3,4,5,6-hexahydro-8-benz[e]azulènyl]-N-[(phénylméthoxy)carbonyl]-DL-homoserine,
- O-[4[(4,5-dihydro-1H-imidazol-2-yl)hydrazono]-1,2,3,4,5,6-hexahydro-8-benz[e]azulènyl]-N-[(phénylméthoxy) carbonyl]-DL-homoserine,
- O-[4-[(1,2,3,4-tétrahydro 6-pyrimidinyl) hydrazono] 9,10-diméthoxy 1,2,3,4,5,6-hexahydro 8-benz[e]azulenyl] N-[(phénylméthoxy) carbonyl] DL-homoserine,
- ester (2,3-dihydroxypropylique) de 0-(9,10-diméthoxy 1,2,3,4,5,6,-hexahydro 4-[(1,4,5,6-tétrahydro 2-pyrimidinyl) hydrazono]-8-benz(e)azulényl] N-[(phénylméthoxy) carbonyl] DL-homoserine,
- O-[4-[(4,5-dihydro 1H-imidazol-2-yl) hydrazono] 9,10-diméthoxy 1,2,3,4,5,6-hexahydro 8-benz(e)azulenyl] N-[(8-quinoléinyl) sulfonyl] DL-homoserine,
- monochlorhydrate de O-[4-[(4,5-dihydro 1H-imidazol-2-yl) hydrazono] 9,10-diméthoxy 1,2,3,4,5,6-hexahydro 8-benz(e)-azulényl] N-[[3-[4-(3-pyridinyl) 1H-imidazol-1-yl] propoxy] carbonyl] DL-homoserine,
- Acide 5-[[4-[(4,5-dihydro 4-oxo 1H-imidazol-2-yl) hydrazono] 9,10-diméthoxy 1,2,3,4,5,6-hexahydro 8-benz(e)azulényl] oxy] pentanoique,
- O-[9,10-diméthoxy 1,2,3,4,5,6-hexahydro 4-[(4,5,6,7-tétrahydro 1H-1,3-diazépin-2-yl) hydrazono] 8-benz(e)-azulényl] N-[(phénylméthoxy) carbonyl] DL-homoserine,
- O-[9,10-diméthoxy 1,2,3,4,5,6-hexahydro 4-[(3a,4,5,6,7,7a-hexahydro 1H-benzimidazol-2-yl) hydrazono] 8-benz (e)azulényl] N-[(phénylméthoxy) carbonyl] DL-homoserine.

L'invention a également pour objet un procédé de préparation des composés de formule générale (I) caractérisé en ce que l'on soumet un composé de formule (II) :

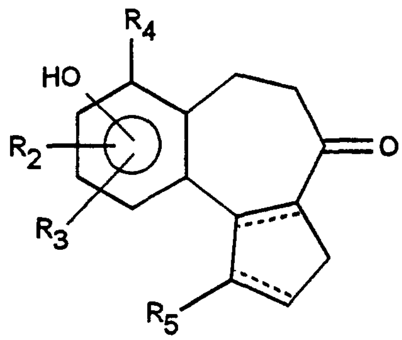

(II)

dans laquelle $R_2$, $R_3$, $R_4$ et $R_5$ sont tels que décrits précédemment à l'exception de la valeur hydroxyle, soit à l'action d'un composé de formule (F1) en présence d'une base,

Hal-[A]-[B]-$COR_6$ (F1)

ou d'un composé de formule (F'1) en présence d'une phosphine et d'azodicarboxylate de diéthyle :

HO-[A]-[B]-$COR_6$ (F'1)

dans lesquelles Hal est un atome d'halogène, [A], [B] et $R_6$ sont tels que décrits précédemment, [B] pouvant également représenter le groupement

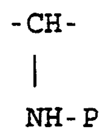

,

P étant un groupement protecteur de la fonction amine, afin d'obtenir un composé de formule (IIIa) :

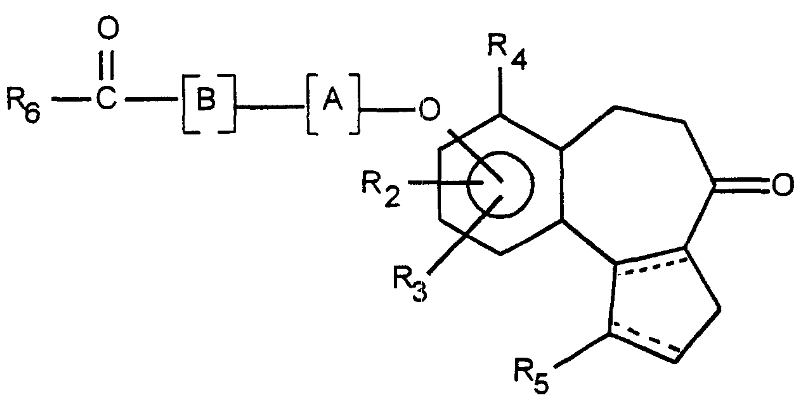

(IIIa)

soit à l'action d'un groupement activant puis d'un composé de formule (F2) en présence d'un catalyseur :

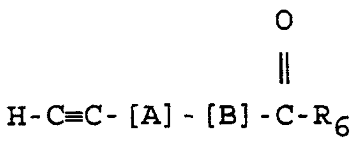

(F2)

afin d'obtenir un composé de formule (IIIb) :

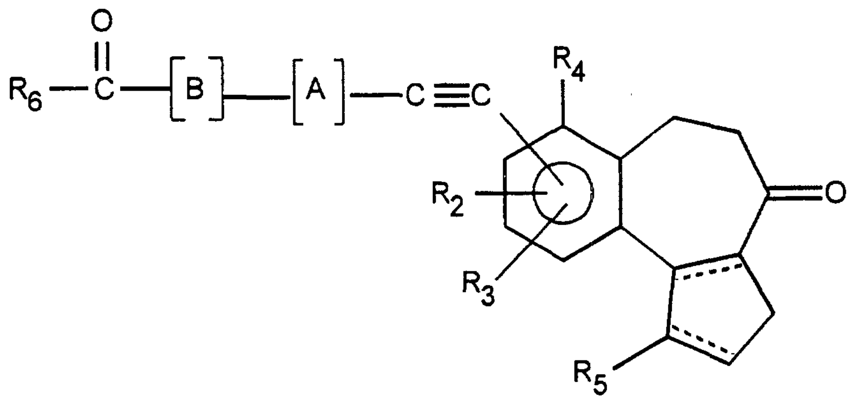

(IIIb)

composés de formule (IIIa) ou (IIIb) que l'on soumet à l'action d'un composé de formule (F3) :

$H_2N\text{-}G$ (F3)

dans laquelle G est tel que décrit précédemment, afin d'obtenir les composés de formule (IVa) et (IVb) correspondant à certains produits de formule (I) :

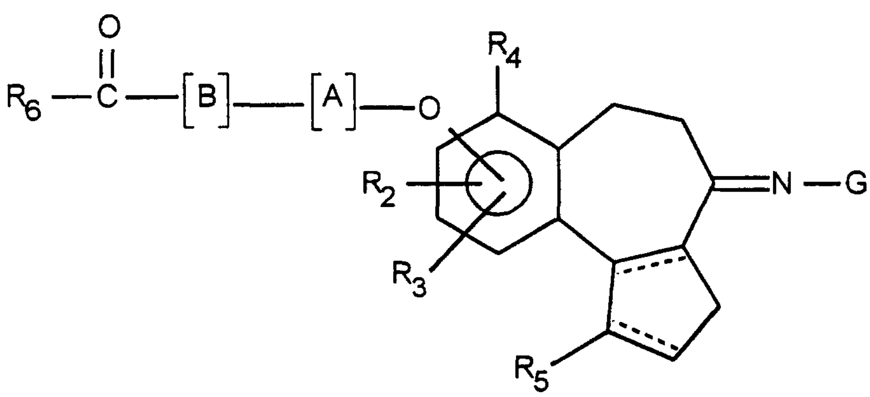

(IVa)

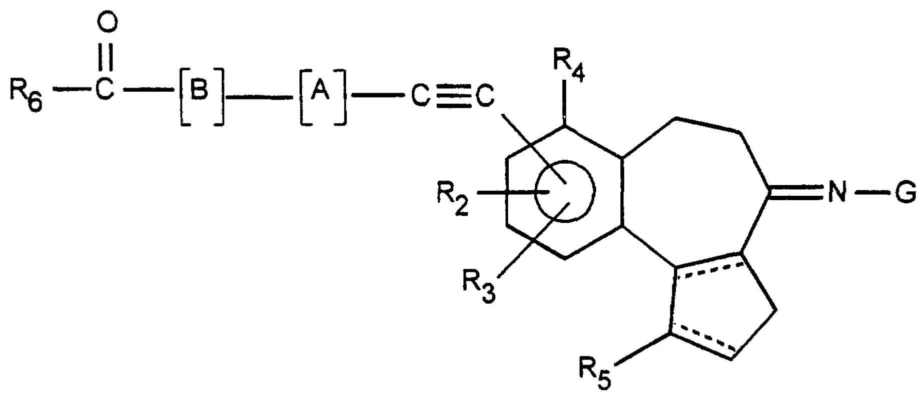

(IVb)

que l'on soumet le cas échéant, dans un ordre approprié,

- à l'action d'une base ou d'un acide afin de cliver l'ester et d'obtenir l'acide correspondant,
- à l'action d'un agent réducteur apte à réduire partiellement ou totalement les insaturations,
- à l'action d'un agent d'hydratation de la triple liaison,
- à l'action d'un agent de déalkylation,
- à l'action d'un agent de déprotection de la fonction NH-P en bêta de $CO-R_6$ lorsque [B] représente le groupe CH-NHP,
- à la formation du groupement $NH-SO_2R_c$, $NH-CO_2R_c$, $NHCOR_c$, $NH-SO_2-NH-R_c$, $NH-CO-NHR_c$ à partir de l'amine correspondante en bêta de $COR_6$, pour obtenir les composés de formule (I) correspondant que l'on soumet le cas échéant à l'action d'un acide ou d'une base afin d'obtenir les sels correspondants ou à l'action d'un agent d'estérification afin d'obtenir les esters correspondants.

L'action du composé de formule Hal-[A]-[B]-$COR_6$ (F1) est effectuée de préférence en présence d'une base minérale telle que le carbonate de potassium ou le carbonate de sodium en présence d'un solvant dipolaire aprotique tel que le diméthylformamide. Hal est de préférence un atome de chlore ou de brome.

L'action du composé de formule HO-[A]-[B]-$COR_6$ ($F'_1$) est effectuée en présence d'une phosphine telle que la triphénylphosphine et d'un agent tel que le diéthyl azodicarboxylate (DEAD) dans un solvant aprotique tel que le chlorure de méthylène.

L'action du composé de formule H-C≡C-[A]-[B]-$COR_6$ (F2) est précédée par celle d'un groupement activant tel que l'anhydride triflique de formule $(CF_3SO_2)_2O$ en présence d'une base telle que la pyridine afin de former le triflate correspondant de formule $(OSO_2CF_3)$ puis effectuée en présence d'un dérivé du palladium ($Pd^0$) tel que le $Pd(PPh_3)_4$.

L'action de $NH_2$-G (F3) s'effectue, soit sans solvant, soit dans un solvant alcoolique tel que l'éthanol ou le butanol. Le synthon $NH_2$-G est éventuellement utilisé sous la forme d'un sel tel que le chlorhydrate ou le bromhydrate.

La réaction de saponification de la fonction ester s'effectue par exemple par action d'une base alcaline telle que la soude ou la potasse dans le tétrahydrofuranne ou un alcool inférieur tel que le méthanol ou l'éthanol. On peut également cliver l'ester en milieu acide selon les méthodes connues de l'homme du métier.

La réduction des insaturations peut s'effectuer soit de manière totale par action d'hydrogène en présence d'un catalyseur tel que le palladium sur charbon ou un catalyseur au rhodium tel que le réactif de Wilkinson soit de manière partielle (alkynylène devient alkénylène) par action d'un catalyseur empoisonné tel que le palladium sur sulfate de baryum empoisonné par la pyridine ou la triéthylamine.

La réaction d'hydratation permettant d'accéder à un groupement $-CH_2CO$-[A]-[B]-$COR_6$ à partir de -C≡C-[A]-[B]-$COR_6$ s'effectue de préférence par action d'eau en présence de sulfate de mercure.

La réaction de déalkylation permettant d'accéder aux produits de formule (I) avec $R_2$, $R_3$, $R_4$ ou $R_5$ représentant des hydroxyles s'effectue en présence de chlorure d'aluminium ou de tribromure de bore.

La fonctionnalisation de $NH_2$ en alpha de $COR_6$, [B] représentant $CH-NH_2$ ou $CH-NH_2$, Hcl, s'effectue selon les méthodes classiques connues en chimie organique.

La formation de $NHSO_2R_c$ à partir de l'amine correspondante s'effectue de préférence par action de $R_cSO_2Hal$ en présence d'une base par exemple la triéthylamine.

La formation de $NHCO_2R_c$ à partir de l'amine correspondante s'effectue de préférence par action de $R_cOH$ selon la méthode décrite dans J. Org. Chem., <u>61</u>, 3929-3934 après avoir fait agir au préalable le triphosgène en présence de bicarbonate de sodium afin d'obtenir intermédiairement l'isocyanate.

Les réactions de salification peuvent être effectuées dans des conditions usuelles. On opère par exemple,

pour salifier le groupement terminal $CO_2H$ de $R_1$, en présence d'un sel de sodium tel que le carbonate de sodium ou le carbonate acide de sodium ou de potassium.

De même, la salification de l'amine ou de l'aminoguanidine que peuvent représenter G, par un acide, est réalisée dans les conditions usuelles. On opère par exemple avec l'acide chlorhydrique, par exemple en solution éthérée.

L'estérification éventuelle des produits est effectuée dans les conditions classiques connues de l'homme du métier.

On opère en général en faisant réagir l'acide de formule (I) ou un dérivé fonctionnel avec un réactif capable d'introduire le groupe ester dont une liste non exhaustive figure ci-dessus dans la définition de $R_6$.

Les produits de formule générale (F1), (F'1), (F2), (F3), sont connus ou préparés selon les méthodes connues de l'homme du métier.

On peut également inverser l'ordre de greffage des différents réactifs, à savoir on soumet le composé de formule (II) à l'action d'un composé de formule F3 afin d'obtenir intermédiairement le produit de formule (IIIc) :

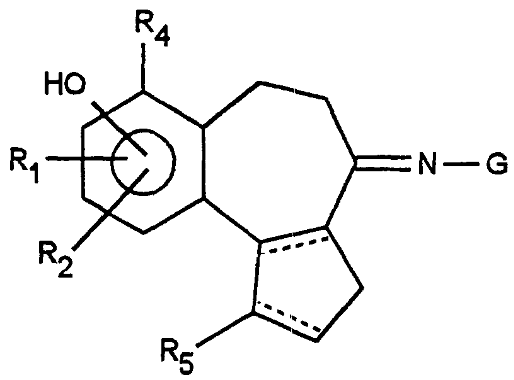

(IIIc)

que l'on soumet à l'action d'un composé de formule (F1), (F'1) ou (F2) afin d'obtenir les produits de formules (IVa) et (IVb) correspondants.

Dans ce cas, il faudra le cas échéant, prévoir une protection du groupement G du produit de formule (IIIc) puis, après introduction de (F1), (F'1) ou de (F2), une déprotection selon les méthodes connues de l'homme du métier (T.W. GREENE Protective Groups in Organic Synthesis. John Wiley and Sons Inc. 1991).

La réaction de déprotection du groupement NH-P en bêta de CO-$R_6$, [B] représentant le groupe CH-NHP, s'effectue également selon les méthodes connues de l'homme du métier, notamment lorsque P représente le groupe $CO_2tBu$, par une réaction de décarboxylation telle que par exemple par action de l'acide chlorhydrique.

L'os est constamment soumis à un processus dynamique qui inclut la résorption osseuse et la formation osseuse. Ces processus sont médiés via des cellules spécialisées. La formation osseuse est le résultat du dépôt d'une matrice minérale par les ostéoblastes et la résorption osseuse est le résultat de la dissolution de cette matrice osseuse par les ostéoclastes. L'ostéoporose est caractérisée par une perte sèche de cette matrice osseuse. Un ostéoclaste mature activé résorbe l'os après adhésion à la matrice osseuse via la sécrétion d'enzymes protéolytiques, et de protons à l'intérieur de la zone d'adhésion, aboutissant à des dépressions ou des creusements de la surface de l'os qui apparaissent au moment où l'ostéoclaste se détache de l'os.

Les composés de formule (I) ainsi que leurs sels d'addition pharmaceutiquement acceptables présentent d'intéressantes propriétés pharmacologiques. Ces composés inhibent la résorption osseuse qui est médiée via les ostéoclastes.

Les composés de l'invention sont ainsi utiles dans le traitement de maladies provoquées par la perte de la matrice osseuse, notamment, l'ostéoporose, l'hypercalcémie de malignité, l'ostéopénie due aux métastases osseuses, les parodontites, l'hyperparathyroidisme, les érosions periarticulaires dans l'arthrite rhumatoïde, la maladie de paget, l'ostéopénie induite par l'immobilisation, les traitements par les glucocorticoïdes ou les déficiences d'hormones sexuelles mâles ou femelles.

Ils peuvent également être utilisés pour le traitement des désordres inflammatoires, cancéreux et cardiovasculaires incluant l'athérosclérose, la resténose.

Ils peuvent enfin être utilisés comme inhibiteurs de l'angiogenèse et donc dans le traitement des tumeurs, par inhibition de leur néovascularisation, des rétinopathies diabétiques et des néphropathies.

Des études récentes ont montré que la fixation de l'ostéoclaste à l'os est médié par des récepteurs : les intégrines.

Les intégrines sont une superfamille de récepteurs médiant les processus d'adhésion cellule/cellule et plus particulièrement cellule/matrice, incluant notamment $\alpha$2b$\beta$3 comme récepteur des plaquettes sanguines (fibrinogène) et $\alpha$v$\beta$3 comme récepteur de la vitronectine, des sialoprotéines osseuses comme l'ostéopontine et la thrombospondine.

Ces récepteurs qui sont des hétérodimères protéiques composés de deux sous unités $\alpha$ et $\beta$, possèdent des sites de fixation d'ions divalents comme le $Ca^{2+}$ notamment et un site de reconnaissance de leur ligand prédéfini par la qualité de leurs sous unités.

Le récepteur $\alpha$v$\beta$3 est une glycoprotéine transmembranaire qui est exprimée dans un grand nombre de cellules incluant les cellules endothéliales, les cellules du muscle lisse, l'ostéoclaste et des cellules cancéreuses ce qui entraîne ainsi une pluripotentialité des composés selon l'invention.

Les récepteurs $\alpha$v$\beta$3 exprimés au niveau de la membrane des ostéoclastes sont à la base du processus d'adhésion/résorption, contribuent à l'organisation du cytosquelette cellulaire, et sont impliqués dans l'ostéoporose (Ross et al., J. Biol. Chem., 1987, 262, 7703).

Les récepteurs $\alpha$v$\beta$3 exprimés au niveau des cellules du muscle lisse de l'aorte, stimulent leur migration vers la neointima, ce qui entraîne la formation de l'athérosclérose et la survenue de resténose post-angioplastique (Brown et al, cardiovascular Res. (1994), 28, 1815).

Les cellules endothéliales secrètent des facteurs de croissance qui sont mitogènes pour l'endothélium et peuvent contribuer à la formation de nouveaux vaisseaux sanguins (Angiogenèse). La stimulation angiogénique provoque la formation de nouveaux vaisseaux sanguins.

Les antagonistes de l'intégrine $\alpha$v$\beta$3 peuvent ainsi entraîner une régression des tumeurs cancéreuses en induisant l'apoptose des vaisseaux sanguins angiogéniques. (Brook et al. Cell (1994) 79, 1157).

Les ligands naturels de l'intégrine $\alpha$v$\beta$3 contiennent tous le motif RGD (Arg-Gly-Asp). Les peptides contenant ce motif RGD ainsi que des anticorps anti $\alpha$v$\beta$3 sont connus pour leur capacité d'inhibition de la résorption de la dentine, d'empêchement de l'adhésion des ostéoclastes sur les matrices minéralisées (Horton et al. Exp. Cell. Res. (1991), 195, 368).

Le peptide Echistatine isolé du venin de serpent contenant également un motif RGD est décrit comme inhibiteur de l'adhésion des ostéoclastes à l'os, et est donc un puissant inhibiteur de la résorption osseuse dans les tissus en culture in vitro (Sato et al. J. Cell. Biol. (1990), 111, 1713) et in vivo chez le rat (Fisher et al. Endocrinology (1993), 132, 1441).

Les composés de formule (I) ainsi que leurs sels d'addition et leurs esters pharmaceutiquement acceptables peuvent posséder notamment une affinité vis-à-vis du récepteur de la vitronectine $\alpha$v$\beta$3 ou vis-à-vis d'autres intégrines ayant pour ligand la vitronectine ($\alpha$v$\beta$1, $\alpha$v$\beta$5, $\alpha$2b$\beta$3) en inhibant la liaison à leur ligand naturel.

Cette propriété rend ainsi les composés de l'invention utilisables pour la prévention ou le traitement de maladies dont la pathologie sous-jacente est provoquée par les ligands ou les cellules qui interagissent avec le récepteur de la vitronectine.

Ces composés peuvent posséder également une activité vis-à-vis d'autres intégrines qui interagissent avec leur ligand via la séquence tripeptidique RGD, leur conférant des propriétés pharmacologiques utilisables pour traiter les pathologies associées à ces récepteurs.

Cette activité vis-à-vis des intégrines rend ainsi les composés de l'invention utilisable dans le traitement de nombreuses maladies telles que celles mentionnées plus haut ou dans la revue de Dermot Cox DN&P 8(4) Mai 1995, 197-205 dont le contenu est intégré dans la présente demande.

L'invention a donc pour objet les composés de formule (I) à titre de médicaments, ainsi que leurs sels d'addition ou leurs esters pharmaceutiquement acceptables.

Parmi les médicaments de l'invention, on peut citer particulièrement les composés décrits dans la partie expérimentale.

Parmi ces produits, l'invention a plus particulièrement pour objet, à titre de médicaments, les composés de formule (I) listés précédemment.

La posologie varie en fonction de l'affection à traiter et de la voie d'administration : elle peut varier par exemple de 1 mg à 1000 mg par jour chez l'adulte par voie orale.

L'invention s'étend aux compositions pharmaceutiques renfermant comme principe actif au moins un médicament tel que défini ci-dessus.

Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparations injectables, de pommades, de crèmes, de gels, de microsphères, de nanosphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (II), dans laquelle le radical hydroxy est en position 10, $R_2$ en position 8 et $R_3$ en position 9, représentent un groupement O-(Alk) ou O-$(CH_2)_{0-3}$-Ar, $R_4$ et $R_5$ sont des atomes d'hydrogène, sont préparés selon la méthode décrite dans la demande de brevet européen n° 0729933 et dans la partie expérimentale ci-après (préparation 2).

Les deux autres isomères de position peuvent être préparés de la manière suivante :

On soumet un composé de formule (IIA) :

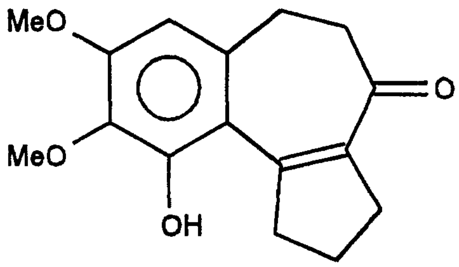

(IIA)

à l'action d'un réactif de déalkylation, afin d'obtenir le composé de formule (IIB) :

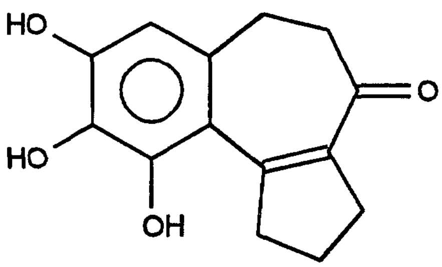

(IIB)

composé de formule (IIB) que l'on soumet :

**soit** à l'action d'un réactif de protection des diols en milieu basique, afin d'obtenir sélectivement le produit de formule (IIC) :

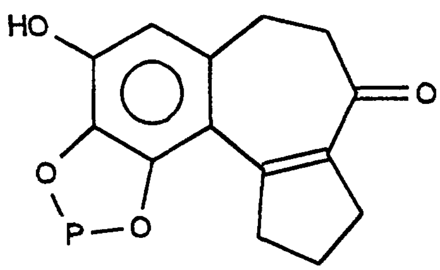

(IIC)

dans laquelle P représente le reste d'un réactif de protection des diols,

que l'on soumet successivement à l'action d'un réactif de protection du phénol, d'un réactif de déprotection des diols, d'un agent d'alkylation puis d'un agent de déprotection du phénol afin d'obtenir le composé de formule (IID) correspondant au produit de formule (II) trisubstitué avec OH en position 8 :

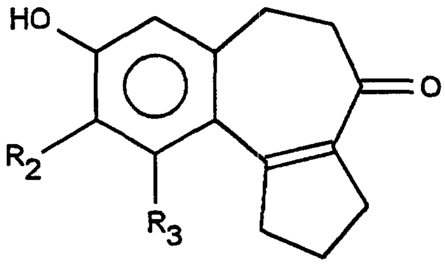

(IID)

**soit** à l'action successivement d'un agent de protection du phénol, d'un agent d'alkylation puis d'un agent de déprotection afin d'obtenir le composé de formule (IIE) correspondant au produit de formule (II) trisubstitué avec OH en position 9

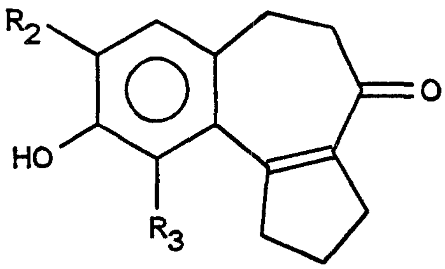

(IIE)

Par réactif de déalkylation, on entend de préférence des agents tels que le tribromure de bore ou le chlorure d'aluminium.

Le réactif de protection des diols que l'on fait réagir sur les produits de formule (IIB) peut être un dérivé du bore tel que l'acide borique, un borate de trialkyle, par exemple de triméthyle ou de triéthyle, ou encore le borax.

Par agent de protection du phénol, on entend notamment un halogénure tel que le chlorure ou le bromure de mésyle ou de tosyle ou encore un dérivé benzylé tel que le tosylate ou le mésylate de benzyle.

Par réactif de déprotection des diols, on entend notamment un acide fort tel que l'acide chlorhydrique, l'acide sulfurique ou bien l'acide paratoluène sulfonique ou encore un oxydant, par exemple l'eau oxygénée, dans le cas d'une protection par un dérivé du bore.

Par agent d'alkylation, on entend tout agent classique connu de l'homme du métier pour alkyler les phénols. On peut citer, par exemple un halogénure d'alkyle tel que le chlorure de méthyle ou d'éthyle, un sulfate d'alkyle tel que le sulfate de méthyle ou d'éthyle, ou encore le diazométhane.

Par agent de déprotection, on entend une base telle que la soude, la potasse ou encore le carbonate de sodium ou de potassium.

Les produits de formule (II) monosubstitués, dans lesquels $R_2$, $R_3$, $R_4$ et $R_5$ représentent un atome d'hydrogène, sont préparés selon une méthode analogue à celle décrite dans la demande de brevet européen n° 0729933 :

(i) On soumet un composé de formule (a) :

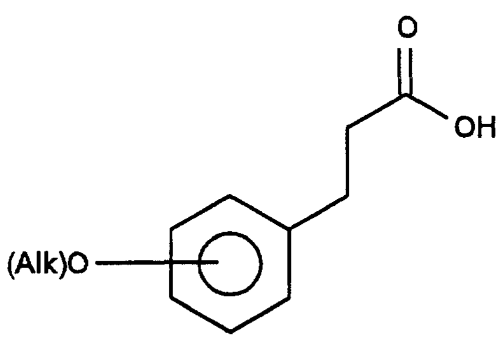

(a)

dans laquelle O-(Alk) est en position méta ou para du groupement alkylcarboxylique, (Alk) étant tel que défini précédemment, à l'action d'un agent d'halogénation pour obtenir l'halogénure d'acyle correspondant,

(ii) que l'on soumet à l'action d'un réactif de formule (b) :

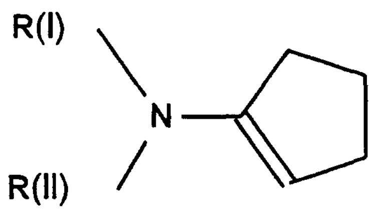

(b)

dans laquelle R(I) et R(II), identiques ou différents représentent un groupement alkyle renfermant de 1 à 6 atomes de carbone, ou R(I) et R(II) ensemble avec l'atome d'azote auquel ils sont liés, représentent un hétérocycle à 5 ou 6 chaînons, saturé ou insaturé, renfermant éventuellement un autre hétéroatome choisi parmi O et N,

pour obtenir un composé de formule (c) :

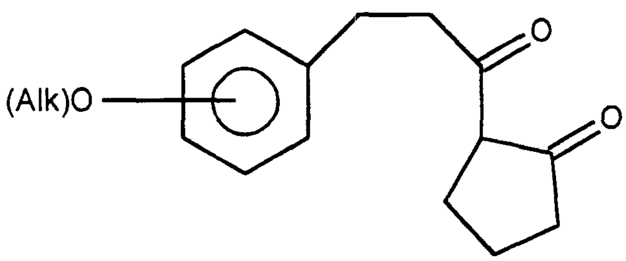

(c)

(iii) que l'on soumet à l'action d'un agent d'halogénation pour obtenir un composé de formule (d) :

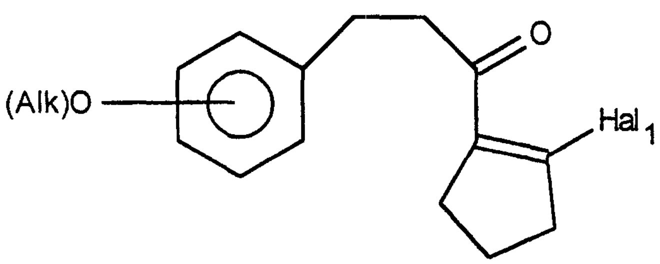

(d)

dans laquelle $Hal_1$ représente un atome d'halogène,

(iv) que l'on soumet à l'action d'un acide de Lewis, pour obtenir un composé de formule (e) :

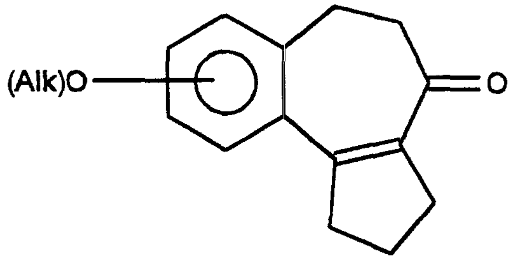

(e)

(v) que l'on soumet à un réactif de déalkylation afin d'obtenir le produit de formule (IIF) correspondant au produit de formule (II) monosubstitué attendu :

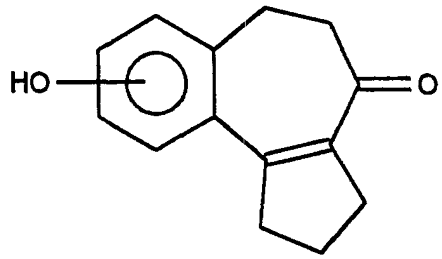

(IIF)

Les produits de formule (II) disubstitués, dans laquelle $R_2$ représente O-(Alk) ou O-$(CH_2)_{0-3}$-Ar, $R_3$, $R_4$ et $R_5$ sont des atomes d'hydrogène et OH et $R_2$ étant en position 8, 9 ou 10, sont préparés selon la méthode telle que décrite ci-dessus à partir du composé de formule (a') :

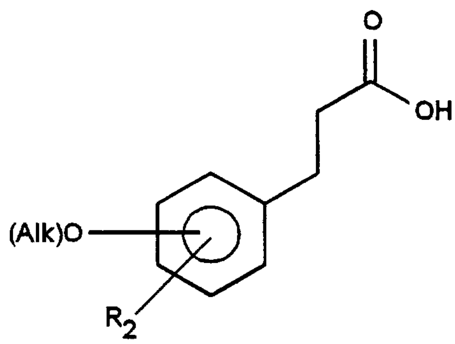

(a')

dans laquelle O-(Alk) et $R_2$ sont en position méta ou para de la chaîne alkyle carboxylique, $R_2$ étant un groupement O-(Alk) ou -$(CH_2)_{0-3}$-Ar, successivement aux réactions (i), (ii), (iii), (iv) et (v) et on obtient les produits de formule (IIG) correspondant aux produits de formule (II) bisubstitués attendus :

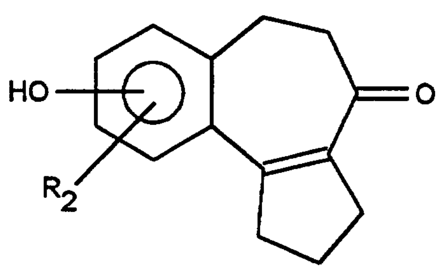

(IIG)

L'agent d'halogénation que l'on fait agir sur le composé de formule (a) ou (a') est par exemple le chlorure de thionyle, le chlorure d'oxalyle ou tout autre agent connu de l'homme du métier pour préparer un halogénure d'acide.
Le réactif de formule (b) est préparé au départ de la cyclopentanone et d'une amine secondaire, par exemple la diéthylamine, la pipéridine, la pipérazine ou, de préférence, la morpholine. On opère en présence d'un catalyseur acide fort, par exemple l'acide paratoluène sulfonique.
L'action de l'énamine de formule (b) sur l'halogénure d'acide est réalisée de préférence en présence d'une amine tertiaire telle que la triéthylamine ou la pyridine.
L'agent d'halogénation que l'on fait réagir sur le composé de formule (c), ou son équivalent disubstitué de formule (c'), peut être par exemple le chlorure de thionyle, le phosgène, l'oxychlorure de phosphore ou, de préférence, le chlorure d'oxalyle.

L'acide de Lewis utilisé pour cycliser le composé de formule (d), ou son équivalent disubstitué de formule (d') est par exemple le chlorure d'aluminium, le tétrachlorure de titane, ou de préférence le chlorure ferrique, ou le tétrachlorure d'étain. La réaction, comme celles qui précèdent, peut être conduite, par exemple, dans un solvant halogéné tel que le chlorure de méthylène, le chloroforme ou le dichloroéthane.

Le réactif de déalkylation du composé de formule (e), ou son équivalent disubstitué de formule (e') afin d'obtenir les phénols correspondant est de préférence le chlorure d'aluminium ou le tribromure de bore.

Les produits de formule (II) dans laquelle $R_4$ est différent de l'atome d'hydrogène, sont préparés par des méthodes classiques de substitution électrophile et nucléophiles aromatiques connues de l'homme du métier.

Les produits de formule (II) dans laquelle $R_5$ est différent de l'atome d'hydrogène sont préparés selon les méthodes connues de l'homme du métier et notamment selon la méthode décrite dans la demande de brevet européen n° 0729933, c'est-à-dire par halogénation puis action de l'eau ou d'un alcool approprié.

Les produits de formule (II) dans laquelle $R_5$ est un atome d'hydrogène et dans laquelle il y a une double liaison en position 1-2 sont préparés selon les méthodes connues de l'homme du métier et notamment selon la méthode décrite dans la demande de brevet européen n° 0729933, c'est-à-dire par déshydratation ou désalcoxylation en milieu acide anhydre.

Les produits de formule (II) dans laquelle la jonction entre le cycle à 5 et le cycle à 7 est saturée sont préparés selon les méthodes classiques d'hydrogénation notamment en présence de palladium sur charbon de la double liaison correspondante.

L'introduction de $R_4$, $R_5$ ainsi que la réaction d'hydrogénation s'effectue de préférence sur les composés de formule (IIA), (IID), (IIE), (IIF) ou (IIG).

Les produits de formule (II) dans lesquels $R_2$ et $R_3$, en position ortho l'un de l'autre forment un cycle du type $-O-(CRdRe)_n-O$ tel que défini précédemment, sont également préparés selon les méthodes connues de l'homme du métier et notamment selon la méthode décrite ci-après dans la partie expérimentale.

L'invention a également pour objet, à titre de produits intermédiaires, les produits de formule (IIIa), (IIIb), (IIIc) et (II) étant entendu que les composés de formule (IIc) et les composés suivants :

- 2,3,5,6-tétrahydro-9,10-diméthoxy-8-hydroxy-benz[e]azulèn-4(1H)-one,
  et 2,3,5,6-tétrahydro-8,9-diméthoxy-10-hydroxy-benz[e]azulèn-4 (1H) -one,
  sont exclus. La préparation de ces 2 composés figure ci-après dans la partie expérimentale.

Les exemples suivants illustrent l'invention sans toutefois la limiter.

**PREPARATION 1 : 2,3,5,6-tétrahydro-8,9,10-trihydroxy-benz[e] azulen-4(1H)-one**

Stade A : Acide-3,4,5-triméthoxy-benzènepropanoïque

On ajoute 6,8 g de carbonate de potassium à une solution de 21,44 g d'acide 3,4,5-triméthoxyphénylepropénoïque et 45 ml d'eau puis on hydrogène pendant une heure sous une pression de 1200-1300 mbar en présence de 1,8 g de charbon actif à 10 % de palladium, on absorbe ainsi 2,1 l d'hydrogène. On filtre, lave à l'eau et acidifie avec 50 ml d'acide chlorhydrique (2N). On essore, lave à l'eau et sèche sous pression réduite à température ambiante. On obtient ainsi 19,8 g du produit attendu (P.F. = 102-103°C).

Spectre I.R. ($CHCl_3$)

Carbonyle : {1712 $cm^{-1}$ (max) aromatique: {1592 $cm^{-1}$
{1740 $cm^{-1}$ (ép) {1510 $cm^{-1}$

Spectre R.M.N. ($CDCl_3$)

2,69(t)} $=C-CH_2-CH_2-CO$ 3,83(s)} 3 $H_3CO-C-$
2,91(t)} | 3,85(s)}

6,43(s) 2H aromatiques
10,50(m) 1H mobile

Stade B : Chlorure de 3,4,5-triméthoxy-benzenepropanoyle

On sèche avec 1,5 g de sulfate de magnésium une solution de 6 g du produit obtenu au stade A dans 21 ml de chlorure de méthylène, après filtration on refroidit à 5°C et ajoute 2,2 ml de chlorure de thionyle puis agite la solution 20 heures à température ambiante. On évapore à sec sous pression réduite en procédant à deux entraînements avec du cyclohexane on recueille ainsi 6,46 g du produit recherché. (P.F. = 60°C) Stade C : 2-[3-(3,4,5-triméthoxyphényl)-1-oxopropyl]-cyclopentanone

A une solution refroidie à 5°C de 2,4 ml de 1-(N-morpholinyl)cyclopentène obtenu comme décrit ci-après, 2,31 ml de triéthylamine et 15 ml de chlorure de méthylène on ajoute en 1 heure 30 à +5°C une solution de 4,27 g du produit obtenu au stade B dans 15 ml de chlorure de méthylène. On agite 1 heure à +5°C puis en laissant la température remonter on ajoute 10 ml d'acide chlorhydrique 2 N, agite 1 heure à température ambiante, décante, lave à l'eau puis avec une solution saturée de bicarbonate de sodium, sèche, filtre et évapore à sec sous pression réduite. On obtient 5 g du produit attendu. On purifie le produit brut par dissolution dans 10 volumes d'acétate d'éthyle, extrait avec une solution de soude N, lave la phase alcaline avec de l'acétate d'éthyle, on acidifie à pH 1 avec de l'acide chlorhydrique concentré, extrait avec du chlorure de méthylène, sèche et évapore à sec sous pression réduite. On recueille 2,75 g de produit purifié.

Spectre I.R. ($CHCl_3$) :
Carbonyle: {1741 $cm^{-1}$ aromatique: {1592 $cm^{-1}$
{1709 $cm^{-1}$ {1509 $cm^{-1}$
Carbonyle: {1658 $cm^{-1}$
+ C=C {~1610 $cm^{-1}$ avec OH sous forme chélatée

Spectre R.M.N. ($CDCl_3$)
6,41(s) 2H arom. (base d'intégration)
3,81(s) 3,82(s)} 9H en tout
3,83(s) 3,85(s)} 4 types de $CH_3O$-C=

1,86(m) $CH_2$-**$CH_2$**-$CH_2$ ~1,5H
1,95 à 2,95(m) ~7,5H en tout dont =C-$CH_2$ de plusieurs types
3,26(t) ~0,4H CO-**CH**-$CH_2$
|
CO
11,2(m large) H mobile

**Préparation de 1-(N-morpholinyl)-cyclopentène utilisé au stade C :**

On agite pendant 4 heures 30 au reflux, en éliminant l'eau formée, une solution de 100 ml de cyclohexane, 20 ml de cyclopentanone, 50 ml de morpholine et 100 mg d'acide paratoluène sulfonique. Après évaporation du solvant sous pression réduite, on distille sous 12 - 13 mbar de pression et recueille 27,44 g de produit recherché (Eb. = 83°C).

Stade D : 1-(2-chloro-1-cyclopenten-1-yl)-3-(3,4,5-triméthoxyphényl)-propan-1-one

A une solution de 23 g de produit obtenu au stade C et 230 ml de chloroforme, on ajoute à température ambiante 13 ml de chlorure d'oxalyle. On agite trois heures à température ambiante, on concentre à pression réduite en procédant à deux entraînements au cyclohexane. On obtient 28 g de produit brut que l'on recristallise dans un mélange de 50 ml de cyclohexane et 50 ml d'éther diisopropylique après concentration partielle. On essore, lave avec de l'éther diisopropylique et sèche sous pression réduite. On obtient 16,24 g du produit attendu. (P.F. = 93°C)

| Spectre I.R. ($CHCl_3$) : | |
|---|---|
| 1659 $cm^{-1}$ :<br>1599 $cm^{-1}$ | Carbonyle |
| 1586 $cm^{-1}$ :<br>1508 $cm^{-1}$ | C=C + aromatique |

Spectre R.M.N. $CDCl_3$

1,93(m) : $CH_2$ central
2,69(m)-2,81(m) : C-**$CH_2$**-C= du cyclopentène
2,85(t,j=7,5) - 3,08(t,j=7,5) : les autres =C-**$CH_2$**-C 2,44 : **$CH_3$**-C= 3,68 - 3,81 : les $OCH_3$
6,59-6,68(d,j=2) : les CH= aromatiques couplés méta
7,31-7,80(d,j=8) : les aromatiques.

Stade E : 2,3,5,6-tétrahydro-8,9,10-triméthoxy-benz[e]azulèn-4(1H)-one

On agite 20 heures à température ambiante 900 mg du produit obtenu au stade D, 9 ml de 1,2-dichloroéthane et 0,9 ml de chlorure stannique. On ajoute ensuite 9 ml d'eau et glace et décante, lave à l'eau, réextrait une fois avec du chlorure de méthylène, sèche sur sulfate de magnésium, filtre et évapore à sec sous pression réduite, pour obtenir 1 g du produit attendu (brut) que l'on purifie par chromatographie sur silice en éluant avec du cyclohexane à 10 % d'acétate d'éthyle, puis à 25 % d'acétate d'éthyle. Après concentration on recueille 700 mg de produit que l'on cristallise dans 5 ml de n-hexane, puis refroidit à 0°C, essore, lave avec le minimum de n-hexane, sèche sous pression réduite à température ambiante pour obtenir 630 mg de produit attendu.
(P. F. = 101-102°C).

Spectre RMN ($CDCl_3$)

| | | | |
|---|---|---|---|
| 1,86(m) | le $CH_2$ central | | |
| 2,65(dd) | 2H } | 3,84} | |
| 2,72(t) | 2H } les autres $CH_2$ | 3,86} | Les OMe |
| 2,84(dd) | 2H } | 3,90} | |
| 3,06 | 2H } | | |
| 6,59(s) | H aromatique | | |

Stade F : 2,3,5,6-tétrahydro-8,9,10-trihydroxy-benz[e]azulèn-4(1H)-one

En opérant comme au stade B de la préparation ibis, on obtient le produit déméthylé attendu.

**PREPARATION 1bis : 2,3,5,6-tétrahydro-8,9,10-trihydroxybenz[e]azulen-4(1H)-one**

Stade A : 2,3,5,6-tétrahydro-8,9,10-triméthoxy-benz[elazulèn-4(1H)-one

On agite pendant 2 heures 30 à 20°C, 60 g du produit obtenu à la préparation 2, 600 ml de 1,2-dichloroéthane, 342 ml de soude 2N, 1,2 g de bromure de tétrabutylammonium et 33 ml du sulfate diméthylique. On introduit alors, 39 ml de triéthylamine afin de détruire l'excès de sulfate diméthylique et agite une heure à 20°C ± 2°C. On ajoute 342 ml

d'eau déminéralisée, agite 15 minutes à 20°C ± 2°C, décante, réextrait la phase aqueuse deux fois avec à chaque fois 120 ml de 1,2-dichloroéthane. Les phases 1,2-dichloroéthane sont réunies et lavées par 4 x 240 ml d'eau déminéralisée, puis par 1 x 300 ml d'acide chlorhydrique N, puis par 3 x 240 ml d'eau déminéralisée (jusqu'à la neutralité). Les phases organiques réunies sont séchées sur sulfate de sodium, filtrées et concentrées à pression ordinaire à 83°C jusqu'à un volume résiduel de 480 ml.

Stade B : 2,3,5,6-tétrahydro-8,9,10-trihydroxy-benz[e]azulèn-4(1H)-one

On chauffe au reflux, pendant une heure, 480 ml de la solution obtenue en (A) avec 102,3 g de chlorure d'aluminium anhydre. On refroidit le milieu à 0°C ± 2°C puis ajoute en deux heures un mélange de 600 ml d'eau déminéralisée et 192 ml d'acide sulfurique pur (concentré) préalablement refroidi vers 0°C en maintenant la température du milieu réactionnel inférieure à 20°C. On introduit, en 5 minutes à 20°C ± 2°C, 300 ml d'eau déminéralisée et agite 16 heures à 20°C ± 2°C, essore, lave deux fois avec à chaque fois 60 ml de 1,2-dichloroéthane, puis à l'eau déminéralisée, sèche sous pression réduite et obtient 52,2 g du produit recherché.

**PREPARATION 2 : 8,9-diméthoxy-10-hydroxy-2,3,5,6-tétrahydrobenz[e]azulen-4(1H)-one**

Stade A : Acide 3,4-diméthoxy 5-[[(4-méthylphényl)-sulfonyl]oxy]-benzenepropanoique

On opère comme au stade A de la préparation 1 en utilisant 29,76 g de l'acide 3,4-diméthoxy-5-[[[(4-méthylphényl)sulfonyl]oxy]phényl]-cinnamique dont la préparation est donnée ci-après, 43,5 g de carbonate de potassium, 60 ml de méthanol et 1,48 g de charbon actif palladié à 10 %. On obtient ainsi 28,23 g du produit recherché sous forme de cristaux incolores (P. F. = 148-149°C).

| Spectre U.V. (EtOH) | |
|---|---|
| Pour | M = 380,4 |
| max 226nm | ε= 22100 |
| infl 263nm | ε= 2000 |
| infl 269nm | ε= 2400 |
| max 274nm | ε= 2800 |
| infl 279nm | ε= 2500 |
| infl 307nm | ε= 450 |

| Spectre R.M.N. ($CDCl_3$) | |
|---|---|
| 2,45(s) $CH_3$- | |
| 2,61(m) =C-$CH_2$-$CH_2$-C= | 3,68(s) 2 $CH_3O$-C= |
| 2,86(m) | 3,81(s) |
| 6,61(d,j=2) | 7,32(dl) $H_3$ $H_5$ |
| 6,65(d,j=2) $H_4$ $H_6$ | 7,80(dl) $H_2$ $H_6$ |

Stade B : Chlorure de 3,4-diméthoxy 5-[[(4-méthylphényl)sulfonyl]oxy]-benzènepropanoyle

On opère comme au stade B de la préparation 1 en utilisant 1,9 g du produit obtenu au stade A, 9,5 ml de chlorure de méthylène et 0,7 ml de chlorure de thionyle. On obtient 2,24 g du produit recherché utilisé tel quel pour le stade suivant.

Stade C : 2-[3-[3,4-diméthoxy-5-[[(4-méthylphényl)sulfonyl]oxy]-phényl]-1-oxopropyl]-cyclopentanone

On opère comme au stade C de la préparation 1 à partir de 2,24 g du chlorure d'acide obtenu au stade B et en utilisant 770 mg de 1-(N-morpholinyl)-cyclopentène (préparé au stade C de la préparation 1), 6 ml de chlorure de méthylène et 0,77 ml de triéthylamine. Après recristallisation dans l'éther diisopropylique on obtient 1,27 g du produit recherché
(P. F. = 84°C).

Spectre I.R. ($CHCl_3$)

Carbonyle : {1742 $cm^{-1}$ O-$SO_2$ {1374 $cm^{-1}$

{1709 $cm^{-1}$ 1178 $cm^{-1}$ 1658 $cm^{-1}$

C=C + aromatique {1608 $cm^{-1}$ 1599 $cm^{-1}$ 1586 $cm^{-1}$ 1508 $cm^{-1}$

Spectre R.M.N ($CDCl_3$)

2,44(s) **$CH_3$**-O

3,67(s) } 2 $OCH_3$

3,79(s) 3,81(s) }

6,59 à 6,65(m) 2H arom. en ortho des O.

7,32(dl) $H_3$ $H_5$

7,89(dl) $H_2$ $H_6$

13,58(m large) OH forme énol

1,8 à 3,4(m) 10 à 11 H autres protons

Spectre U.V.

**1** - EtOH (+ dioxane) pour M = 446,52

max 225nm $\epsilon$= 23000

max 282nm $\epsilon$= 7900

infl 270, 277, 290, 300, 313 nm

**2** - EtOH (NaOH 0,1N)

max 310 nm $\epsilon$= 21600

infl 268, 272, 276 nm

Stade D : 1-(2-chloro-1-cyclopenten-1-yl)-3-[3,4-diméthoxy-5-[[(4-méthylphényl)sulfonyl]oxy]-phényl]-propan-1-one

On opère comme au stade D de la préparation 1 en utilisant 8,7 g du produit obtenu au stade C, 70 ml de chloroforme et 3,5 ml de chlorure d'oxalyle. Après cristallisation dans l'éther diisopropylique on obtient 7,75 g du produit recherché (P. F. = 73°C). Ce produit est utilisé tel quel pour le stade suivant.

Un échantillon analytique a été obtenu par recristallisation dans 2,5 volumes de chlorure de méthylène et 5 volumes d'éther diisopropylique suivie par concentration à 3 volumes, essorage, lavage à l'éther diisopropylique et séchage sous pression réduite à température ambiante (P.F. = 77 - 78°C).

Spectre I.R. ($CHCl_3$)
Carbonyle : {1659 $cm^{-1}$
C=C aromatique : {1599 $cm^{-1}$ - 1586 $cm^{-1}$ - 1508 $cm^{-1}$
Spectre U.V. (EtOH)
max 227nm $\epsilon$= 26100
infl 248nm $\epsilon$= 12800
infl 272nm $\epsilon$= 5300
infl 280nm $\epsilon$= 3200
infl 320nm

Spectre R.M.N. ($CDCl_3$)
1,93(m) -C-**$CH_2$**-C- central }
2,69(m)} les C-**$CH_2$**-C= }
2,81(m)} }
2,85(t,j=7,5)} les autres =C-**$CH_2$**-C
3,08(t,j=7,5)}

2,44 **$CH_3$**-C=
3,68} les $OCH_3$
3,81}
6,59 (d,j=2) les CH aromatiques
6,68 (d,j=2) couplés méta
7,31 (d,j=8)}
7,80 (d,j=8)}

Stade E : 8,9-diméthoxy-10-[[(4-méthylphényl)sulfonyl]oxy]2,3,5,6-tétrahydro-benz[e]azulèn-4(1H)-one

A une solution de 2,32 g du produit obtenu au stade C dans 50 ml de 1,2-dichloroéthane on ajoute à température ambiante 1,65 g de chlorure ferrique à 98 %. On agite 48 heures à température ambiante puis coule sur un mélange d'eau et glace, agite énergiquement pendant 15 mn et extrait avec du chlorure de méthylène, lave à l'eau, puis avec une solution aqueuse saturée de chlorure de sodium. Après séchage et évaporation à sec sous pression réduite on obtient 2,15 g du produit brut que l'on chromatographie en éluant avec du cyclohexane à 50 % d'acétate d'éthyle, on recueille 1,8 g de produit que l'on chromatographie à nouveau et recristallise dans le mélange chloroforme/éther diisopropylique pour obtenir 720 mg du produit recherché (P.F. = 138°C).

Spectre I.R. ($CHCl_3$)
Carbonyle : {1650 $cm^{-1}$
{1599 $cm^{-1}$
C=C + {1556 $cm^{-1}$
aromatique {1512 $cm^{-1}$ - 1498 $cm^{-1}$
Spectre U.V. (EtOH)
max 230nm ε= 25300
infl 254nm ε= 9400
max 323nm ε= 10300

Spectre R.M.N. ($CDCl_3$)
~1,61(m) (2H) $CH_2$ central
~2,41 Ph-$CH_3$
~2,50 à 2,80 $CH_2$-C=
|
3,88(s)} les $OCH_3$
3,90(s)}

6,74 H4
7,21(d)} C-Ph-$SO_2$
7,64(d)}

Stade F : 8,9-diméthoxy-10-hydroxy-2,3,5,6-tétrahydro-benz [e]azulèn-4(1H)-one

On chauffe au reflux pendant 2 heures un mélange de 350 g du produit obtenu au stade E ci-dessus, 1750 ml de méthanol, 350 ml d'eau déminéralisée et 350 ml de lessive de soude pure (concentrée). On refroidit le milieu réactionnel vers 2°C ± 2°C et introduit en 45 minutes 467 ml d'acide chlorhydrique concentré en maintenant la température à 2°C ± 2°C. On ajoute alors 1645 ml d'eau déminéralisée en 10 mn et en maintenant la température à 2°C ± 2°C, puis le milieu réactionnel est agité 30 minutes toujours à 2°C ± 2°C. On essore les cristaux formés, lave par clairçages à 5 reprises avec à chaque fois 700 ml d'eau déminéralisée à 20°C puis sèche à 40°C sous pression réduite pour obtenir 199,1 g du produit recherché.

**Préparation de l'Acide 3,4-diméthoxy 5-[[(4-méthylphényl)sulfonyl]oxy]-cinnamique** utilisé au départ de la préparation 2.

Stade A : 3,4-diméthoxy-5-[[(4-méthylphényl)-sulfonyl]oxy]benzoate de méthyle.

On ajoute en 10 minutes à température ambiante 303 ml de triéthylamine à un mélange agité de 200 g de gallate de méthyle et 2 litres de chlorure de méthylène. Après dissolution on refroidit à 0-5°C puis ajoute en 1 heure à cette température 130 ml de dichlorodiméthylsilane, agite encore 30 minutes à cette température. En maintenant la température à 0-5°C on ajoute en 25 minutes 303,2 ml de triéthylamine puis en 15 minutes 227,6 g de chlorure de tosyle. On agite encore une heure à 0-5°C, et ajoute en 10 minutes sous agitation et en laissant évoluer la température jusqu'à 20-22°C, 200 ml d'acide acétique, puis 500 ml d'eau déminéralisée, on agite encore 15 minutes à 20°C. On distille le chlorure de méthylène à volume constant (3,3 l) sous pression réduite en remplaçant par de l'eau déminéralisée, on agite 2 heures à 20°C, essore, lave avec de l'eau déminéralisée pour obtenir 523 g (poids humide) de 3,4-dihydroxy-5-[[(4-méthylphényl)sulfonyl]oxy]-benzoate de méthyle (3-tosylgallate de méthyle). Le produit humide obtenu est repris par 2,17 l de soude (2N) et 2,17 l de chlorure de méthylène. On agite à 20°C jusqu'à dissolution puis ajoute à 20°C, 18 g de bromure de tétrabutylammonium puis, en 15 minutes à 20°C, 237 ml de sulfate diméthylique. Le milieu réactionnel est agité 1,5 heure à 20-22°C. On ajoute, à 20-22°C, 78 ml de triéthylamine et agite une nuit à 20-22°C, puis décante et lave avec 400 ml d'eau déminéralisée et ajoute 20 ml d'acide acétique pur à la phase organique agite 15 minutes, ajoute 400 ml d'eau déminéralisée, puis décante. On concentre à sec les phases organiques réunies, d'abord à pression atmosphérique puis sous pression réduite à 40 mm Hg et 60°C extérieur. On entraîne avec 400 ml de méthanol puis, reprend l'extrait sec obtenu avec 600 ml de méthanol chauffe au reflux jusqu'à dissolution totale du produit, puis refroidit à 0-5°C agite une heure à cette température. On essore et lave par deux fois avec 200 ml de méthanol à -10°C et sèche à 40°C sous pression réduite on recueille ainsi 330,4 g de : 3,4-diméthoxy-5-[[(4-méthylphényl)-sulfonyl]oxy]-benzoate de méthyle. Le produit brut est purifié par recristallisation dans 330 ml de toluène. Après 2 heures d'agitation à -10°C on essore, lave par deux fois 82 ml de toluène refroidi à -15°C et sèche sous pression réduite à 40°C pour obtenir 230,3 g du produit purifié recherché.

Stade B : acide 3,4-diméthoxy 5-[[(4-méthylphényl)sulfonyl] oxy] -cinnamique

**a)** On refroidit à 0°C, 600 ml de toluène et ajoute 202 ml d'une solution de Vitride® à 70 % dans le toluène à 0°C et ajoute en une heure 67,6 ml de morpholine à 0-2°C, on laisse remonter la température jusqu'à 18°C. On utilise la solution ainsi obtenue immédiatement pour l'étape suivante.

**b)** On agite 10 minutes à 20-22°C, 200 g de 3,4-diméthoxy-5-[[(4-méthylphényl)-sulfonyl]oxy]-benzoate de méthyle obtenu au stade A et 1400 ml de toluène jusqu'à dissolution totale. On ajoute en une heure à 10°C la solution du réactif obtenue ci-dessus. On agite encore une heure en laissant la température remonter à 18°C.
On introduit en une heure, à 10°C, une solution refroidie à 10°C de 200 ml d'acide sulfurique concentré et 1000 ml d'eau déminéralisée. On agite 16 heures à 20°C puis décante la phase organique, lave par 5 x 200 ml d'eau déminéralisée, sèche, filtre et lave par 3 x 100 ml de chlorure de méthylène. La solution d'aldéhyde intermédiaire ainsi obtenue est utilisée telle quelle à l'étape suivante.

**c)** On chauffe 16 heures à 70°C ± 2°C (en éliminant, à pression ordinaire, le chlorure de méthylène) la solution d'aldéhyde intermédiaire obtenue ci-dessus, 200 ml de 2-picoline, 120 g d'acide malonique et 20 ml de pipéridine. On refroidit à 20-22°C, et en maintenant cette température on ajoute en 15 minutes une solution de 200 ml d'acide chlorhydrique concentré et 400 ml d'eau déminéralisée. On agite 2 heures à 20-22°C, puis refroidit à 0°C, essore les cristaux formés, lave à l'eau déminéralisée, sèche sous pression réduite à 40°C pour obtenir 171,7 g de l'acide 3,4-diméthoxy 5-[[(4-méthylphényl)-sulfonyl]oxy}phényl]-cinnamique attendu.

**PREPARATION 3 : 9,10-diméthoxy-8-hydroxy-2,3,5,6-tétrahydrobenz[e]azulen-4(1H)-one**

Stade A : 9,10-dihydroxy-8-[[(4-méthylphényl)sulfonyl]oxy]2,3,5,6-tétrahydro-benz[e]azulèn-4(1H)-one

On agite pendant 1 heure 30 à 20°C ± 2°C, 30 g de 2,3,5,6-tétrahydro-8,9,10-trihydroxy-benz[e]azulèn-4(1H)-one obtenu selon la préparation 1 ou 1bis, 300 ml de tétrahydrofuranne, 60 ml de triéthylamine et 12,9 ml de triméthylborate. On ajoute 30 g de chlorure de tosyle et agite 16 heures à 20°C ± 2°C puis en 10 minutes à 20°C ± 2°C verse le milieu réactionnel sur un mélange agité de 900 ml d'eau déminéralisée et 150 ml d'acide chlorhydrique concentré, puis on ajoute 90 ml de tétrahydrofuranne et 60 ml de chlorure de méthylène. On agite la solution obtenue une heure à 20°C, puis introduit 150 ml de chlorure de méthylène et agite encore 15 minutes, décante et réextrait par 2 x 75 ml de chlorure de méthylène. Les phases organiques réunies sont lavées avec 4 x 150 ml d'eau déminéralisée et réextraites par 75 ml de chlorure de méthylène, après concentration sous pression réduite de 20 mbars jusqu'à refus de distillation à 50°C pour obtenir 47,6 g du produit recherché.

Stade B : 9,10-diméthoxy-8-[[(4-méthylphényl)sulfonyl]oxy]2,3,5,6-tétrahydro-benz[e]azulèn-4(1H)-one

On agite 16 heures à 20°C, 47,6 g du produit obtenu ci-dessus, 300 ml de chlorure de méthylène, 300 ml de soude (2N), 0,6 g du bromure de tétrabutylammonium et 30 ml de sulfate diméthylique. On introduit alors 30 ml de triéthylamine afin de détruire l'excès de sulfate diméthylique, le milieu réactionnel est agité encore une heure à 20°C ± 2°C, puis ajoute 150 ml d'eau déminéralisée agite encore 15 minutes puis décante. La phase aqueuse était réextraite avec 2 x 75 ml de chlorure de méthylène et les phases organiques réunies sont lavées par 3 x 120 ml d'eau déminéralisée puis 120 ml d'acide chlorhydrique N et 3 x 120 ml d'eau déminéralisée, les phases organiques sont réunies et

séchées sur sulfate de sodium, puis on ajoute en 1 heure 120 g de gel de silice (60 Mesh) à 20°C ± 2°C sous agitation et agite encore une heure à 20°C, filtre, lave avec du chlorure de méthylène et concentre à sec sous pression réduite à 50°C pour obtenir 47,4 g du produit recherché.
On purifie le produit brut par recristallisation dans 390 ml d'éthanol après distillation de 90 ml d'éthanol, on agite 3 heures à 0°C ± 2°C. On essore, lave avec 30 ml d'éthanol à 0°C, puis sèche sous pression réduite à 40°C pour obtenir 41,1 g du produit recherché (P.F. = 129°C).

Stade C : 9,10-diméthoxy 8-hydroxy 2,3,5,6-tétrahydrobenz[e]azulèn-4(1H)-one

On ajoute 4,5 g de potasse puis 10 ml de triéthylamine dans une suspension comprenant 10 g de 9,10-diméthoxy 8-(((4-méthylphényl) sulfonyl) oxy) 2,3,5,6-tétrahydro-benz[e]azulèn-4(1H)-one obtenu comme au stade B et 100 ml de méthanol. On chauffe 1 heure au reflux, acidifie par addition de 20 ml d'acide acétique puis ajoute 20 ml d'eau. On extrait au dichlorométhane, lave à l'eau, évapore le solvant à 40°C sous pression réduite et recueille 5,7 g de produit attendu.

**PREPARATION 4 : 2,3,5,6-tétrahydro-8-hydroxy-9-méthoxybenz[e]azulen-4(1H)-one et 2,3,5,6-tétrahydro-9-hydroxy-8-méthoxy-benz[e]azulen-4(1H)-one**

On opère de manière équivalente à la préparation 2 stades B, C, D, E et F mais à partir d'acide 3-(3,4-diméthoxy-phényl) propionique, et on obtient 1,08 g de produit brut renfermant un mélange de produit monohydroxylé (8-OH/9-OMe et 9-OH/8-OMe) que l'on sépare par chromatographie sur silice en utilisant comme mélange éluant le mélange cyclohexane/acétate d'éthyle 7/3. On obtient ainsi les deux régioisomères suivants :
8-OH/9-OMe 0,494 g Rf (cyclohexane/acétate d'éthyle 6/4)=0,42 8-OMe/9-OH 0,041 g Rf (cyclohexane/acétate d'éthyle 6/4)=0,33

**PREPARATION 5 : 2,3,5,6-tétrahydro-8-hydroxy-10-méthoxybenz[e]azulen-4(1H)-one et 2,3,5,6-tétrahydro-10-hydroxy-8-méthoxy-benz [e] azulen-4 (1H)-one**

On opère de manière équivalente à la préparation 2 stades B, C, D, E et F mais à partir d'acide 3-(3,5-diméthoxy-phényl) propionique, et on obtient 1,428 g d'un produit renfermant un mélange des produits monohydroxylés (8-OH/10-OMe et 10-OH/8-OMe) et dihydroxylés (8-OH/10-OH), que l'on sépare par chromatographie sur silice en utilisant comme mélange éluant le mélange cyclohexane/acétate d'éthyle 7/3.

**PREPARATION 6 : 2,3,5,6-tétrahydro-9-hydroxy-benz[e]azulen-4(1H)-one**

On opère de manière équivalente à la préparation 2 stades B, C, D, E et F mais à partir de l'acide 3-(4-méthoxyphényl) propionique. La déméthylation s'est opérée avec du tribromure de bore.
(Rf = 0,15 cyclohexane/acétate d'éthyle 7/3).

**PREPARATION 7 : 2,3,5,6-tétrahydro-8-hydroxy-benz[e]azulen-4(1H)-one**

On opère de manière équivalente à la préparation 2 stades B, C, D, E et F mais à partir de 10,0 g d'acide 3-(3-méthoxyphényl) propionique, et on obtient 2,9 g de produit attendu. La déméthylation s'est opérée avec du tribromure de bore.
(Rf = 0,15 dichlorométhane/acétate d'éthyle 95/5).

**PREPARATION 8 : Ester méthylique de l'acide (DL)-4-bromo 2 (phénylméthoxycarbonylamino) butanoïque.**

On agite 18 heures à 120°C dans une enceinte close 25 g de bromhydrate de 2-amino 4-butyrolactone dans 200 ml d'acide acétique à 24% d'acide bromhydrique gazeux. On refroidit à température ambiante, ramène à pression atmosphérique, concentre sous pression réduite, reprend le résidu dans 200 ml de méthanol puis fait barboter un courant d'acide chlorhydrique pendant 2 heures en maintenant la température inférieure à 35°C. On évapore le solvant sous pression réduite et obtient l'ester méthylique de l'acide 2-amino 4-bromo butanoïque que l'on reprend dans 250 ml d'acétone et 100 ml d'eau, neutralise à l'aide de soude 2N puis ajoute lentement 35 ml de chloroformiate de benzyle.
On agite pendant 48 heures, filtre, extrait à l'acétate d'éthyle, évapore le solvant, chromatographie le résidu sur silice (éluant : cyclohexane-acétate d'éthyle 7-3) et récupère 27,9 g de produit attendu. F = 90°C.

**PREPARATION 9 : Ester méthylique de l'acide 4-bromo 2-(terbutoxycarbonylamino) butanoïque.**

On agite pendant 48 heures à température ambiante 5,7 g de l'ester méthylique de l'acide 2-amino 4-bromo Butanoique préparé comme à la préparation 8 dans 120 ml de méthanol avec 24 ml de triéthylamine et 9 g de diterbutyl dicarbonate. On évapore les solvants, reprend le résidu à l'eau et au dichlorométhane, filtre, extrait au dichlorométhane, évapore les solvants, chromatographie le résidu sur silice (éluant : cyclohexane-ACOEt-TEA 7-3-0,5) et obtient 1,575 g de produit attendu de rf = 0,52.

**PREPARATION 10 : 4-(3-pyrimidinyl) 1H-imidazol 1-propanol.**

On mélange 505 mg d'éthylate de sodium dans 12,5 ml de diméthylformamide, ajoute 1 g de 3-(1H-imidazol-4-yl) pyridine puis 0,64 ml de chloropropanol et agite 16 heures à 55°C. On évapore le solvant sous pression réduite, chromatographie le résidu (éluant : $CH_2Cl_2$-MeOH 98-2) et récupère 1,015 g de produit attendu.

| Spectre IR ($CHCl_3$) | |
|---|---|
| OH | 3626 $cm^{-1}$ + associé |
| hétérocycle | 1601, 1578, 1551, 1499 $cm^{-1}$ |

**PREPARATION 11 : Hydrazone de hexahydro-2H-1,3-diazepin-2-one.**

On chauffe à 65°-70°C pendant 1 heure une suspension comprenant 3,3 g de nitroguanidine, 7 ml d'eau, 3,47 g de potasse et 5 g de dichlorhydrate de diamine; On ajoute 10,5 g de zinc, agite 30 minutes à température ambiante, puis ajoute 2 ml d'acide acétique, chauffe 1 heure à 40°C, filtre, ajoute 3 g de chlorure d'ammonium puis 4 g de bicarbonate de sodium. On extrait au dichlorométhane, évapore le solvant sous pression réduite, chromatographie le résidu sur silice (éluant : $CH_2Cl_2$-MeOH-$NH_4OH$ 8-4-2) et récupère 1,650 g de produit attendu.

**PREPARATION 12 : Monobromhydrate de 3a,4,5,6,7,7a-hexahydro 2-(propylthio) 1H-benzimidazole.**

On chauffe au reflux jusqu'à complète dissolution 1 g de octahydro 2H-benzimidazol-2-thione et 1,3 ml de bromopropane dans 20 ml d'éthanol. On évapore le solvant sous pression réduite, reprend le résidu dans un minimum de dichlorométhane, ajoute de l'éther isopropylique, évapore les solvants sous pression réduite, recristallise dans l'éther isopropylique, essore et sèche le produit attendu avec un rendement de 95%. F = 136°C.

**EXEMPLE 1 : Acide 7-((4-(((amino)iminométhyl)hydrazono)-9,10-diméthoxy-1,2,3,4,5,6-hexahydrobenz(e)azulen-8-yl)oxy)-heptanoique**

**Stade A :** Ester méthylique de l'acide 7-(4-oxo)-9,10-diméthoxy-1,2,3,4,5,6-hexahydrobenz(e)azulen-8-yl)oxy)-heptanoique

On agite à 40°C pendant 4 heures une suspension renfermant 0,684 g de 2,3,5,6-tétrahydro-8-hydroxy-9,10-diméthoxybenz[e]azulen-4(1H)-one (préparation 3), 12 ml de diméthylformamide (DMF), 12 ml de tétrahydrofuranne (THF), 0,7 g de carbonate de potassium et 0,835 g de 7-bromo oenanthate de méthyl. Après évaporation sous pression réduite le produit brut est chromatographié sur silice en éluant avec un mélange chlorure de méthylène ($CH_2Cl_2$)/acétone 95/5. On obtient ainsi 1,000 g de produit purifié sous la forme d'une huile jaune.

Rf $CH_2Cl_2$/acétone 95/5 : 0,5
IR ($CHCl_3$)
C=O 1732 $cm^{-1}$
OMe 1438 $cm^{-1}$
cétone conjuguée 1641 $cm^{-1}$
C=C 1592 $cm^{-1}$, 1557 $cm^{-1}$, 1492 $cm^{-1}$
+ aromatique

Stade B : Ester méthylique de l'acide 7-((4-(((amino)iminométhyl)hydrazono)-9,10-diméthoxy-1,2,3,4,5,6-hexahydrobenz(e)azulen-8-yl)oxy)-heptanoique

On agite 48 heures à température ambiante une suspension de 0,5 g du produit du stade A précédent, 5 ml

d'éthanol et 0,330 g de chlorhydrate d'amino guanidine, évapore le solvant sous pression réduite, et purifie le produit brut par chromatographie sur silice en éluant avec un mélange $CH_2Cl_2$/méthanol (MeOH)/ammoniaque 80/20/4. On obtient ainsi 0,466 g de produit purifié sous la forme d'une mousse blanche.

Rf $CH_2Cl_2$/méthanol (MeOH)/ammoniaque 80/20/4 : 0,8
IR (Nujol)
NH/$NH_2$ 3495 $cm^{-1}$, 3155 $cm^{-1}$ + associés
C=O 1731 $cm^{-1}$
C=N 1674 $cm^{-1}$
C=C 1625 $cm^{-1}$
aromatique 1595 $cm^{-1}$ (F)
NH/$NH_2$ 1534 $cm^{-1}$, 1491 $cm^{-1}$

Stade C : Acide 7-((4-(((amino)iminométhyl)hydrazono)-9,10-diméthoxy-1,2,3,4,5,6-hexahydrobenz(e)azulen-8-yl) oxy)-heptanoique

On agite pendant 3 heures à température ambiante la solution renfermant 0,44 g du produit obtenu au stade précédent, 5 ml d'éthanol et 2 ml de soude 1N, puis on neutralise avec 2 ml d'acide chlorhydrique 1N. Après évaporation sous pression réduite, on purifie le produit brut par chromatographie sur silice en éluant avec un mélange $CH_2Cl_2$/méthanol (MeOH)/ammoniaque 80/20/4. On obtient ainsi 0,192 g de produit purifié recristallisé dans le méthanol.

Rf $CH_2Cl_2$/méthanol (MeOH)/ammoniaque 80/20/4 : 0,17
RMN ($D_2O$ + 1 goutte de soude 1N)
3,92 tl 2H C<u>H</u>$_2$-O
2,17 t 2H C<u>H</u>$_2$-COOH
1,34 m 4H
1,55 m 2H  $CH_2$ centraux + $CH_2$-C=
1,70 m 4H
2,50 à 2,90 m 8H
6,62 s 1H $H_7$ aromatique
3,64 s 3H OC<u>H</u>$_3$
3,73 s 3H OC<u>H</u>$_3$

| Microanalyse | | | |
|---|---|---|---|
| % calculé<br>% trouvé | C 62,86<br>C 62,9 | H 7,47<br>H 7,5 | N 12,21<br>N 12,1 |

En opérant de manière équivalente à l'exemple 1 stades A, B et C, à partir de 2,3,5,6-tétrahydro-8-hydroxy-9,10-diméthoxy-benz[e]azulen-4(1H)-one (préparation 3), mais avec des groupements alkylants et des groupements G-$NH_2$ différents, on a préparé les produits de formule (I) suivants :

**EXEMPLE 2** : Acide 4-((4-(((amino)iminométhyl)hydrazono)-9,10-diméthoxy-1,2,3,4,5,6-hexahydrobenz(e)azulen-8-yl)oxy)-butanoique

**EXEMPLE 3** : Acide 4-((4-(((amino)iminométhyl)hydrazono)-9,10-diméthoxy-1,2,3,4,5,6-hexahydrobenz(e)azulen-8-yl)oxy)-pentanoique

**EXEMPLE 4** : Acide 5-((4-(((amino)carbonyl)hydrazono)-9,10-diméthoxy-1,2,3,4,5,6-hexahydro-8-benz(e)azulenyl)oxy)-pentanoique

**EXEMPLE 5** : Acide 6-((4-(((amino)iminométhyl)hydrazono)-9,10-diméthoxy-1,2,3,4,5,6-hexahydro-8-benz(e) azulenyl)oxy)-hexanoique

**EXEMPLE 6** : Acide 5-(9,10-diméthoxy-1,2,3,4,5,6-hexahydro-4-(4,5-dihydro-1H-imidazol-2-yl)hydrazono)-8-benz(e)azulenyl) oxy)-pentanoique

**EXEMPLE 7** : Acide 5-((4-(((amino)thiocarbonyl)hydrazono)9,10-diméthoxy-1,2,3,4,5,6-hexahydro-8-benz(e) azulenyl)oxy)pentanoique

**EXEMPLE 8** : Acide 6-(4((4,5-dihydro-1H-imidazol-2-yl) hydrazono)-9,10-diméthoxy-1,2,3,4,5,6-hexahydro-8-benz (e) azulenyl)oxy)-hexanoique

**EXEMPLE 9** : Acide 5-((4-(((amino)iminométhyl)hydrazono)-9,10-diméthoxy-1,2,3,4,5,6-hexahydro-8-benz(e) azulenyl)oxy)-3,3-diméthyl-4-oxo-pentanoique

**EXEMPLE 10** : Acide 5-(4((4,5-dihydro-1H-imidazol-2-yl) hydrazono)-9,10-diméthoxy-1,2,3,4,5,6-hexahydro-8-benz(e) azulenyl)oxy)-3,3-diméthyl-4-oxo-pentanoique

**EXEMPLE 11** : Acide 4-(4((4,5-dihydro-1H-imidazol-2-yl) hydrazono)-9,10-diméthoxy-1,2,3,4,5,6-hexahydro-8-benz(e) azulenyl)oxy)-butanoique

**EXEMPLE 12** : Acide 4-((9,10-diméthoxy-4-((1,4,5,6-tétrahydro-2-pyrimidinyl)hydrazono)-1,2,3,4,5,6-hexahydro-8-benz(e) azulenyl)oxy)-butanoique

**EXEMPLE 13** : Acide 2-(4((4,5-dihydro-1H-imidazol-2-yl) hydrazono)-9,10-diméthoxy-1,2,3,4,5,6-hexahydro-8-benz(e) azulenyl)oxy)-éthanoique

**EXEMPLE 14** : Acide 3-(4((4,5-dihydro-1H-imidazol-2-yl) hydrazono)-9,10-diméthoxy-1,2,3,4,5,6-hexahydro-8-benz(e) azulenyl)oxy)-propanoique

| Exemple | Pdt de départ | Produit alkylant | $G-NH_2$ | Rf. (1) |
|---|---|---|---|---|
| 2 | P3 | $Br-(CH_2)_3-CO_2Et$ | $H_2N-NH-C(=NH)-NH_2.HCl$ | 0,12 |
| 3 | P3 | $Br-(CH_2)_4-CO_2Et$ | $H_2N-NH-C(=NH)-NH_2.HCl$ | 0,12 |
| 4 | P3 | $Br-(CH_2)_3-CO_2Et$ | $H_2N-NH-C(=O)-NH_2.HCl$ | 0,18 |
| 5 | P3 | $Br-(CH_2)_5-CO_2Et$ | $H_2N-NH-C(=NH)-NH_2.HCl$ | 0,17 |
| 6 | P3 | $Br-(CH_2)_4-CO_2Et$ | $H_2N-NH-$ 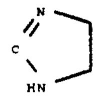 .HBr | 0,27 |
| 7 | P3 | $Br-(CH_2)_4-CO_2Et$ | $H_2N-NH-C(=S)-NH_2.HCl$ | 0,25 |
| 8 | P3 | $Br-(CH_2)_5-CO_2Et$ | $H_2N-NH-$ 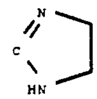 .HBr | 0,6 |
| 9 | P3 | $BrCH_2C(O)C(Me)_2-CH_2CO_2Et$ | $H_2N-NH-C(=NH)-NH_2.HCl$ | 0,3 |
| 10 | P3 | $BrCH_2C(O)C(Me)_2-CH_2CO_2Et$ | $H_2N-NH-$ 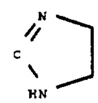 .HBr | 0,5 |
| 11 | P3 | $Br-(CH_2)_3-CO_2Et$ | $H_2N-NH-$ 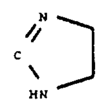 .HBr | 0,3 |
| 12 | P3 | $Br-(CH_2)_3-CO_2Et$ | $H_2N-NH-$ 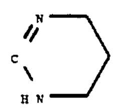 .HBr | 0,63 |
| 13 | P3 | $Br-(CH_2)-CO_2Et$ | $H_2N-NH-$ 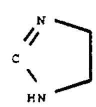 .HBr | 0,3 |
| 14 | P3 | $Br-(CH_2)_2-CO_2Et$ | $H_2N-NH-$ 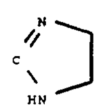 .HBr | 0,22 |

(1)dichlorométhane/méthanol/ammoniaque 80/20/4

**EXEMPLE 15 : Chlorhydrate de l'acide 5-((4-(((amino)iminométhyl)hydrazono)-9,10-diméthoxy-1,2,3,4,5,6-hexahydrobenz (e)azulen-8-yl)oxy)-pentanoique**

On mélange 86 mg du produit de l'exemple 3 avec 2 ml d'eau et 4 l d'acide chlorhydrique 0,1N puis après quelques minutes, lyophilise le milieu. On obtient 91 mg de sel attendu.

**EXEMPLE 16 : Acide 4-((4-(((amino)iminométhyl)hydrazono)-8,9-diméthoxy-1,2,3,4,5,6-hexahydro-10-benz(e) azulenyl)oxy)butanoique**

**EXEMPLE 17 : Acide 5-((4-(((amino)iminométhyl)hydrazono)-8,9-diméthoxy-1,2,3,4,5,6-hexahydro-10-benz(e) azulenyl)oxy)pentanoique**

On opère comme à l'exemple 1 stades A, B et C, mais à partir de 2,3,5,6-tétrahydro-10-hydroxy-8,9-diméthoxy-benz [e]azulen-4(1H)-one (préparation 2).

| Exemple | Pdt de départ | produit alkylant | G-$NH_2$ | Rf. |
|---|---|---|---|---|
| 16 | $(II)_A$ | Br-$(CH_2)_3$-$CO_2$Et | $H_2$N-NH-C(=NH)-$NH_2$.HCl | 0,07 |
| 17 | $(II)_A$ | Br-$(CH_2)_4$-$CO_2$Et | $H_2$N-NH-C(=NH)-$NH_2$.HCl | 0,07 |

**EXEMPLE 18 : Acide 4-((4-(((amino) iminométhyl) hydrazono)8,10-diméthoxy-1,2,3,4,5,6-hexahydro-9-benz(e) azulenyl)oxy)butanoique**

**EXEMPLE 19 : Acide 5-((4-(((amino)iminométhyl)hydrazono)**

**8,10-diméthoxy-1,2,3,4,5,6-hexahydro-9-benz(e)azulenyl)oxy)pentanoique**

On opère comme à l'exemple 1 stades A, B et C, mais à partir de 2,3,5,6-tétrahydro-9-hydroxy-8,10-diméthoxy-benz [e]azulen-4(1H)-one.

| Exemple | Pdt de départ | produit alkylant | G-$NH_2$ | Rf. |
|---|---|---|---|---|
| 18 | $(II)_{E1}$ | Br-$(CH_2)_3$-$CO_2$Et | $H_2$N-NH-C(=NH)-$NH_2$.HCl | 0,10 |
| 19 | $(II)_{E1}$ | Br-$(CH_2)_4$-$CO_2$Et | $H_2$N-NH-C(=NH)-$NH_2$.HCl | 0,07 |

**EXEMPLE 20 : Acide 4-((4((4,5-dihydro-1H-imidazol-2-yl)hydrazono)-1,2,3,4,5,6-hexahydro-9-benz(e)azulenyl) oxy)butanoique**

Stade A : Ester éthylique de l'acide 4-(4-oxo)-1,2,3,4,5,6-hexahydrobenz(e)azulen-9-yl)oxy)-butanoique

On agite à température ambiante pendant une nuit une suspension renfermant 0,6 g de 2,3,5,6-tétrahydro-9-hydroxybenz[e]azulen-4(1H)-one (préparation 6), 12 ml de diméthylformamide (DMF), 12 ml de tétrahydrofuranne (THF), 0,7 g de carbonate de potassium et 0,7 ml de bromobutyrate d'éthyle. Après évaporation sous pression réduite le produit brut est chromatographié sur silice en éluant avec un mélange chlorure de méthylène (dichlorométhane/ acétone 95/5). On obtient ainsi 0,608 mg de produit purifié sous la forme d'une huile jaune.

IR ($CHCl_3$)
C=O 1728$cm^{-1}$
cétone conj. 1641 $cm^{-1}$
C=C aromatiques 1610 $cm^{-1}$, 1590 $cm^{-1}$, 1569 $cm^{-1}$, 1499 $cm^{-1}$

Stade B : Ester éthylique de l'acide 4-((4-((4,5-dihydro-1H-imidazolin-2-yl)hydrazono)-1,2,3,4,5,6-hexahydrobenz(e) azulen-9-yl)oxy)-butanoique

On agite 24 heures au reflux 608 mg du produit du stade A précédent, 10 ml de butanol et 600 mg de bromhydrate d'amino guanidine cyclique suivant : le (4,5-dihydro-1H-imidazolin-2-yl)-hydrazine, évapore le solvant sous pression réduite, et purifie le produit brut par chromatographie sur silice en éluant avec un mélange $CH_2Cl_2$/méthanol

(MeOH)/ammoniaque 80/20/4. On obtient ainsi 0,604 g de produit attendu.

IR ($CHCl_3$)
=C-NH- 3451 $cm^{-1}$
C=O 1728 $cm^{-1}$ (ester)
C=N + C=C + aromatiques : 1627 $cm^{-1}$ (F), 1568 $cm^{-1}$, 1548 $cm^{-1}$, 1497 $cm^{-1}$, 1488 $cm^{-1}$

Stade C : Acide 7-((4,5-dihydro-1H-imidazol-2-yl)hydrazono)1,2,3,4,5,6-hexahydrobenz(e)azulen-9-yl)oxy)-butanoique.

On agite pendant 4 heures à température ambiante la solution renfermant 0,604 g du produit obtenu au stade précédent, 8 ml d'éthanol, 5 ml de tétrahydrofuranne et 2 ml de soude 2N, puis on neutralise avec 2 ml d'acide chlorhydrique. Après évaporation sous pression réduite, on purifie le produit brut par chromatographie sur silice en éluant avec un mélange dichlorométhane ($CH_2Cl_2$)/méthanol (MeOH)/ammoniaque 80/20/4. On obtient ainsi 0,298 g de produit purifié recristallisé dans le méthanol.
Rf (dichlorométhane/méthanol/ammoniaque 80/20/4) : 0,2

| RMN ($D_2O$ + 1 goutte de soude 1N) | |
|---|---|
| 1,71 (l) 2H | O-$CH_2$-C$\underline{H}_2$-$CH_2$-CO |
| 1,96 (m) 2H | $CH_2$ en 2 (cyclopentène) |
| 2,30 (t) 2H | C$\underline{H}_2$-CO |
| 2,50 à 2,75 8H | C$\underline{H}_2$-C= |
| 3,45 (sl) 4H | C$\underline{H}_2$-N= |
| 3,89 (tl) 2H | Ph-O-C$\underline{H}_2$-C |
| 6,70 (m) 2H | $H_{10}$ et $H_8$ |
| 7,00 (d, J=8) | $H_7$ |

**EXEMPLE 21 : Acide 4-(4((4,5-dihydro-1H-imidazol-2-yl) hydrazono)-1,2,3,4,5,6-hexahydro-8-benz(e)azulenyl) oxy)-butanoique**

On opère comme à l'exemple 20 mais à partir de 0,856 g de 2,3,5,6-tétrahydro-8-hydroxy-benz[e]azulen-4 (1H)-one (préparation 7) et on obtient 0,299 g de produit attendu. Rf (dichlorométhane/méthanol/ammoniaque 80/20/4) : 0,27

**EXEMPLE 22 : Acide 5-((8-(((amino)iminométhyl)hydrazono)6,7,8,9,10,11-hexahydro-azuleno(5,6-d)-1,3-benzodioxol-4-yl)oxy)-pentanoique**

Stade A : Ester éthylique de l'acide 5-(((4-oxo)-9,10-dihydroxy-1,2,3,4,5,6-hexahydrobenz(e)azulen-9-yl)oxy)-pentanoique

1) protection

A une solution, sous atmosphère inerte, de 10 g de 2,3,5,6-tétrahydro-8,9,10-trihydroxy-benz[e]azulen-4(1H)-one (préparation 1) dans 100 ml de tétrahydrofuranne, on ajoute 4,42 ml de triméthoxyborate et 20,4 ml de triéthylamine en maintenant la température entre 37 et 39°C, puis agite à température ambiante pendant 3 heures.

2) alkylation et déprotection

On ajoute ensuite 9,7 ml de bromo-5valérate d'éthyle, 100 ml de diméthylformamide et 8,4 g de carbonate de potassium et agite 2 jours à 60°C. Le mélange réactionnel est ensuite traité avec 120 ml d'eau et 50 ml d'acide chlorhydrique concentré 36N, on agite pendant 1 heure rajoute de l'acétate d'éthyle, sépare les phases organiques et aqueuses. La phase organique est ensuite lavée, séchée et évaporée sous pression réduite. On obtient un produit brut que l'on purifie par chromatographie sur silice en éluant avec le mélange cyclohexane/acétate d'éthyle 70/30. On obtient 5,2 g de produit pur attendu.

Rf (dichlorométhane/méthanol 95/5) = 0,82

Rf (cyclohexane/acétate d'éthyle 70/30) = 0,23

Stade B : Ester éthylique de l'acide 5-(((8-oxo)6,7,8,9,10,11-hexahydro-azuleno(5,6-d)-1,3-benzodioxol-4-yl)oxy))-pentanoique

On mélange, sous atmosphère inerte, à 60°C pendant 1 heure, 2,5 g du produit obtenu au stade précédent, 17 ml de diméthylformamide, 3,6 g de CsF et 1,4 ml de dibromométhane. Après filtration et rinçage au méthanol, on évapore sous pression réduite et purifie le produit brut par chromatographie sur silice en éluant avec le mélange cyclohexane/acétate d'éthyle 85/15. On obtient 1,73 g de produit pur attendu.

(F= 118°C)
Rf (cyclohexane/acétate d'éthyle 80/20) = 0,25

Stade C : Ester éthylique de l'acide 5-((8-(((amino)iminométhyl)hydrazono)-6,7,8,9,10,11-hexahydro-azuleno(5,6-d)-1,3-benzodioxol-4-yl)oxy)-pentanoique

On mélange 1 nuit à 120°C 551 mg du produit obtenu au stade précédent et 467 mg de chlorhydrate d'aminoguanidine, puis on purifie par chromatographie en éluant avec un mélange dichlorométhane/méthanol/ammoniaque 80/20/4. On obtient 174 mg du produit attendu.
Rf (dichlorométhane/méthanol/ammoniaque 80/20/4) 0,98

Stade D : Acide 5-((8-(((amino)iminométhyl)hydrazono)6,7,8,9,10,11-hexahydro-azuleno(5,6-d)-1,3-benzodioxol-4-yl)oxy)-pentanoique

On mélange à température ambiante pendant 1 heure 30, 274 mg du produit obtenu au stade précédent et 1,86 ml de soude 1N, neutralise ensuite par une solution d'acide chlorhydrique 1N, et évapore sous pression réduite. Le produit brut est purifié par chromatographie en éluant avec le mélange dichlorométhane/méthanol/ammoniaque 80/20/4. On obtient 141 mg du produit attendu.
Rf (dichlorométhane/méthanol/ammoniaque 80/20/4) 0,23

| RMN (DMSO) | |
|---|---|
| 1,55 à 1,9 (m) 4H | $O-CH_2-C\underline{H}_2-C\underline{H}_2-CH_2-CO$ |
| 1,55 à 1,9 (m) 2H | $CH_2$ en 2 (cyclopentène) |
| 2,26 (t) 2H | $C\underline{H}_2-CO$ |
| 2,65 à 3,00 (m) 8H | $C\underline{H}_2-C=$ |
| 4,07 (t) 2H | $O-C\underline{H}_2-CH_2-$ |
| 5,95 (s) 2H | $-O-C\underline{H}_2-O$ |
| 6,61 (m) 1H | $H_8$ |
| H mobiles (m, large) | $NH-C(=NH)-NH_2$ |

**EXEMPLE 23 : Acide 5-((8-(((amino)iminométhyl)hydrazono)-2,2-diphényl-6,7,8,9,10,11-hexahydro-azuleno (4,5-e)-1,3-benzodioxol-4-yl)oxy)-pentanoique**

On opère comme à l'exemple précédent, à partir de 374 mg du produit obtenu au stade A de l'exemple précédent et 0,19 ml de diphényldichlorométhane. On obtient 198 mg de produit attendu.
Rf (dichlorométhane/méthanol/ammoniaque 80/20/4)= 0,17

**EXEMPLE 24 : O-[4-[(4,5-dihydro-1H-imidazol-2-yl)hydrazono)9,10-diméthoxy-1,2,3,4,5,6-hexahydro-8-benz(e) azulenyl]-N-[(phénylméthoxy)carbonyl]-DL-homoserine.**

Stade A : Ester méthylique de la O-[(4-oxo)-9,10-diméthoxy-1,2,3,4,5,6-hexahydro-8-benz(e)azulenyl]-N-[(phénylméthoxy)carbonyl]-DL-homoserine.

On agite 1 nuit à température ambiante 0,6 g de 2,3,5,6-tétrahydro-8-hydroxy-9,10-diméthoxy-benz[e]azulen-4(1H)-one (préparation 3), 10 ml de diméthylformamide, 10 ml de tétrahydrofuranne, 1 g de carbonate de potassium et 0,867 g d'ester méthylique de l'acide (DL)-4-bromo-2-(phénylméthoxycarbonylamino) butanoïque préparé comme

à la préparation 8. Après évaporation sous pression réduite le produit brut est chromatographié sur silice en éluant avec un mélange chlorure de méthylène ($CH_2Cl_2$)/acétone 95/5. On obtient ainsi 1,166 g de produit purifié sous la forme d'une huile jaune.

IR ($CHCl_3$)
C=O 1740 $cm^{-1}$ (ép.), 1721 $cm^{-1}$
cétone conj. 1642 $cm^{-1}$
C=C aromatiques 1593 $cm^{-1}$, 1559 $cm^{-1}$, 1508 $cm^{-1}$, 1493 $cm^{-1}$

Stade B : Ester méthylique de la O-[4-[(4,5-dihydro-1H-imidazol-2-yl)hydrazono)-9,10-diméthoxy-1,2,3,4,5,6-hexahydro-8-benz(e)azulenyl]-N-[(phénylméthoxy)carbonyl]-DL-homoserine.

On agite 24 heures à 120°C, 539 mg du produit du stade A précédent, 15 ml de butanol et 600 mg de bromhydrate d'amino guanidine cyclique suivant : le (4,5-dihydro-1H-imidazol-2-yl)-hydrazine, évapore le solvant sous pression réduite, et purifie le produit brut par chromatographie sur silice en éluant avec un mélange $CH_2Cl_2$/méthanol (MeOH)/ammoniaque 80/20/4. On obtient ainsi 0,641 g de produit attendu.

IR ($CHCl_3$)
=C-NH- 3451 $cm^{-1}$ + associés
C=O 1740 $cm^{-1}$ (ép.), 1720 $cm^{-1}$ (max)
C=N + C=C + aromatiques + amide II :1667 $cm^{-1}$ (F), 1606 $cm^{-1}$, 1508 $cm^{-1}$, 1490 $cm^{-1}$.

Stade C : O-[4-[(4,5-dihydro-1H-imidazol-2-yl)hydrazono)9,10-diméthoxy-1,2,3,4,5,6-hexahydro-8-benz(e)azulenyl]-N-[(phénylméthoxy)carbonyl]-DL-homoserine.

On agite pendant 2 heures à température ambiante la solution renfermant 0,6 g du produit obtenu au stade précédent, 10 ml d'éthanol et 2 ml de soude 2N, puis on neutralise avec 2 ml d'acide chlorhydrique. Après évaporation sous pression réduite, on purifie le produit brut par chromatographie sur silice en éluant avec un mélange $CH_2Cl_2$/méthanol (MeOH)/ammoniaque 80/20/4. On obtient ainsi 0,349 g de produit purifié recristallisé dans le méthanol.
Rf $CH_2Cl_2$/méthanol (MeOH)/ammoniaque 80/20/4 : 0,37

**EXEMPLE 25 : O-[4-[(4,5-dihydro-1H-imidazol-2-yl)hydrazono)1,2,3,4,5,6-hexahydro-8-benz(e)azulenyl]-N-[(phénylméthoxy)carbonyl]-DL-homoserine.**

On opère de manière équivalente à l'exemple 24 mais à partir de 0,428 g de 2,3,5,6-tétrahydro-9-hydroxy-benz[e]azulen-4(1H)-one (Préparation 7). On obtient 245 mg de produit attendu.
Rf $CH_2Cl_2$/méthanol (MeOH)/ammoniaque 80/20/4 : 0,5

**EXEMPLE 26 : O-[4-[(1,2,3,4-tétrahydro 6-pyrimidinyl) hydrazono] 9,10-diméthoxy 1,2,3,4,5,6-hexahydro 8-benz [e]azulenyl] N-[(phénylméthoxy) carbonyl] DL-homoserine.**

Stade A : monobromhydrate de l'ester méthylique de O-[9,10-diméthoxy 1,2,3,4,5,6-hexahydro 4-[(1,4,5,6-tétrahydro 2-pyrimidinyl) hydrazono] 8-benz(e)azulényl] N-[(phénylméthoxy) carbonyl] DL-homoserine.

On opère comme à l'exemple 24 stade B en utilisant au départ 200 mg du produit obtenu comme à l'exemple 24 stade A dans 2 ml de butanol et 74,5 mg du monobromhydrate de l'hydrazone de tétrahydro-2(1H)-pyrimidinone et chauffe au reflux pendant 16 heures. On laisse revenir à température ambiante, extrait au dichlorométhane, sèche, évapore le solvant sous pression réduite et obtient 152 mg de produit atendu.

Stade B : O-[4-[(1,2,3,4-tétrahydro 6-pyrimidinyl) hydrazono] 9,10-diméthoxy 1,2,3,4,5,6-hexahydro 8-bent[e]azulenyl] N-[(phénylméthoxy) carbonyl] DL-homoserine.

On opère comme à l'exemple 24 stade C en utilisant 131 mg de produit obtenu au stade A ci-dessus en solution dans 1,3 ml d'éthanol et 0,43 ml de soude N. On neutralise par addition d'acide chlorhydrique N, évapore le solvant, filtre, sèche 78 mg de produit attendu. F = 172°C.

| Spectre RMN ($CDCl_3$) | |
|---|---|
| 1,90 (m) (2H) | $CH_2$ en 9 |
| 2,03 (m) | $CH_2$ central |
| 2,36 (m) (2H) | |
| 2,60 à 3,00 (8H) | les =C-$CH_2$ |
| 3,48 (ml) (4H) | les =N-$CH_2$ |
| 3,77 (s) 3,78 (s) (9H) | les =C-OMe |
| 4,01 (m) (1H) 4,17 | Φ-O-$CH_2$ |
| 4,67 (p) | =C-CH-N-C= |
| 5,14 (sl) | COO-$CH_2$-Φ |
| 6,13 (d) | =C-NH-CH |
| 6,49 (sl) | $H_4$ |
| ≈7,36 (m) (5H) | Φ-C |

**EXEMPLE 27 : Ester (2,3-dihydroxypropylique) de 0-(9,10-diméthoxy 1,2,3,4,5,6,-hexahydro 4-[(1,4,5,6-tétrahydro 2-pyrimidinyl) hydrazono]-8-benz(e)azulényl] N-[(phénylméthoxy) carbonyl] DL-homoserine.**

Stade A :Ester [(2,2-diméthyl 1,3-dioxolan-4-yl) méthylique] de 0-[9,10-diméthoxy 1,2,3,4,5,6-hexahydro 4-[(1,4,5,6-tetrahydro-2-pyrimidinyl) hydrazono] 8-benz(e)azulényl] N-[(phénylméthoxy) carbonyl] DL-homoserine.

On refroidit à 0°C 0,3 g de produit préparé comme à l'exemple 26 dans 1 ml de diméthylformamide et 1 ml de dichlorométhane, 96 mg de 1-(3-diméthylaminopropyl) 3-éthylcarbodiimide chlorhydrate et 68 mg de 1-hydroxy benzotriazole hydrate. On agite 30 minutes à température ambiante, introduit 0,06 ml de solketal et poursuit l'agitation pendant 3 heures et demie. On dilue le milieu réactionnel par de l'eau, extrait au dichlorométhane et récupère 0,6 g de produit brut que l'on purifie par chromatographie sur silice (éluant : $CHCl_2$-MeOH 90-10). On obtient 0,352 g de produit attendu.

| Spectre IR ($CHCl_3$) | |
|---|---|
| NH | 3400 $cm^{-1}$ |
| C=O | 1745 (ep), 1719 $cm^{-1}$ |
| C=N, C=C } aromatique, amide II } | 1672 (F), 1645,1597, 1565 (f), 1507, 1492 $cm^{-1}$ |

Stade B : Ester (2,3-dihydroxypropylique) de 0-(9,10-diméthoxy 1,2,3,4,5,6,-hexahydro 4-[(1,4,5,6-tétrahydro 2-pyrimidinyl) hydrazono]-8-benz(e)azulényl] N-[(phénylméthoxy) carbonyl] DL-homoserine.

On agite 6 heures à température ambiante 0,320 g de produit obtenu au stade A dans 3 ml d'éthanol et 1 ml d'acide chlorhydrique 2N. Après évaporation des solvants et chromatographie sur silice (éluant : $CH_2Cl_2$-MeOH-$NH_4OH$ 80-20-4), on obtient 0,112 g de produit attendu.

Spectre RMN ($CDCl_3$)

| | | |
|---|---|---|
| 1,88 (m) (2H) | } | |
| 1,98 | } | $CH_2$ centraux et $CH_2$ en 9 |
| 2,36 | } | |
| 2,60 à 3,10 (8H) | | $=C-CH_2$ |
| 3,43 (m) (4H) | | $=N-N-CH_2$ |
| 3,79 (s) | } | |
| 3,81 | } | Φ-OMe |
| 3,60 à 3,90 | | $O-CH_2-CH-O$ |
| ≈4,00 à 4,30 | } | $COO-CH_2-CH$ |
| | } | $Φ-O-CH_2-CH_2$ |
| 4,64 (m) (1H) | | =C-CH-N-C= |
| 5,13 (s) | | $COO-CH_2-Φ$ |
| 6,53 (s) | | $H_4$ |
| 7,20 à 7,38 | | les Φ-C |

**EXEMPLE 28 : O-[4-[(4,5-dihydro 1H-imidazol-2-yl) hydrazono] 9,10-diméthoxy 1,2,3,4,5,6-hexahydro 8-benz(e) azulenyl] N-[(8-quinoléinyl) sulfonyl] DL-homoserine.**

Stade A : Ester méthylique de O-(9,10-diméthoxy 1,2,3,4,5,6-hexahydro 4-oxo 8-benz(e)azulényl) N-[(1,1-diméthyléthoxy) carbonyl] DL-homoserine.

On agite à température ambiante pendant 65 heures, 4,1 g de produit préparé comme à la préparation 3 et 5 g de l'ester préparé comme à la préparation 9 dans 50 ml de diméthylformamide et 50 ml de tétrahydrofuranne en présence de 5 g de carbonate de potasium et de diméthylaminopyridine. On évapore le solvant sous pression réduite, purifie le résidu par chromatographie sur silice (éluant : $CH_2Cl_2$-acétone 95-5) et récupère 7,3 g de produit attendu.

| Spectre IR ($CHCl_3$) | |
|---|---|
| =C-NH | 3430 $cm^{-1}$ |
| C=O | 1744 $cm^{-1}$ (ester méthylique) |
| | 1710 $cm_{-1}$ (NH-BOC) |
| | 1648 $cm^{-1}$ (cétone conjuguée) |
| aromatique + amide II 1593, 1559, 1493 $cm^{-1}$ | |

Stade B : Monochlorhydrate de l'ester méthylique de O-(9,10-diméthoxy 1,2,3,4,5,6-hexahydro 4-oxo 8-benz(e)azulényl) DL-homoserine.

On ajoute à 3 reprises 10 ml d'acide chlorhydrique dans l'acétate d'éthyle à 6 g de produit préparé au stade A dans 10 ml d'acétate d'éthyle puis agite 16 heures à température ambiante. On évapore le solvant sous pression réduite et obtient 0,656 g de produit attendu que l'on utilise tel quel au stade suivant.

Stade C : O-(9,10-diméthoxy 1,2,3,4,5,6-hexahydro 4-oxo 8-benz(e)azulényl) N-[(8-quinoléinyl) sulfonyl] DL-homoserine.

On reprend 0,656 g du produit obtenu ci-dessus dans 5 ml de dichlorométhane, ajoute 1 ml de triéthylamine et 0,638 g de 8-chlorosulfonyl quinoléine et agite 2 heures à température ambiante. Après évaporation des solvants sous pression réduite et chromatographie sur silice (éluant : $CHCl_2$-MeOH 95-5), on récupère 0,956 g de produit attendu.

Stade D : ester méthylique de O-[4-[(4,5-dihydro 1H-imidazol-2-yl) hydrazono] 9,10-diméthoxy 1,2,3,4,5,6-hexahydro 8-benz(e)azulényl] N-[(8-quinoléinyl) sulfonyl] DL-homoserine.

On agite 16 heures à 120°C, 0,9 g du produit du stade A précédent, 5 ml de butanol et 0,6 g de bromhydrate d'aminoguanidine cyclique suivant : le (4,5-dihydro 1H-imidazol-2-yl) hydrazine, évapore le solvant sous pression réduite et obtient 0,786 g de produit attendu utilisé tel quel pour le stade suivant.

Stade E : O-[4-[(4,5-dihydro 1H-imidazol-2-yl) hydrazono] 9,10-diméthoxy 1,2,3,4,5,6-hexahydro 8-benz(e)azulenyl] N-[(8-quinoléinyl) sulfonyl] DL-homoserine.

On agite pendant 2 heures à température ambiante la solution renfermant 0,786 g du produit obtenu au stade précédent, 5 ml de méthanol et 2 ml de soude 2N, puis on neutralise avec 2 ml d'acide chlorhydrique 2N et agite pendant 10 minutes. Après évaporation sous pression réduite, on purifie le produit brut par chromatographie sur silice en éluant avec un mélange $CH_2Cl_2$-méthanol-ammoniaque 80-20-4. On obtient 0,438 g de produit attendu après recristallisation dans le méthanol.
Rf = 0,40 ($CHCl_2$-MeOH-$NH_4OH$ 80-20-4).

Spectre RMN (DMSO)

| | | |
|---|---|---|
| 1,81 (sl) | | $CH_2$ en 9 |
| 2,40 à 3,20 | | les =C-$CH_2$ |
| 3,35 (l) | | les =N-$CH_2$ |
| 3,55 (sl) | | les =C-OMe |
| 6,63 (sl) | | $H_4$ |
| 7,58 (dd) | | $H'_3$ |
| 7,67 (t) | | $H'_6$ |
| 8,19 (d), 8,29 (d) | | $H'_5$ et $H'_7$ |
| 8,45 (d) | | $H_4$ |
| 8,90 (d) | | $H_2$ |
| 7,31 (sl) | } | |
| 7,97 | } | H mobiles |
| 10,40 (f) | } | |
| 12,62 | } | |

**EXEMPLE 29 : Monochlorhydrate de O-[4-[(4,5-dihydro 1H-imidazol-2-yl) hydrazono] 9,10-diméthoxy 1,2,3,4,5,6-hexahydro 8-benz(e)azulényl] N-[[3-[4-(3-pyridinyl) 1H-imidazol-1-yl] propoxy] carbonyl] DL-homoserine.**

Stade A : 4-[[9,10-diméthoxy 1,2,3,4,5,6-hexahydro 4-oxo 8-benz(e)azylényl] oxy] 2-isocyanato butanoate de méthyle.

On agite 10 minutes à 0°C 450 mg de l'amine obtenue au stade B de l'exemple 28 dans 10,2 ml d'une solution aqueuse saturée en bicarbonate de sodium et 10,2 ml de dichlorométhane. On ajoute dans la phase organique du milieu réactionnel 204 mg de triphosgène en solution dans 2 ml de dichlorométhane, agite 10 minutes, extrait au dichlorométhane, sèche, évapore le solvant sous pression réduite et obtient 430 mg de produit attendu que l'on utilise tel quel au stade suivant.

Stade B : O-[9,10-diméthoxy 1,2,3,4,5,6-hexahydro 4-oxo 8-benz(e)azulényl] N-[[3-[4-(3-pyridinyl) 1H-imidazol-1-yl] propoxy] carbonyl] DL-homoserine.

On refroidit à 0°C 430 mg du produit obtenu au stade A dans 20 ml de dichlorométhane et ajoute 414 mg de

l'alcool préparé comme à la préparation 10 dans 10 ml de dichlorométhane. On laisse revenir à température ambiante, maintient sous agitation pendant 48 heures, évapore le solvant sous pression réduite, chromatographie le résidu sur alumine (éluant : $CH_2Cl_2$-MeOH) et récupère 298 mg de produit attendu. Stade C : monobromhydrate de O-[4[(4,5-dihydro 1H-imidazol-2-yl) hydrazono] 9,10-diméthoxy 1,2,3,4,5,6-hexahydro 8-benz(e)azulényl] N-[[3-[4-(3-pyridinyl) 1H-imidazol-1-yl] propoxy] carbonyl] DL-homoserine.

On opère comme à l'exemple 24 stade B en utilisant au départ 277 mg du produit obtenu au stade B ci-dessus et 164 mg du bromhydrate d'aminoguanidine cyclique dans 13 ml de butanol. Après chromatographie sur alumine (éluant : $CH_2Cl_2$-MeOH 95-5), on obtient 289 mg de produit attendu.

Spectre IR ($CHCl_3$)

| | |
|---|---|
| C=O | 1746 (ep) 1723 (max) $cm^{-1}$ |
| syst. conjugué + aromatique + amide II | 1668,1625(F),1599(ep),1551,1509,1489 $cm^{-1}$ |
| OH | 3618 $cm^{-1}$ |

Stade D : Monochlorhydrate de O-[4-[(4,5-dihydro 1H-imidazol-2-yl) hydrazono] 9,10-diméthoxy 1,2,3,4,5,6-hexahydro 8-benz(e)azulényl] N-[[3-[4-(3-pyridinyl) 1H-imidazol-1-yl] propoxy] carbonyl] DL-homoserine.

On ajoute 0,3 ml de soude N à 277 mg de produit obtenu au stade C ci-dessus dans 10 ml d'éthanol, agite 30 minutes, ajoute 10 ml d'eau, acidifie le milieu réactionnel jusqu'à pH = 2,5 à l'aide d'acide chlorhydrique N, évapore les solvants sous pression réduite, chromatographie le résidu sur silice (éluant : $CH_2Cl_2$-MeOH-$NH_4OH$ 40-10-2), évapore le filtrat sous pression réduite, reprend le résidu dans l'éther isopropylique, filtre le précipité, le sèche et recueille 126 mg de produit attendu.

Spectre RMN (DMSO)

| | |
|---|---|
| 1,78 (m) (2H) } 1,90 à 2,30 (m) (5H) } | les 3-$CH_2$ |
| 1,60 à 2,90 (m) (8H) | les $CH_2$-C= |
| 3,53 (sl) (≈4H) | N-$CH_2$-$CH_2$-N |
| 3,69 (s), 3,71 (s) | $CH_3$O-C= |
| 3,90 à 4,20 (m) (7 à 8H) | les $CH_2$ et CH |
| 6,73 (s) | $H_4$ |
| 7,20 (d, large) | CO-NH-CH- |
| 7,35 (dd) | $H'_5$ |
| 8,04 (d) | $H'_4$ |
| 8,37 (dl) | $H'_6$ |
| 8,94 (sl) | $H'_2$ |
| 7,72 (s), 7,80 (s) | CH=imidazole. |

**EXEMPLE 30 : Acide 5-[[4-[(4,5-dihydro 4-oxo 1H-imidazol-2-yl) hydrazono] 9,10-diméthoxy 1,2,3,4,5,6-hexahydro 8-benz(e)azulényl] oxy] pentanoïque.**

On mélange à température ambiante 300 mg du produit préparé au stade A de l'exemple 3 dans 6 ml d'éthanol avec 61 mg de bicarbonate de soude et 0,7 ml de bromoacétate d'éthyle. On évapore les solvants, chromatographie le résidu sur silice (éluant : $CH_2Cl_2$-MeOH 95-5) et obtient 139 mg de l'ester éthylique intermédiaire. On mélange pendant 2 heures à température ambiante 110 mg de cet ester dans 1 ml d'éthanol en présence de 0,5 ml de soude 2N. Après neutralisation du milieu réactionnel à l'aide d'acide chlorhydrique 2N, on filtre le précipité formé, le sèche et récupère 44 mg de produit attendu.

Spectre RMN (DMSO)

| | |
|---|---|
| ≈ 1,73 (m) (6H) | $CH_2$ centraux et $CH_2$ en 9 |
| 2,30 (t) (2H) | $=C-CH_2$ (chaîne) |
| 2,30 à 3,20 | $=C-CH_2$ |
| 3,73 (s), 3,75 (s) | Φ-OMe |
| 3,83 (sl) | $=C-N-CH_2-C=$ |
| 4,02 (t) | $Φ-O-CH_2$ |
| 6,76 (s) | $H_4$ |
| 7,20 (sl) (1H) } | |
| 8,28 (sl) (1H) } | H mobiles |
| 12,04 (1H) } | |

**EXEMPLE 31 : O-[9,10-diméthoxy 1,2,3,4,5,6-hexahydro 4-[(4,5,6,7-tétrahydro 1H-1,3-diazépin-2-yl) hydrazono] 8-benz(e)-azulényl] N-[(phénylméthoxy) carbonyl] DL-homoserine.**

On mélange pendant 16 heures à 130°C 1 g du composé préparé à l'exemple 24 stade A dans 5 ml de butanol et 0,9 g d'aminoguanidine cyclique préparé comme à la préparation 11. On évapore le solvant sous pression réduite, chromatographie le résidu sur silice (éluant : $CH_2Cl_2$-MeOH 90-10) et obtient 0,8 g d'ester intermédiaire que l'on agite à température ambiante pendant 1 heure et demie dans 3 ml de méthanol avec 2 ml de soude 2N. Après neutralisation du milieu réactionnel à l'aide d'acide chlorhydrique 2N et évaporation des solvants sous pression réduite, on chromatographie le résidu sur silice (éluant : $CH_2Cl_2$-MeOH-$NH_4OH$ 90-15-2) et récupère 0,22 g de produit attendu.

**EXEMPLE 32 : O-[9,10-diméthoxy 1,2,3,4,5,6-hexahydro 4-[(3a,4,5,6,7,7a-hexahydro 1H-benzimidazol-2-yl) hydrazono] 8-benz(e)azulényl] N-[(phénylméthoxy) carbonyl] DL-homoserine.**

On opère comme aux stades B et C de l'exemple 24 en utilisant au départ 200 mg du composé préparé à l'exemple 24 stade A et 176 mg d'amino guanidine cyclique préparé comme à la préparation 12. On récupère 102 mg de l'ester intermédiaire dont on utilise 100 mg pour la réaction de saponification. On obtient 59 mg de produit attendu. Rf = 0,24 ($CH_2Cl_2$-MeOH-$NH_4OH$ 85-15-3).

Compositions pharmaceutiques

On a préparé des comprimés répondant à la formule suivante :

- produit de l'exemple 1 50 mg
- Excipient (talc, amidon, stéarate de magnésium) QS pour un comprimé terminé à 120 mg

## Etude pharmacologique des produits de l'invention

**1 - Etude par les produits de l'invention du déplacement de la liaison : Vitronectine/récepteur Vitronectine ($\alpha_v\beta_3$)** Protocole :

Des plaques 96 puits MaxiSorp sont coatées une nuit à 4°C, avec 100 µl de Vitronectine humaine (cf Yatohgo et al. Cell., Structure and fraction 13 : 281-292 (1988)) à 2 µg/ml, (Dilution en tampon de coating).

Le lendemain, les puits sont vidés et les ligands (Vitronectine) sont ensuite fixés (voir tampon de fixation) pendant 1H à température ambiante sous agitation douce.

Les puits sont lavés six fois (voir tampon de lavage), puis on ajoute par puits et dans cet ordre :

- 40 µl de tampon d'incubation,
- 10 µl de la dilution du produit à tester,

(les produits sont dilués dans un mélange 50/50 de DMSO-$H_2O$) - 50 µl de récepteur $\alpha_v\beta_3$ humain (cf Pytela et al. Methods Enzymol (1987) 144:475) (dilution en tampon d'incubation, à adapter suivant le lot de récepteur et selon le ligand).

Le ligand, le récepteur $\alpha_v\beta_3$ humain et les produits à étudier sont incubés pendant 3 heures à température ambiante sous agitation douce.

Les puits sont à nouveau lavés six fois, puis incubés pendant 2 heures à température ambiante sous agitation douce, en présence de 100 µl d'anticorps 4B12-HRP, anti-récepteur couplé à une peroxydase (l'anticorps 4B12-HRP est dilué en tampon d'incubation. La dilution est à adapter suivant le lot de récepteur).

Les puits sont ensuite lavés six fois avant la mesure de liaison ligand-récepteur faite par l'intermédiaire d'un kit révélateur de peroxydase (TMB Microwell Peroxidase Substrate System Kirkegaard : Réf. cat. 50-76-00).

Ce kit contient un flacon A de substrat (3,3',5,5'-tétraméthylbenzidine à 0,4 g/l) et un flacon B ($H_2O_2$ à 0,02 % en tampon Citrate/Acide citrique). Extemporanément, un volume de A est mélangé à un volume de B, puis le mélange réactionnel est distribué à raison de 100 µl/puits. La réaction enzymatique se développe en 12' pour Vitronectine/$\alpha_v\beta_3$, puis son évolution est stoppée par l'addition de 100 µl d'acide phosphorique **1M**.
La densité optique est mesurée à 450 nm.

Tampons :

- tampon de coating : Carbonate 0,05 M, NaOH pH 9,6
- tampon de fixation : PBS contenant 0,5 % de BSA (pH 7,4)
- tampon de lavage : PBS contenant 0,05 % de Tween 20 (pH 7,4)
- tampon d'incubation :
  - . 50 mM TRIS pH 7,4
  - . 0,5 % BSA
  - . 0,05 % Tween 20
  - . 1 mM $MnCl_2$
  - . 50 µ M $CaCl_2$
  - . 50 µM $MgCl_2$
  - . 100 mM NaCl.

Expression des résultats :

On trace la courbe suivante : le pourcentage de liaison de la vitronectine humaine en fonction du logarithme de la concentration de chaque produit testé.

Pour chaque produit on détermine l'$IC_{50}$ suivant la formule suivante :

$$IC_{50} = (BO + Bmin)/2$$

BO = Maximum de liaison en l'absence de tout produit
Bmin = Minimum de liaison en présence de la concentration la plus élevée de produit.

**2 - Test du calvarium de souris**

<u>Principe</u>

Injection d'une dose traceuse de $^{45}Ca$ ($CaCl_2$) à des souris femelles en gestation afin d'étudier la résorption osseuse en mesurant la libération de $^{45}Ca$ à partir des calottes crâniennes des nouveaux-nés.

<u>But</u>

Détermination de l'activité d'une molécule sur la résorption osseuse, étude ex-vivo.

Produits

1) Produit à tester :

Véhicule : DMSO, H20/BSA (0,1 %)
Dose : Variables (10 μM en screening).

2) Produits de référence :

Echistatine (réf. H-9010-BACHEM)
Véhicule : H20/BSA
Dose : 10 μM.

3) Traceur radioactif :

$^{45}Ca$ sous forme de solution aqueuse de $CaCl_2$-réf. CES3 AMERSHAM ou NEZ-013 NEN.
Véhicule : Sérum physiologique
Dose : 25 μCi/souris/0,4 ml
Milieu de culture

CMRL 1066 avec rouge de phénol (réf. 041-01535 M/GIBCO) supplémenté par 0,1 % BSA et de la pénicilline/streptomycine. Méthode

1) Injection de $^{45}Ca$ aux souris gestantes (OF1, souche : Swiss)

a) Préparation de la solution marquée :

190 μl de la solution mère de calcium à 2 mci/ml est ajoutée à 6 ml de sérum physiologique.

b) Injection :

Au 17ème jour de gestation les souris reçoivent 400 μl de cette solution par voie intraveineuse soit 25 μCi/souris.

2) Prélèvement du tissu (calotte crânienne (calvarium))

Six jours après leur naissance les nouveaux-nés sont décapités, puis la tête est récupérée, et la peau incisée de la nuque au front. La calotte crânienne est prélevée par découpage aux ciseaux et à l'aide d'un emporte-pièce, deux demi-calvaria exactement identiques (un à gauche et un à droite) sont sectionnés dans les os pariétaux. L'un servira de témoin, l'autre sera utilisé pour tester le produit à étudier.

3) Phase de "rinçage"

Chaque demi calvarium est déposé dans un puits d'une plaque 24 puits, contenant 1 ml de milieu, sur un support de polyéthylène et nylex 100 µm, afin d'éviter tout contact avec le fond du puits.
Après 24 heures, les supports polyéthylène portant les calvaria sont transférés dans des nouvelles plaques 24 puits contenant 1 ml de milieu frais et les produits à tester ou leurs solvants. 200 µl de milieu des premières plaques sont prélevés dans chaque puits et un premier comptage de la radioactivité est effectué (valeur A).
Ce changement de milieu permet d'éliminer tout le stress mécanique lié au prélèvement.

4) Phase de "résorption"

48 heures après la mise en contact des tissus avec les produits étudiés, 200 µl de milieu sont prélevés dans chaque puits et comptés (valeur B), de façon à déterminer la quantité de $^{45}$Ca libérée dans le milieu pendant la phase dite de résorption.
Le calvarium est alors totalement déminéralisé dans 1 ml d'acide trichloracétique 5 % et après digestion, 200 µl sont également prélevés et comptés de façon à déterminer la quantité de calcium restant dans l'os (valeur C).

Expression des résultats

On calcul un % de résorption osseuse pour chaque demi-calvarium (chaque puits) de la façon suivante :

% résorption osseuse = dpm B/dpm (A+B+C) x 100

La somme des dpm A+B+C représente la quantité de $45_{Ca}$ incorporé dans chaque pièce osseuse le jour du prélèvement.
Pour mesurer l'effet d'un produit on fait pour chaque point le rapport du pourcentage de résorption osseuse du puits traité et du puits témoin correspondant. La valeur trouvée appelée indice de résorption est comprise entre 0 et 1 si le produit inhibe la résorption osseuse et est >1 si le produit la potentialise. On fait alors la moyenne des 6 indices (puisqu'il y a 6 points/groupe) de chaque produit ce qui donne un indice/produit. Si on retranche cet indice de la valeur 1, on obtient le pouvoir d'inhibition du produit, que l'on peut exprimer en pourcentage.
Par ailleurs un test statistique (Student T-test) est effectué en comparant point/point les indices de résorption individuels.

RESULTATS :

| Exemples | Test de compétition binding Vn/VR (($\alpha_v\beta_3$) $IC_{50}$ en µM | Calvarien de souris % d'inhibition à 10 µM |
|---|---|---|
| EX. 2 | 0,45 | 19 |
| EX. 3 | 2,1 | - |
| EX. 6 | 0,11 | 7,5 |
| EX. 8 | 2,79 | - |
| EX. 10 | 0,8 | 8 |
| EX. 11 | 0,05 | 7 |
| EX. 22 | 2,36 | - |
| EX. 12 | 0,35 | 12 |
| EX. 14 | 0,75 | 14 |
| EX. 24 | 0,03 | 18 |
| EX. 20 | 0,079 | 11 |
| EX. 21 | 0,037 | - |

(suite)

| Exemples | Test de compétition binding Vn/VR (($\alpha_v\beta_3$) $IC_{50}$ en µM | Calvarien de souris % d'inhibition à 10 µM |
|---|---|---|
| EX. 25 | 0,013 | 26 |
| EX. 26 | 0,006 | 30 |
| EX. 27 | 0,170 | 39 |
| EX. 28 | 0,015 | 27 |
| EX. 29 | 0,028 | 18 |
| EX. 31 | 0,055 | 20 |
| EX. 32 | 0,035 | - |

**Revendications**

**1.** Composés de formule générale (I) :

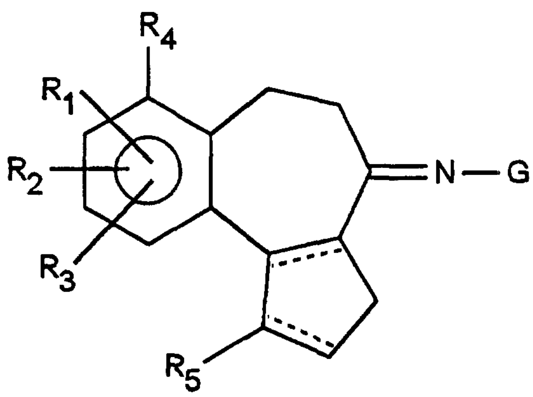

(I)

dans laquelle $R_1$ représente un groupement $-C\equiv C-[A]-[B]-COR_6$, $-CH=CH-[A]-[B]-COR_6$, $-(CH_2)_2-[A]-[B]-COR_6$, $-O-[A]-[B]-COR_6$, $-CH_2CO-[A]-[B]-COR_6$, -[A]- représentant

- soit un radical hydrocarboné bivalent dérivé d'une structure linéaire ou ramifiée, saturé ou insaturé, comportant de 1 à 12 atomes de carbone et de 1 à 6 hétéroatomes choisis parmi les atomes d'oxygène, d'azote ou de soufre,
- soit un radical bivalent dérivé d'un hydrocarbure acyclique, linéaire ou ramifié, saturé ou insaturé, comportant de 1 à 12 atomes de carbone,

[B] représentant un radical phényle, un radical CH(Z), ou une simple liaison,
Z représente un atome d'hydrogène, un groupement
$(D)_{0-6}$-NRaRb, $(D)_{0-6}$-NH-$SO_2$-Rc, $(D)_{0-6}$-NH-$CO_2$-Rc,
$(D)_{0-6}$-NH-CO-Rc, $(D)_{0-6}$-NH-$SO_2$-NH-Rc, $(D)_{0-6}$-NH-CO-NH-Rc,
$(D)_{0-6}$-$CO_2$-Rc, $(D)_{0-6}$-$SO_2$-Rc, $(D)_{0-6}$-CO-Rc ou $(D)_{0-6}$-Rc dans lesquels $(D)_{0-6}$ est un radical bivalent dérivé d'un hydrocarbure acyclique, linéaire ou ramifié, saturé ou insaturé, comportant de 0 à 6 atomes de carbone,
Ra, Rb et Rc représentent un atome d'hydrogène, un radical $(CH_2)_{0-3}$-Ar dans lequel Ar représente un groupement aryle carbocyclique renfermant de 6 à 18 atomes de carbone, un radical $(CH_2)_{0-3}$-Het dans lequel Het représente un radical dérivé d'un hétérocycle aromatique ou non aromatique, saturé ou non saturé, comportant de 1 à 9 atomes de carbone et de 1 à 5 hétéroatomes choisis parmi les atomes d'oxygène, d'azote ou de soufre, un radical $(CH_2)_{0-3}$-Alk dans lequel Alk représente radical dérivé d'un hydrocarbure, non aromatique, linéaire, ramifié ou cyclique, saturé ou insaturé, et comportant de 1 à 12 atomes de carbone, les radicaux Het, Ar et Alk pouvant être non substitués ou substitués, ou encore, Ra et Rb représentent ensemble avec l'atome d'azote auquel ils sont liés un hétérocycle azoté, aromatique ou non aromatique, saturé ou insaturé, renfermant éventuellement un ou plusieurs hétéroatomes choisis parmi les atomes d'oxygène, d'azote ou de soufre, ce radical pouvant être substitué ou non substitué,

- $R_6$ représente un radical hydroxyle, un radical O-Alk, O-Ar, $NH_2$, NH-Alk, $N(Alk)_2$ ou le reste d'un amino acide L ou D,
  Alk et Ar étant tels que définis précédemment et pouvant être substitués ou non substitués,
- $R_2$ et $R_3$ identiques ou différents représentent ou bien un atome d'hydrogène, un radical hydroxyle, un radical O-Alk ou un radical $O-(CH_2)_{0-3}$-Ar, Alk et Ar étant tels que définis précédemment, ou bien $R_2$ et $R_3$ forment ensemble un cycle du type $-O-(CRdRe)_n-O-$, n étant un entier de 1 à 5, Rd et Re indépendamment l'un de l'autre représente un atome d'hydrogène, un radical alkyle renfermant de 1 à 6 atomes de carbone, ou un radical phényle,
- $R_4$ représente un atome d'hydrogène, un atome d'halogène, un groupement hydroxyle, amino, nitro, cyano, CF3, acyle ou acyloxy renfermant de 1 à 12 atomes de carbone alkyle, alkényle, alkynyle, alkylthio, alkoxy, alkylamino, dialkylamino, dialkylaminoalkyle, dialkylaminoalkyloxy, dans lesquels le terme alkyle renferme de 1 à 6 atomes de carbone,
- $R_5$ représente un atome d'hydrogène, un radical hydroxyle, un atome d'halogène, un radical O-Alk ou un radical $O-(CH_2)_{0-3}$-Ar, Alk et Ar étant tels que définis précédemment,
- G représente,
  **soit** un radical de formule G1

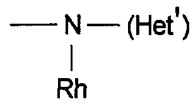

dans lequel Rh est un atome d'hydrogène ou un groupement Alk tel que défini précédemment et (Het') est un hétérocycle de formule générale :

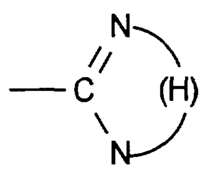

dans lequel (H) forme, avec le motif N=C-NH-, le reste d'un hétérocycle aromatique ou non aromatique, mono ou bicyclique, saturé ou non saturé, comportant de 1 à 9 atomes de carbone et de 2 à 5 hétéroatomes choisis parmi les atomes d'oxygène, d'azote et de soufre, ce radical pouvant être substitué ou non substitué,

- **soit** un radical NRaRb (radical G2), Ra et Rb étant tels que définis plus haut,
- **soit** un radical (Het) (radical G3) tel que défini plus haut,
- **soit** un radical -NRh-C(=X)-NHRc (radical G4), dans lequel X est un atome de soufre, d'oxygène ou NH, Rh et Rc sont tels que définis précédemment,
- **soit** un radical $-NRh-SO_2Rc$, (radical G5), dans lequel Rh et Rc sont tels que définis précédemment,
  les traits en pointillés représentent une éventuelle seconde liaison, ainsi que les sels d'addition avec les acides et les bases et les esters,
  $R_1$, $R_2$ et $R_3$ pouvant être en position 8, 9 ou 10 du tricycle, les substituants éventuels des radicaux (Alk), (Ar), (Het), (Het') ou NRaRb formant un hétérocycles pouvant être
- halogène
- alkyle, alkényle, alkynyle renfermant de 1 à 12 atomes de carbone,
- oxo, cyano, nitro, formyl, carboxy et carboxyalkyle renfermant de 1 à 6 atomes de carbone, carboxamide,
- alkoxy renfermant de 1 à 12 atomes de carbone,
- alkylthio renfermant de 1 à 12 atomes de carbone,
- amino, alkylamino renfermant de 1 à 12 atomes de carbone, dialkylamino renfermant de 2 à 24 atomes de carbone,
- aminoalkyle renfermant de 1 à 12 atomes de carbone,
- dialkylaminoalkyle renfermant de 3 à 25 atomes de carbone,

- dialkylaminoalkyloxy renfermant de 3 à 25 atomes de carbone,
- hydroxyle éventuellement acylé renfermant de 1 à 12 atomes de carbone,
- acyle renfermant de 1 à 12 atomes de carbone ou benzoyle éventuellement substitués par un atome de chlore, d'iode ou de fluor,
- aryle,carbocyclique ou hétérocyclique, ou aralkyle éventuellement substitués par halogène, alkyle, alkoxy, alkylthio, aminoalkyle ou dialkylamino indiqués ci-dessus.

**2.** Composés de formule générale (I) telle que définie à la revendication 1, répondant à la formule générale (I') :

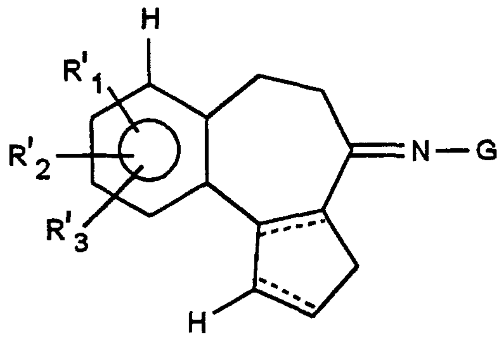

(I')

dans laquelle $R'_1$ représente un groupement $-C{\equiv}C-[A']-[B']-COR'_6$, $-CH{=}CH-[A']-[B']-COR'_6$, $-(CH_2)_2-[A']-[B']-COR'_6$, $-O-[A']-[B']-COR'_6$, $-CH_2CO-[A']-[B']-COR'_6$, -[A']- représentant un radical bivalent alkylène, alkénylène ou alkynylène renfermant de 1 à 6 atomes de carbone, [B'] représentant un radical CH(Z') ou une simple liaison, Z' représente un atome d'hydrogène, un groupement $(CH_2)_{0-6}$-NRaRb, $(CH_2)_{0-6}-NH-SO_2$-Rc, $(CH_2)_{0-6}-NH-CO_2$-Rc, $(CH_2)_{0-6}$-NH-CO-Rc, $(CH_2)_{0-6}-NH-SO_2$-NH-RC, $(CH_2)_{0-6}$-NH-CO-NH-Rc, $(CH_2)_{0-6}-CO_2$-Rc, $(CH_2)_{0-6}-SO_2$-Rc, $(CH_2)_{0-6}$-CO-Rc ou $(CH_2)_{0-6}$-Rc, Ra, Rb et Rc étant tels que définis à la revendication 1, $R'_6$ représente un radical OH, amino ou alkoxy renfermant de 1 à 8 atomes de carbone, éventuellement substitué par un ou plusieurs radicaux choisi parmi les radicaux hydroxy, amino, phénylalkylamino ou dialkylamino, $R'_2$ et $R'_3$ représentent un atome d'hydrogène ou un radical méthoxy, et G est tel que défini à la revendication 1, les traits en pointillés représentent une éventuelle seconde liaison, ainsi que les sels d'addition avec les acides et les bases et les esters.

**3.** Composés de formule générale (I) telle que définie à la revendication 1 ou 2 dans laquelle $R_6$ représente un groupement -OH, $-OCH_3$, $-OCH_2CH_3$, $-O-(CH_2)_2-OH$,

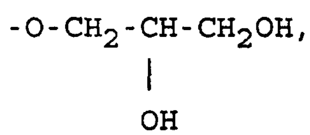

$-O-(CH_2)_2-NH_2$, $-O-(CH_2)_2-N-(CH_3)_2$, $-NH_2$ ou $-O-(CH_2)$-phényle, ainsi que les sels d'addition avec les acides et les bases et les esters.

**4.** Composés de formule générale (I) telle que définie à la revendication 1, 2 ou 3, dans laquelle $R_1$ représente un groupement $O-(CH_2)_{0-6}$-CH(Z')-COOH ou $-(CH_2)_{0-7}$-CH(Z')-COOH, ainsi que les sels d'addition avec les acides et les bases et les esters.

**5.** Composés de formule générale (I) telle que définie à l'une quelconque des revendications 1 à 4, dans laquelle (Z') est un atome d'hydrogène, ainsi que les sels d'addition avec les acides et les bases et les esters.

**6.** Composés de formule générale (I) telle que définie à l'une quelconque des revendications 1 à 4, dans laquelle (Z') est le groupement $(CH_2)_{0-6}-NH-CO_2$-Rc ou $(CH_2)_{0-6}$-NH-Rb, Rb et Rc étant tels que définis à la revendication 1, ainsi que les sels d'addition avec les acides et les bases et les esters.

**7.** Composés de formule générale (I) telle que définie à la revendication 6, dans laquelle Rb et Rc sont les groupements $(CH_2)_{0\text{-}3}$-Ar ou $(CH_2)_{0\text{-}3}$-Alk, Ar et Alk étant tels que définis à la revendication 1 et pouvant être substitué ou non substitué, ainsi que les sels d'addition avec les acides et les bases et les esters.

**8.** Composés de formule générale (I) telle que définie à l'une des revendications 1 à 7, dans laquelle G est un groupement G4 de formule générale -NH-C(=NH)-NHRc, Rc étant tel que défini à la revendication 1, ainsi que les sels d'addition avec les acides et les bases et les esters.

**9.** Composés de formule générale (I) telle que définie à la revendication 8, dans laquelle Rc est un atome d'hydrogène, ainsi que les sels d'addition avec les acides et les bases et les esters.

**10.** Composés de formule générale (I) telle que définie à l'une des revendications 1 à 7, dans laquelle G est un groupement NH-(Het'), (Het') étant tel que défini à la revendication 1.

**11.** Composés de formule générale (I) telle que définie à la revendication 10, dans laquelle G représente les hétérocycles suivants :

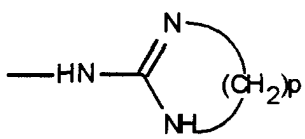
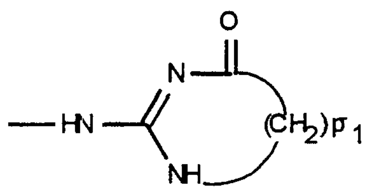
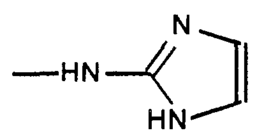

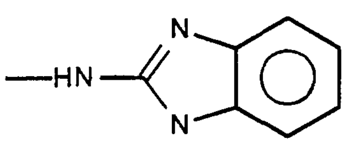
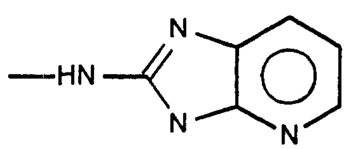
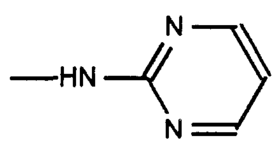

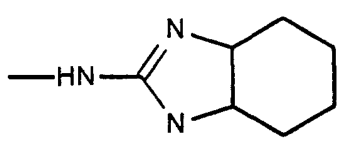

p étant un entier égal à 2, 3 ou 4, ces hétérocycles étant substitués ou non substitués, ainsi que les sels d'addition avec les acides et les bases et les esters.

**12.** Composés de formule générale (I) telle que définie à la revendication 10 ou 11, dans laquelle G est le groupement

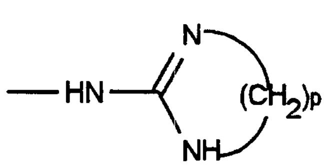

p étant un entier égal à 2, 3 ou 4, ainsi que les sels d'addition avec les acides et les bases et les esters.

13. Composés de formule (I) telle que définie à la revendication 1 dont les noms suivent :

- Acide 4-((4-((aminoiminométhyl)hydrazono)-9,10-diméthoxy-1, 2,3,4,5,6-hexahydro-8-benz(e)azulèn-yl) oxy)-butanoique,
- Acide 5-((4-((aminoiminométhyl)hydrazono)-9,10-diméthoxy-1, 2,3,4,5,6-hexahydro-8-benz(e)azulèn-yl) oxy)-pentanoique,
- Acide 5-((4-((aminoiminométhyl)hydrazono)-8,10-diméthoxy-1, 2,3,4,5,6-hexahydro-9-benz(e)azulèn-yl) oxy)-pentanoique,
- Acide 6-((4-((aminoiminométhyl)hydrazono)-9,10-diméthoxy-1, 2,3,4,5,6-hexahydro-8-benz(e)azulènyl)oxy)-hexanoique,
- Acide 7-((4-((aminoiminométhyl)hydrazono)-9,10-diméthoxy-1, 2,3,4,5,6-hexahydro-8-benz(e)azulènyl)oxy)-heptanoique,
- Acide 5-((9,10-diméthoxy-1,2,3,4,5,6-hexahydro-4-((4,5-dihydro-1H-imidazol-2-yl)hydrazono)-8-benz(e)azulènyl)oxy)pentanoique,
- chlorhydrate de 5-((4-((aminoiminométhyl)hydrazono)-9,10-diméthoxy-1, 2,3,4,5,6-hexahydro-8-benz(e)azulènyl)oxy)pentanoate d'éthyle,
- Acide 4-((4-((aminoiminométhyl)hydrazono)-8,9-diméthoxy-1, 2,3,4,5,6-hexahydro-10-benz(e)azulènyl)oxy)-butanoique,
- Acide 5-((4-((aminoiminométhyl)hydrazono)-8,9-diméthoxy-1, 2,3,4,5,6-hexahydro-10-benz(e)azulènyl)oxy)-pentanoique,
- Acide 5-((4-(((amino)carbonyl)hydrazono)-9,10-diméthoxy-1, 2,3,4,5,6-hexahydro-8-benz(e)azulènyl)oxy)-pentanoique,
- Acide 5-((4-(((amino)thiocarbonyl)hydrazono)-9,10-diméthoxy-1,2,3,4,5,6-hexahydro-8-benz(e)azulènyl) oxy)-pentanoique,
- Acide 4-((4-((aminoiminométhyl)hydrazono)-8,10-diméthoxy-1, 2,3,4,5,6-hexahydro-9-benz(e)azulèn-yl) oxy)-butanoique,
- Acide 6-((4-((4,5-dihydro-1H-imidazol-2-yl)hydrazono)-9,10-diméthoxy-1, 2,3,4,5,6-hexahydro-8-benz(e) azulènyl)oxy)hexanoique,
- Acide 5-((4-((aminoiminométhyl)hydrazono)-9,10-diméthoxy-1, 2,3,4,5,6-hexahydro-8-benz(e)azulènyl)oxy)-3,3-diméthyl-4-oxo-pentanoique,
- Acide 5-((4-((4,5-dihydro-1H-imidazol-2-yl)hydrazono)-9,10-diméthoxy-1, 2,3,4,5,6-hexahydro-8-benz(e) azulènyl)oxy)-3,3-diméthyl-4-oxo-pentanoique,
- Chlorhydrate de l'acide 5-((4-((aminoiminométhyl)hydrazono)-9,10-diméthoxy-1, 2,3,4,5,6-hexahydro-8-benz (e)azulènyl)oxy)-pentanoique,
- Acide 4-((4-((4,5-dihydro-1H-imidazol-2-yl)hydrazono)-9,10-diméthoxy-1, 2,3,4,5,6-hexahydro-8-benz(e) azulènyl)oxy)butanoique,
- Acide 5-((8((aminoiminométhyl)hydrazono)-6,7,8,9,10,11-hexahydro-azulèno(5,6-d)-1,3-benzodioxol-4-yl) oxy)pentanoique,
- Acide 5-((8((aminoiminométhyl)hydrazono)-2,2-diphényl-6,7,8,9,10,11-hexahydro-azulèno(4,5-e)-(1,3)-benzodioxol-4-yl)oxy)-pentanoique,
- Acide 4-((9,10-diméthoxy-4-((1,4,5,6-tétrahydro-2-pyrimidinyl)hydrazono)-1,2,3,4,5,6-hexahydro-8-benz(e) azulènyl) oxy)-butanoique,
- Acide 2-((4-((4,5-dihydro-1H-imidazol-2-yl)hydrazono)-9,10-diméthoxy-1,2,3,4,5,6-hexahydro-8-benz(e)azulènyl)oxy)-éthanoique,
- Acide 3-((4-((4,5-dihydro-1H-imidazol-2-yl)hydrazono)-9,10-diméthoxy-1,2,3,4,5,6-hexahydro-8-benz(e)azulènyl)oxy)-propanoique,
- Acide 4-((4-((4,5-dihydro-1H-imidazol-2-yl)hydrazono)1,2,3,4,5,6-hexahydro-8-benz(e)azulènyl)oxy)-butanoique,
- Acide 4-((4-((4,5-dihydro-1H-imidazol-2-yl)hydrazono)1,2,3,4,5,6-hexahydro-8-benz(e)azulènyl)oxy)-butanoique,
- O-[4[(4,5-dihydro-1H-imidazol-2-yl)hydrazono]-9,10-diméthoxy-1,2,3,4,5,6-hexahydro-8-benz[e]azulènyl]-N-[(phénylméthoxy)carbonyl]-DL-homoserine,
- O-[4[(4,5-dihydro-1H-imidazol-2-yl)hydrazono]-1,2,3,4,5,6-hexahydro-8-benz[e]azulènyl]-N-[(phénylméthoxy)carbonyl]-DL-homoserine,
- O-[4-[(1,2,3,4-tétrahydro 6-pyrimidinyl) hydrazono] 9,10-diméthoxy 1,2,3,4,5,6-hexahydro 8-benz[e]azulenyl] N-[(phénylméthoxy) carbonyl] DL-homoserine,

- ester (2,3-dihydroxypropylique) de 0-(9,10-diméthoxy 1,2,3,4,5,6,-hexahydro 4-[(1,4,5,6-tétrahydro 2-pyrimidinyl) hydrazono]-8-benz(e)azulényl] N-[(phénylméthoxy) carbonyl] DL-homoserine,
- O-[4-[(4,5-dihydro 1H-imidazol-2-yl) hydrazono] 9,10-diméthoxy 1,2,3,4,5,6-hexahydro 8-benz(e)azulenyl] N-[(8-quinoléinyl) sulfonyl] DL-homoserine,
- monochlorhydrate de O-[4-[(4,5-dihydro 1H-imidazol-2-yl) hydrazono] 9,10-diméthoxy 1,2,3,4,5,6-hexahydro 8-benz(e)azulényl] N-[[3-[4-(3-pyridinyl) 1H-imidazol-1-yl] propoxy] carbonyl] DL-homoserine,
- Acide 5-[[4-[(4,5-dihydro 4-oxo 1H-imidazol-2-yl) hydrazono] 9,10-diméthoxy 1,2,3,4,5,6-hexahydro 8-benz(e)azulényl] oxy] pentanoïque,
- O-[9,10-diméthoxy 1,2,3,4,5,6-hexahydro 4-[(4,5,6,7-tétrahydro 1H-1,3-diazépin-2-yl) hydrazono] 8-benz(e)-azulényl] N-[(phénylméthoxy) carbonyl] DL-homoserine,
- O-[9,10-diméthoxy 1,2,3,4,5,6-hexahydro 4-[(3a,4,5,6,7,7a-hexahydro 1H-benzimidazol-2-yl) hydrazono] 8-benz(e)azulényl] N-[(phénylméthoxy) carbonyl] DL-homoserine.

**14.** Procédé de préparation des composés de formule (I) telle que définie à la revendication 1 **caractérisé en ce que** l'on soumet un composé de formule (II) :

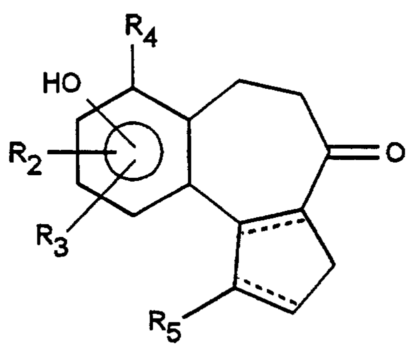

(II)

dans laquelle $R_2$, $R_3$, $R_4$ et $R_5$ sont tels que définis à la revendication 1 à l'exception de hydroxyle
soit à l'action d'un composé de formule (F1) en présence d'une base,

Hal-[A]-[B]-$COR_6$ (F1)

ou d'un composé de formule ($F'_1$) en présence d'une phosphine et d'azodicarboxylate de diéthyle :

OH-[A]-[B]-$COR_6$ ($F'_1$)

dans lesquelles Hal est un atome d'halogène, [A], [B] et $R_6$ sont tels que décrits précédemment, [B] pouvant également représenter le groupement

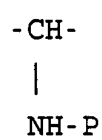 ,

P étant un groupement protecteur de la fonction amine, afin d'obtenir un composé de formule (IIIa) :

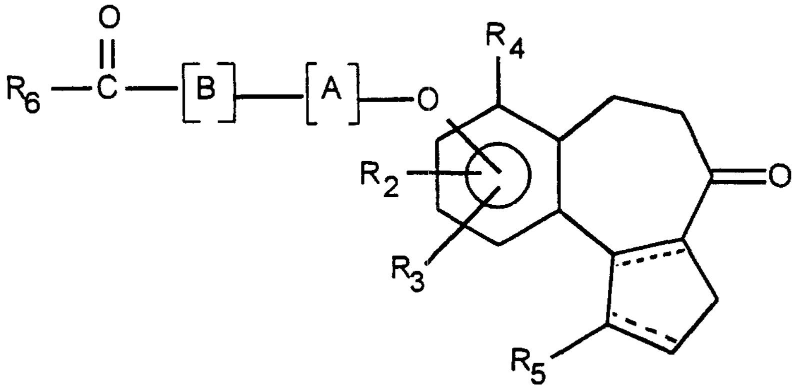

(IIIa)

soit à l'action d'un groupement activant puis d'un composé de formule (F2) en présence d'un catalyseur :

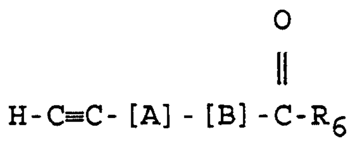

(F2)

afin d'obtenir un composé de formule (IIIb) :

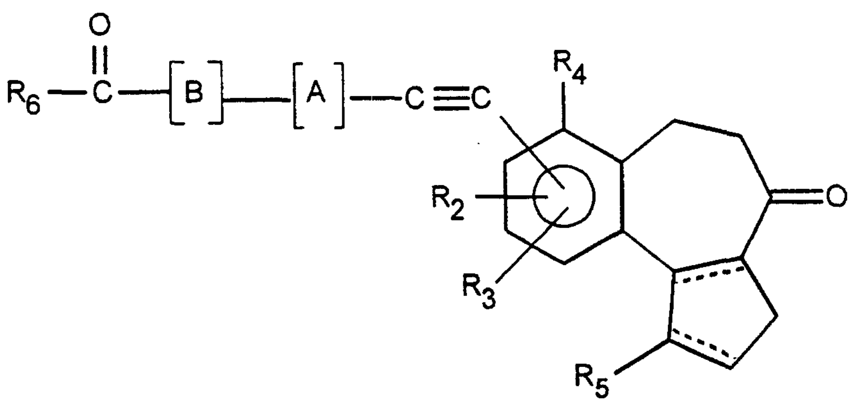

(IIIb)

composés de formule (IIIa) ou (IIIb) que l'on soumet à l'action d'un composé de formule (F3) :

$H_2N\text{-}G$ (F3)

dans laquelle G est tel que défini à la revendication 1, afin d'obtenir les composés de formule (IVa) et (IVb) correspondant à certains produits de formule (I) :

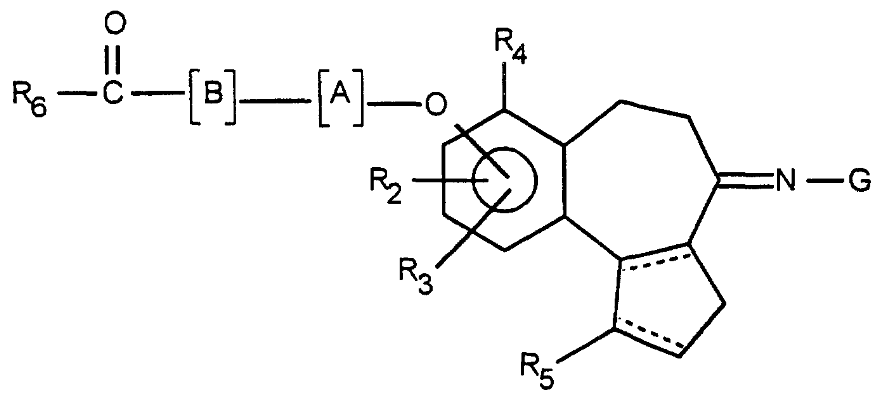

(IVa)

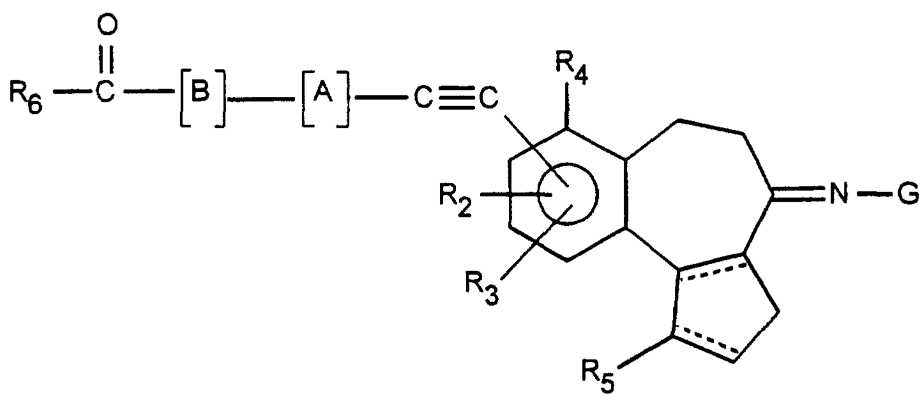

(IVb)

que l'on soumet le cas échéant, dans un ordre approprié, à l'une ou plusieurs des réactions suivantes :

- action d'une base ou d'un acide afin de cliver l'ester et obtenir l'acide correspondant,
- action d'un agent réducteur apte à réduire partiellement ou totalement les insaturations,
- à l'action d'un agent d'hydratation de la triple liaison,
- à l'action d'un réactif de déalkylation,
- à l'action d'un agent de déprotection de la fonction NH-P en bêta de $CO-R_6$ lorsque [B] représente le groupe CH-NHP,
- à la formation du groupement $NH-SO_2R_c$, $NH-CO_2R_c$, $NHCOR_c$, $NH-SO_2-NH-R_c$, $NH-CO-NHR_c$ à partir de l'amine correspondante en bêta de $COR_6$, pour obtenir les composés de formule (I) correspondant que l'on soumet le cas échéant à l'action d'un acide ou d'une base afin d'obtenir les sels correspondants ou à l'action d'un agent d'estérification afin d'obtenir les esters correspondants.

**15.** Procédé de formation des composés de formule (I) telle que définie à la revendication 1, **caractérisé en ce que** l'on soumet préalablement un composé de formule (II) à l'action d'un composé de formule (F3) afin d'obtenir un composé de formule (IIIc) :

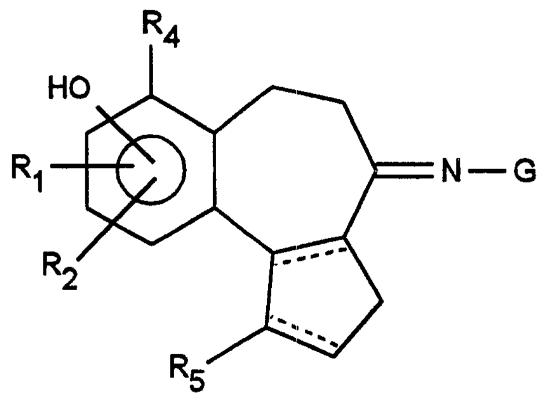

(IIIc)

composé de formule (IIIc) que l'on soumet après le cas échéant protection de G, à l'action d'un composé de formule (F1), (F'$_1$) ou (F2) puis le cas échéant à une réaction de déprotection de G, afin d'obtenir les composés de formule (IVa) et (IVb) correspondants que l'on soumet ensuite, le cas échéant, aux différentes réactions telles que définies à la revendication 14, les composés de formules (II), (F1), (F'$_1$), (F2), (F3), (IVa) et (IVb) étant tels que définis à la revendication 14.

**16.** Procédé de préparation des produits de formule (II) telle que définie à la revendication 14, dans laquelle $R_2$, $R_3$, $R_4$ et $R_5$ sont des atomes d'hydrogène et OH est en position 8, 9 ou 10, **caractérisé en ce que**

(i) on soumet un composé de formule (a) :

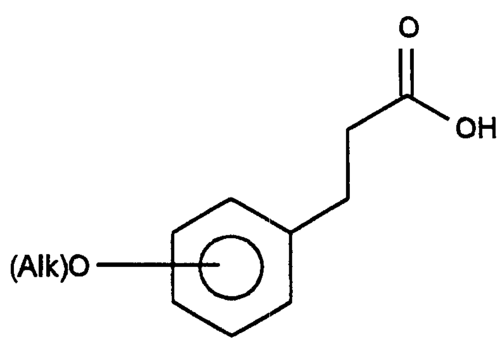

(a)

dans laquelle O-(Alk) est en position méta ou para du groupement alkylcarboxylique, (Alk) étant tel que défini à la revendication 1, à l'action d'un agent d'halogénation pour obtenir l'halogénure d'acyle correspondant,
(ii) que l'on soumet à l'action d'un réactif de formule (b) :

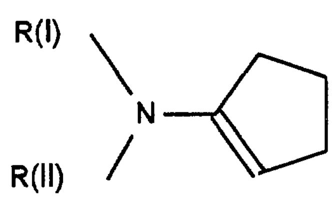

(b)

dans laquelle $R_{(I)}$ et $R_{(II)}$, identiques ou différents représentent un groupement alkyle renfermant de 1 à 6 atomes de carbone, ou $R_{(I)}$ et $R_{(II)}$ ensemble avec l'atome d'azote auquel ils sont liés, représentent un hétérocycle à 5 ou 6 chaînons, saturé ou insaturé, renfermant éventuellement un autre hétéroatome choisi parmi O et N, pour obtenir un composé de formule (c) :

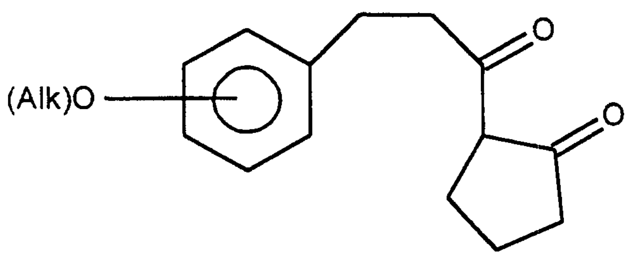

(c)

(iii) que l'on soumet à l'action d'un agent d'halogénation pour obtenir un composé de formule (d) :

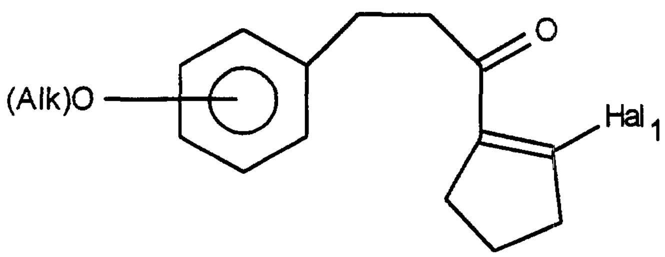

(d)

dans laquelle $Hal_1$ représente un atome d'halogène.
(iv) que l'on soumet à l'action d'un acide de Lewis, pour obtenir un composé de formule (e) :

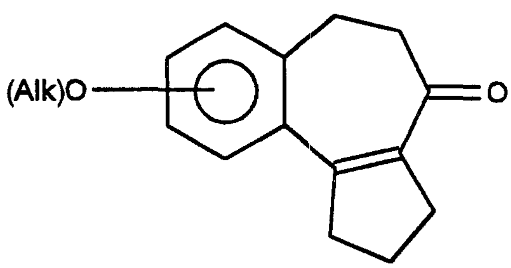

(e)

(v) que l'on soumet à un réactif de déalkylation afin d'obtenir le produit de formule (IIF) correspondant au produit de formule (II) monosubstitué attendu :

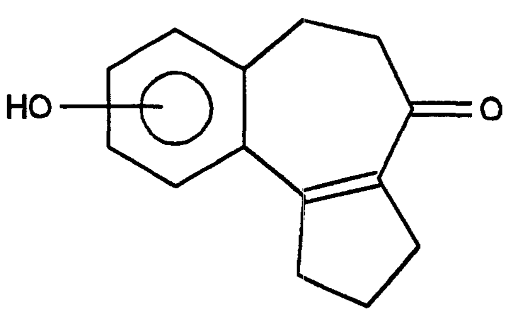

(IIF)

**17.** Procédé de préparation des composés de formule générale (II) telle que définie à la revendication 14 et dans laquelle $R_2$ est soit un groupement OAlk, soit un groupement $O-(CH_2)_{0-3}-Ar$ et $R_3$, $R_4$ et $R_5$ sont des atomes

d'hydrogène, OH et $R_2$ étant en position 8, 9 ou 10, **caractérisé en ce que** on soumet le composé de formule générale (a') :

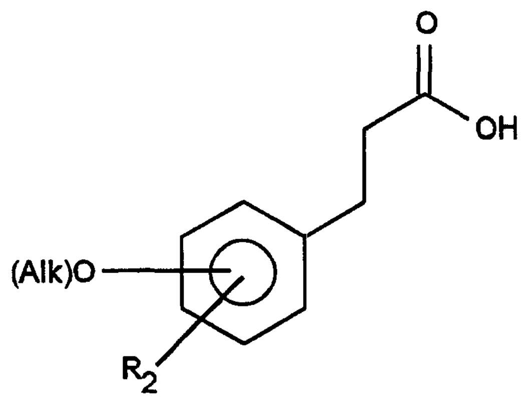

(a')

dans laquelle $R_2$ et O-(Alk) sont en position méta ou para du groupement alkylcarboxylique, successivement aux réactions (i), (ii), (iii), (iv) et (v) afin d'obtenir le produit de formule (IIG) correspondant au produit de formule (II) bisubstitué attendu :

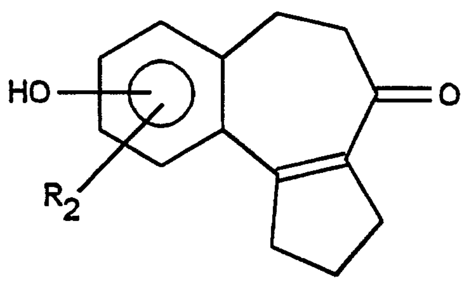

(IIG)

**18.** Procédé de préparation des composés de formule générale (II) telle que définie à la revendication 14 et dans laquelle $R_2$ et $R_3$ représentent un groupement O-(Alk) ou O-$(CH_2)_{0-3}$-(Ar), $R_4$ et $R_5$ sont des atomes d'hydrogène, OH étant en position 9 **caractérisé en ce que** l'on soumet le composé de formule (IIA) :

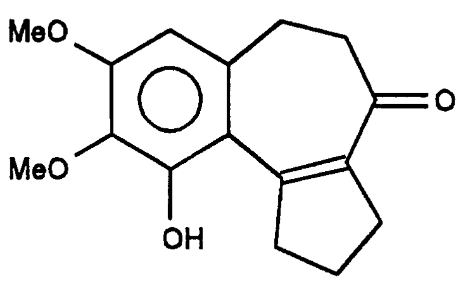

(IIA)

à l'action d'un réactif de déalkylation, afin d'obtenir le composé de formule (IIB) :

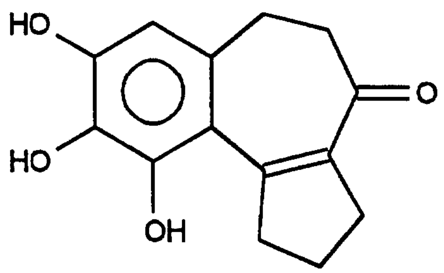

(IIB)

composé de formule (IIB) que l'on soumet :

**soit** à l'action d'un réactif de protection des diols en milieu basique, afin d'obtenir sélectivement le produit de formule (IIC) :

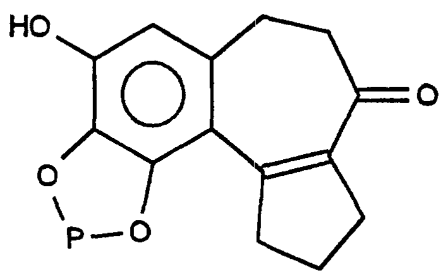

(IIC)

dans laquelle P représente le reste d'un réactif de protection des diols,

que l'on soumet successivement à l'action d'un réactif de protection du phénol, d'un réactif de déprotection des diols, d'un agent d'alkylation puis d'un agent de déprotection du phénol afin d'obtenir le composé de formule (IID) correspondant au produit de formule (II) trisubstitué avec OH en position 8 :

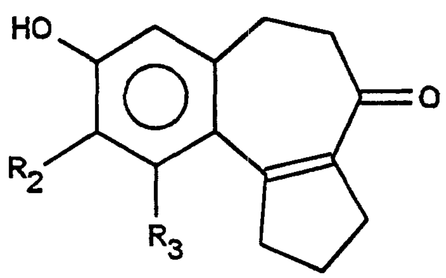

(IID)

**soit** à l'action successivement d'un agent de protection du phénol, d'un agent d'alkylation puis d'un agent de déprotection afin d'obtenir le composé de formule (IIE) correspondant au produit de formule (II) trisubstitué avec OH en position 9

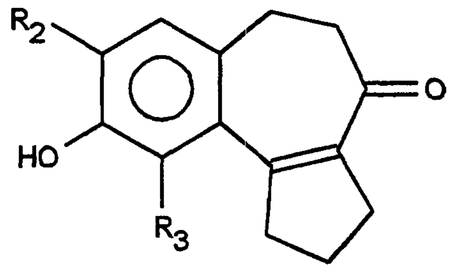

(IIE)

**19.** A titre de médicament, les composés de formule (I) telle que définie à l'une quelconque des revendications 1 à 12, ainsi que leurs sels d'addition et leurs esters pharmaceutiquement acceptables.

**20.** A titre de médicament, les composés de formule (I) tels que définis à la revendication 13.

**21.** Les compositions pharmaceutiques renfermant comme principe actif au moins l'un des médicaments définis à la revendication 19 ou 20.

**22.** A titre de produits intermédiaires nouveaux, les composés de formules générales (IIIa), (IIIb), (IIIc) et (II) telles que définies à l'une des revendication 14 à 18 étant entendu que les composés de formule (IIc) et les composés :

- 2, 3, 5, 6-tétrahydro-9,10-diméthoxy-8-hydroxy-benz[e]azulèn-4(1H)-one,
- et du 2, 3, 5, 6-tétrahydro-8,9-diméthoxy-10-hydroxy-benz[e]azulèn-4(1H)-one, sont exclus.

**Patentansprüche**

**1.** Verbindungen der allgemeinen Formel (I)

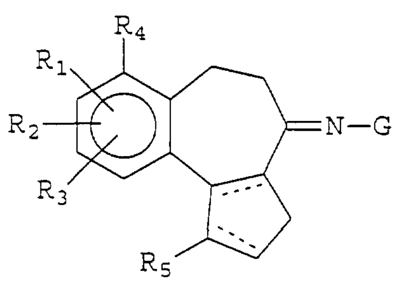

(I)

in der $R_1$ eine Gruppe $-C{\equiv}C-[A]-[B]-COR_6$, $-CH{=}CH-[A]-[B]-COR_6$, $-(CH_2)_2-[A]-[B]-COR_6$, $-O-[A]-[B]-COR_6$, $-CH_2CO-[A]-[B]-COR_6$, darstellt und -[A]- bedeutet:

- entweder einen bivalenten Kohlenwasserstoff-Rest, abgeleitet von einer geraden oder verzweigten, gesättigten oder ungesättigten Struktur mit 1 bis 12 Kohlenstoffatomen und 1 bis 6 Heteroatomen, ausgewählt unter den Atomen von Sauerstoff, Stickstoff oder Schwefel,
- oder einen bivalenten Rest, abgeleitet von einem acyclischen, geraden oder verzweigten, gesättigten oder ungesättigten Kohlenwasserstoff mit 1 bis 12 Kohlenstoffatomen,

[B] einen Rest Phenyl, einen Rest CH(Z) oder eine einfache Bindung darstellt,
Z ein Wasserstoffatom, eine Gruppe $(D)_{0-6}$-NRaRb, $(D)_{0-6}$-NH-$SO_2$-Rc, $(D)_{0-6}$-NH-$CO_2$-Rc, $(D)_{0-6}$-NH-CO-Rc, $(D)_{0-6}$-NH-$SO_2$-MH-Rc,
$(D)_{0-6}$-NH-CO-NH-Rc, $(D)_{0-6}$-$CO_2$-Rc, $(D)_{0-6}$-$SO_2$-Rc, $(D)_{0-6}$-CO-Rc oder
$(D)_{0-6}$-Rc bedeutet, worin $(D)_{0-6}$ ein bivalenter Rest ist, abgeleitet von einem acyclischen, geraden oder verzweigten, gesättigten oder ungesättigten Kohlenwasserstoff mit 0 bis 6 Kohlenstoffatomen,

Ra, Rb und Rc ein Wasserstoffatom, einen Rest $(CH_2)_{0-3}$-Ar, worin Ar eine carbocyclische Gruppe Aryl mit 6 bis 18 Kohlenstoffatomen ist, einen Rest $(CH_2)_{0-3}$-Het, worin Het ein von einem aromatischen oder nicht aromatischen, gesättigten oder ungesättigten Heterocyclus mit 1 bis 9 Kohlenstoffatomen und 1 bis 5 Heteroatomen, ausgewählt unter den Atomen von Sauerstoff, Stickstoff oder Schwefel, abgeleiteter Rest ist, einen Rest $(CH_2)_{0-3}$-Alk, worin Alk ein von einem nicht aromatischen, geraden, verzweigten oder cyclischen, gesättigten oder ungesättigten Kohlenwasserstoff mit 1 bis 12 Kohlenstoffatomen abgeleiteter Rest ist, wobei die Reste Het, Ar und Alk substituiert oder nicht substituiert sein können, darstellen,

oder Ra und Rb auch gemeinsam mit dem Stickstoffatom, an das sie gebunden sind, einen aromatischen oder nicht aromatischen, gesättigten oder ungesättigten Stickstoff-Heterocyclus bedeuten, der gegebenenfalls ein oder mehrere Heteroatome umfaßt, ausgewählt unter den Atomen von Sauerstoff, Stickstoff oder Schwefel, wobei dieser Rest substituiert oder nicht substituiert sein kann,

- $R_6$ einen Rest Hydroxyl, einen Rest O-Alk, O-Ar, $NH_2$, NH-Alk, $N(Alk)_2$ oder den Rest einer Aminosäure L oder D darstellt, wobei Alk und Ar wie vorstehend definiert sind und substituiert oder nicht substituiert sein können,
- $R_2$ und $R_3$, gleich oder verschieden, entweder ein Wasserstoffatom, einen Rest Hydroxyl, einen Rest O-Alk oder einen Rest O-$(CH_2)_{0-3}$-Ar bedeuten, worin Alk und Ar wie vorstehend definiert sind, oder $R_2$ und $R_3$ auch zusammen einen Ring vom Typ -O-$(CRdRe)_n$-O- bilden, worin n eine ganze Zahl von 1 bis 5 ist, Rd und Re unabhängig voneinander ein Wasserstoffatom, einen Rest Alkyl mit 1 bis 6 Kohlenstoffatomen oder einen Rest Phenyl bedeuten,
- $R_4$ ein Wasserstoffatom, ein Halogenatom, eine Gruppe Hydroxyl, Amino, Nitro, Cyano, $CF_3$, Acyl oder Acyloxy mit 1 bis 12 Kohlenstoffatomen, Alkyl, Alkenyl, Alkinyl, Alkylthio, Alkoxy, Alkylamino, Dialkylamino, Dialkylaminoalkyl, Dialkylaminoalkyloxy darstellt, worin der Ausdruck Alkyl 1 bis 6 Kohlenstoffatome umfaßt,
- $R_5$ ein Wasserstoffatom, ein Rest Hydroxyl, ein Halogenatom, ein Rest O-Alk oder ein Rest O-$(CH_2)_{0-3}$-Ar ist, worin Alk und Ar wie vorstehend definiert sind,
- G darstellt:

  entweder einen Rest der Formel G1

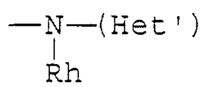

  worin Rh ein Wasserstoffatom oder eine wie vorstehend definierte Gruppe Alk ist und (Het') einen Heterocyclus der allgemeinen Formel

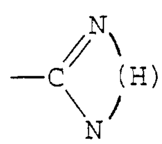

  bedeutet, worin (H) zusammen mit der Struktureinheit N=C-NH- den Rest eines aromatischen oder nicht aromatischen, mono- oder bicyclischen, gesättigten oder ungesättigten Heterocyclus mit 1 bis 9 Kohlenstoffatomen und 2 bis 5 Heteroatomen bildet, ausgewählt unter den Atomen von Sauerstoff, Stickstoff und Schwefel, wobei dieser Rest substituiert oder nicht substituiert sein kann,
- oder einen Rest NRaRb (Rest G2), worin Ra und Rb wie oben definiert sind,
- oder einen Rest (Het) (Rest G3), wie oben definiert,
- oder einen Rest -NRh-C(=X)-NHRc (Rest G4), worin X ein Atom von Schwefel oder Sauerstoff oder NH ist und Rh und Rc wie vorstehend definiert sind,
- oder einen Rest -NRh-$SO_2R_c$ (Rest G5), worin Rh und Rc wie vorstehend definiert sind, wobei die punktierten Linien eine eventuelle zweite Bindung darstellen, sowie die Additionssalze mit Säuren und Basen und die Ester, und

$R_1$, $R_2$ und $R_3$ sich in Position 8, 9 oder 10 des Tricyclus befinden können sowie die eventuellen Substituenten der Reste (Alk), (Ar), (Het), (Het') oder NRaRb, die einen Heterocyclus bilden, sein können

- Halogen,
- Alkyl, Alkenyl, Alkinyl mit 1 bis 12 Kohlenstoffatomen,
- Oxo, Cyano, Nitro, Formyl, Carboxy und Carboxyalkyl mit 1 bis 6 Kohlenstoffatomen, Carboxamid,
- Alkoxy mit 1 bis 12 Kohlenstoffatomen,
- Alkylthio mit 1 bis 12 Kohlenstoffatomen,
- Amino, Alkylamino mit 1 bis 12 Kohlenstoffatomen, Dialkylamino mit 2 bis 24 Kohlenstoffatomen,
- Aminoalkyl mit 1 bis 12 Kohlenstoffatomen,
- Dialkylaminoalkyl mit 3 bis 25 Kohlenstoffatomen,
- Dialkylaminoalkyloxy mit 3 bis 25 Kohlenstoffatomen,
- Hydroxyl, gegebenenfalls acyliert mit 1 bis 12 Kohlenstoffatomen,
- Acyl mit 1 bis 12 Kohlenstoffatomen oder Benzoyl, gegebenenfalls substituiert durch ein Atom von Chlor, Iod oder Fluor,
- Aryl, carbocyclisch oder heterocyclisch, oder Aralkyl, gegebenenfalls substituiert durch Halogen, Alkyl, Alkoxy, Alkylthio, Aminoalkyl oder Dialkylamino wie oben angegeben.

**2.** Verbindungen der allgemeinen Formel (I) wie in Anspruch 1 definiert, entsprechend der allgemeinen Formel (I')

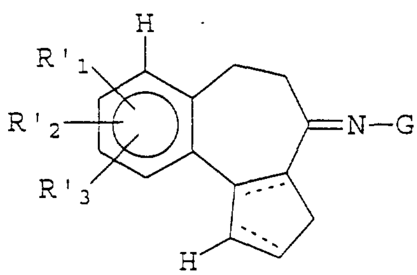

(I')

in der $R'_1$ eine Gruppe $-C{\equiv}C-[A']-[B']-COR'_6$,$-CH{=}CH-[A']-[B']-COR'_6$, - $(CH_2)_2-[A']-[B']-COR'_6$, $-O-[A']-[B']-COR'_6$, $-CH_2CO-[A']-[B']-COR'_6$, darstellt und -[A']- einen bivalenten Rest Alkylen, Alkenylen oder Alkinylen mit 1 bis 6 Kohlenstoffatomen bedeutet, [B'] einen Rest CH(Z') oder eine einfache Bindung darstellt, Z' ein Wasserstoffatom, eine Gruppe $(CH_2)_{0-6}$-NRaRb, $(CH_2)_{0-6}-NH-SO_2-Rc$, $(CH_2)_{0-6}-NH-CO_2-Rc$, $(CH_2)_{0-6}$-NH-CO-Rc, $(CH_2)_{0-6}-NH-SO_2-NH-Rc$, $(CH_2)_{0-6}$-NH-CO-NH-Rc, $(CH_2)_{0-6}-CO_2-Rc$, $(CH_2)_{0-6}-SO_2-Rc$, $(CH_2)_{0-6}$-CO-Rc oder $(CH_2)_{0-6}$-Rc bedeutet, worin Ra, Rb und Rc wie in Anspruch 1 definiert sind, $R'_6$ einen Rest OH, Amino oder Alkoxy mit 1 bis 8 Kohlenstoffatomen darstellt, gegebenenfalls substituiert durch einen oder mehrere Reste, ausgewählt unter den Resten Hydroxy, Amino, Phenylalkylamino oder Dialkylamino, $R'_2$ und $R'_3$ ein Wasserstoffatom oder einen Rest Methoxy bedeuten und G wie in Anspruch 1 definiert ist, wobei die punktierten Linien eine eventuelle zweite Bindung darstellen, sowie die Additionssalze mit Säuren und Basen und die Ester.

**3.** Verbindungen der allgemeinen Formel (I) wie in Anspruch 1 oder 2 definiert, worin $R_6$ eine Gruppe -OH, $-OCH_3$, $-OCH_2CH_3$, -O- $(CH_2)_2$-OH,

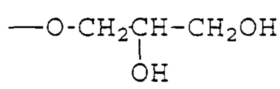 ,

$-O-(CH_2)_2-NH_2$, $-O-(CH_2)_2-N-(CH_3)_2$, $-NH_2$ oder $-O-(CH_2)$-phenyl darstellt, sowie die Additionssalze mit Säuren und Basen und die Ester.

**4.** Verbindungen der allgemeinen Formel (I) wie in Anspruch 1, 2 oder 3 definiert, worin $R_1$ eine Gruppe $O-(CH_2)_{0-6}$-CH(Z')-COOH oder $-(CH_2)_{0-7}$-CH(Z')-COOH darstellt, sowie die Additionssalze mit Säuren und Basen und die Ester.

**5.** Verbindungen der allgemeinen Formel (I) wie in irgendeinem der Ansprüche 1 bis 4 definiert, worin (Z') ein Wasserstoffatom ist, sowie die Additionssalze mit Säuren und Basen und die Ester.

**6.** Verbindungen der allgemeinen Formel (I) wie in irgendeinem der Ansprüche 1 bis 4 definiert, worin (Z') die Gruppe $(CH_2)_{0-6}$-NH-$CO_2$-Rc oder $(CH_2)_{0-6}$-NH-Rb ist und Rb und Rc wie in Anspruch 1 definiert sind, sowie die Additionssalze mit Säuren und Basen und die Ester.

**7.** Verbindungen der allgemeinen Formel (I) wie in Anspruch 6 definiert, worin Rb und Rc Gruppen $(CH_2)_{0-3}$-Ar oder $(CH_2)_{0-3}$-Alk darstellen, wobei Ar und Alk wie in Anspruch 1 definiert sind und substituiert oder nicht substituiert sein können, sowie die Additionssalze mit Säuren und Basen und die Ester.

**8.** Verbindungen der allgemeinen Formel (I) wie in einem der Ansprüche 1 bis 7 definiert, worin G eine Gruppe G4 der allgemeinen Formel -NH-C-(=NH)-NHRc darstellt und Rc wie in Anspruch 1 definiert ist, sowie die Additionssalze mit Säuren und Basen und die Ester.

**9.** Verbindungen der allgemeinen Formel (I) wie in Anspruch 8 definiert, worin Rc ein Wasserstoffatom ist, sowie die Additionssalze mit Säuren und Basen und die Ester.

**10.** Verbindungen der allgemeinen Formel (I) wie in einem der Ansprüche 1 bis 7 definiert, worin G eine Gruppe NH-(Het') bedeutet und (Het') wie in Anspruch 1 definiert ist.

**11.** Verbindungen der allgemeinen Formel (I) wie in Anspruch 10 definiert, worin G die folgenden Heterocyclen darstellt:

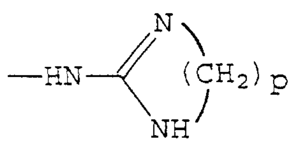
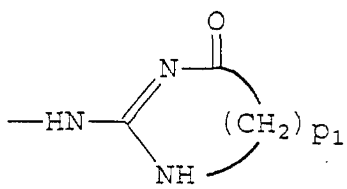
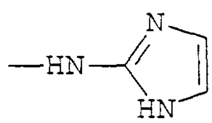

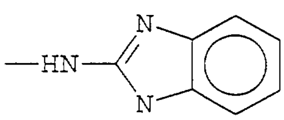
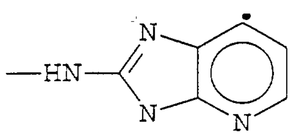
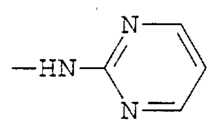
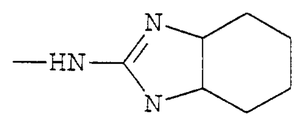

worin p eine ganze Zahl von 2, 3 oder 4 ist und diese Heterocyclen substituiert oder nicht substituiert sind, sowie die Additionssalze mit Säuren und Basen und die Ester.

**12.** Verbindungen der allgemeinen Formel (I) wie in Anspruch 10 oder 11 definiert, worin G die Gruppe

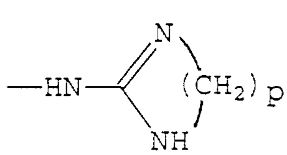

bedeutet und p eine ganze Zahl von 2, 3 oder 4 ist , sowie die Additionssalze mit Säuren und Basen und die Ester.

**13.** Verbindungen der Formel (I) wie in Anspruch 1 definiert, mit den folgenden Namen:

- 4-{[4-((Aminoiminomethyl)-hydrazono)-9,10-dimethoxy-1,2,3,4,5, 6-hexahydro-8-benz(e)-azulenyl]-oxy}-butansäure,

- 5-{[4-((Aminoiminomethyl)-hydrazono)-9,10-dimethoxy-1,2,3,4,5, 6-hexahydro-8-benz(e)-azulenyl]-oxy}-pentansäure,
- 5-{[4-((Aminoiminomethyl)-hydrazono)-8,10-dimethoxy-1,2,3,4,5, 6-hexahydro-9-benz(e)-azulenyl]-oxy}-pentansäure,
- 6-{[4-((Aminoiminomethyl)-hydrazono)-9,10-dimethoxy-1,2,3,4,5, 6-hexahydro-8-benz(e)-azulenyl]-oxy}-hexansäure,
- 7-{[4-((Aminoiminomethyl)-hydrazono)-9,10-dimethoxy-1,2,3,4,5, 6-hexahydro-8-benz(e)-azulenyl]-oxy}-heptansäure,
- 5-{[9,10-Dimethoxy-1,2,3,4,5,6-hexahydro-4-((4,5-dihydro-1H-imidazol-2-yl)-hydrazono)-8-benz(e)-azulenyl]-oxy}-pentansäure,
- 5-{[4-((Aminoiminomethyl)-hydrazono)-9,10-dimethoxy-1,2,3,4,5, 6-hexahydro-8-benz(e)-azulenyl]-oxy}-pentansäure-ethylester-Hydrochlorid,
- 4-{[4-((Aminoiminomethyl)-hydrazono)-8,9-dimethoxy-1,2,3,4,5, 6-hexahydro-10-benz(e)-azulenyl]-oxy}-butansäure,
- 5-{[4-((Aminoiminomethyl)-hydrazono)-8,9-dimethoxy-1,2,3,4,5, 6-hexahydro-10-benz(e)-azulenyl]-oxy}-pentansäure,
- 5-{[4-(((Amino)-carbonyl)-hydrazono)-9,10-dimethoxy-1,2,3,4,5, 6-hexahydro-8-benz(e)-azulenyl]-oxy)-pentansäure,
- 5-{[4-(((Amino)-thiocarbonyl)-hydrazono)-9,10-dimethoxy-1,2,3, 4,5,6-hexahydro-8-benz(e)-azulenyl]-oxy}-pentansäure,
- 4-{[4-((Aminoiminomethyl)-hydrazono)-8,10-dimethoxy-1,2,3,4,5, 6-hexahydro-9-benz(e)-azulenyl]-oxy}-butansäure,
- 6-{[4-((4,5-Dihydro-1H-imidazol-2-yl)-hydrazono)-9,10-dimethoxy-1,2,3,4,5,6-hexahydro-8-benz(e)-azulenyl]-oxy}-hexansäure,
- 5-{[4-((Aminoiminomethyl)-hydrazono)-9,10-dimethoxy-1,2,3,4,5, 6-hexahydro-8-benz(e)-azulenyl]-oxy}-3,3-dimethyl-4-oxo-pentansäure,
- 5-{[4-((4,5-Dihydro-1H-imidazol-2-yl)-hydrazono)-9,10-dimethoxy-1,2,3,4,5,6-hexahydro-8-benz(e)-azulenyl]-oxy}-3,3-dimethyl-4-oxo-pentansäure,
- 5-{[4-((Aminoiminomethyl)-hydrazono)-9,10-dimethoxy-1,2,3,4,5, 6-hexahydro-8-benz(e)-azulenyl]-oxy}-3,3-dimethyl-4-oxo-pentansäure-Hydrochlorid,
- 4-{[4-((4,5-Dihydro-1H-imidazol-2-yl)-hydrazono)-9,10-dimethoxy-1,2,3,4,5,6-hexahydro-8-benz(e)-azulenyl]-oxy}-butansäure,
- 5-{[8-((Aminoiminomethyl)-hydrazono)-9,10-dimethoxy-6,7,8,9, 10,11-hexahydro-azuleno(5,6-d)-1,3-benzodioxol-4-yl]-oxy}-pentansäure,
- 5-{[8-((Aminoiminomethyl)-hydrazono)-2,2-diphenyl-6,7,8,9,10, 11-hexahydro-azuleno(4,5-e)-(1,3)-benzodioxol-4-yl]-oxy}-pentansäure,
- 4-{[9,10-Dimethoxy-4-((1,4,5,6-tetrahydro-2-pyrimidinyl)-hydrazono)-1,2,3,4,5,6-hexahydro-8-benz(e)-azulenyl]-oxy}-butansäure,
- 2-{[4-((4,5-Dihydro-1H-imidazol-2-yl)-hydrazono)-9,10-dimethoxy-1,2,3,4,5,6-hexahydro-8-benz(e)-azulenyl]-oxy}-ethansäure,
- 3-{[4-((4,5-Dihydro-1H-imidazol-2-yl)-hydrazono)-9,10-dimethoxy-1,2,3,4,5,6-hexahydro-8-benz(e)-azulenyl]-oxy}-propansäure,
- 4-{[4-((4,5-Dihydro-1H-imidazol-2-yl)-hydrazono)-1,2,3,4,5,6-hexahydro-8-benz(e)-azulenyl]-oxy}-butansäure,
- 4-{[4-((4,5-Dihydro-1H-imidazol-2-yl)-hydrazono)-1,2,3,4,5,6-hexahydro-8-benz(e)-azulenyl]-oxy}-butansäure,
- O-{4-[(4,5-Dihydro-1H-imidazol-2-yl)-hydrazono]-9,10-dimethoxy-1,2,3,4,5,6-hexahydro-8-benz(e)-azulenyl}-N-[(phenylmethoxy)carbonyl]-DL-homoserin,
- O-{4-[(4,5-Dihydro-1H-imidazol-2-yl)-hydrazono]-1,2,3,4,5,6-hexahydro-8-benz(e)-azulenyl}-N-[(phenylmethoxy)-carbonyl]-DL-homoserin,
- O-{4-[(1,2,3,4-Tetrahydro-6-pyrimidinyl)-hydrazono]-9,10-dimethoxy-1,2,3,4,5,6-hexahydro-8-benz(e)-azulenyl}-N-[(phenylmethoxy)-carbonyl]-DL-homoserin,
- (2,3-Dihydroxypropyl)-ester von O-{9,10-Dimethoxy-1,2,3,4,5,6-hexahydro-4-[(1,4,5,6-tetrahydro-2-pyrimidinyl)-hydrazono]-8-benz(e)-azulenyl}-N-[(phenylmethoxy)-carbonyl]-DL-homoserin,
- O-{4-[(4,5-Dihydro-1H-imidazol-2-yl)-hydrazono]-9,10-dimethoxy-1,2,3,4,5,6-hexahydro-8-benz(e)-azulenyl}-N-[(8-chinolinyl)-sulfonyl]-DL-homoserin,
- O-{4-[(4,5-Dihydro-1H-imidazol-2-yl)-hydrazono]-9,10-dimethoxy-1,2,3,4,5,6-hexahydro-8-benz(e)-azulenyl}-N-{[3-(4-(3-pyridinyl)1H-imidazol-1-yl)-propoxy]-carbonyl}-DL-homoserin-Monohydrochlorid,

- 5-{[4-((4,5-Dihydro-4-oxo-1H-imidazol-2-yl)-hydrazono)-9,10-dimethoxy-1,2,3,4,5,6-hexahydro-8-benz(e)-azulenyl]-oxy}-pentansäure,
- O-{9,10-Dimethoxy-1,2,3,4,5,6-hexahydro-4-[(4,5,6,7-tetrahydro-1H-1,3-diazepin-2-yl)-hydrazono]-8-benz (e)-azulenyl}-N-[(phenylmethoxy)-carbonyl]-DL-homoserin,
- O-{9,10-Dimethoxy-1,2,3,4,5,6-hexahydro-4-[(3a,4,5,6,7,7a-hexahydro-1H-benzimidazol-2-yl)-hydrazono]-8-benz(e)-azulenyl}-N-[(phenylmethoxy)-carbonyl]-DL-homoserin.

**14.** Verfahren zur Herstellung der Verbindungen der Formel (I) wie in Anspruch 1 definiert, **dadurch gekennzeichnet, daß** man eine Verbindung der Formel (II)

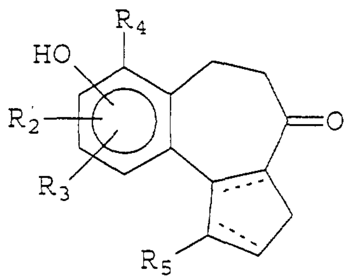

(II)

in der $R_2$, $R_3$, $R_4$ und $R_5$ wie in Anspruch 1 definiert sind, mit Ausnahme von Hydroxyl, entweder der Einwirkung einer Verbindung der Formel (F1) in Anwesenheit einer Base,

Hal- [A] - [B] -$COR_6$ (F1)

oder einer Verbindung der Formel (F'1) in Anwesenheit eines Phosphins und Azodicarbonsäure-diethylester

OH-[A]-[B]-$COR_6$ (F'1)

unterzieht, worin Hal ein Halogenatom ist, [A], [B] und $R_6$ wie vorstehend beschrieben sind, wobei [B] ebenfalls die Gruppe

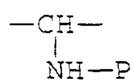

darstellen kann und P eine Schutzgruppe für die Aminfunktion ist, um eine Verbindung der Formel (IIIa)

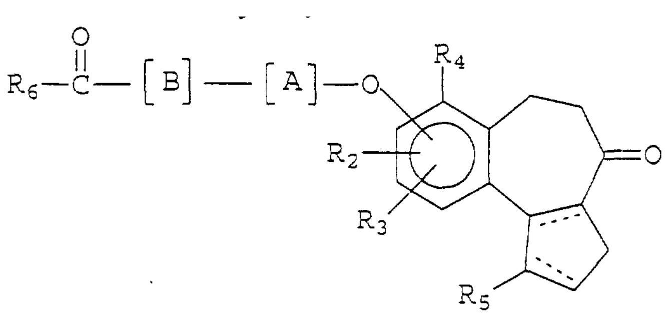

(IIIa)

zu erhalten, oder der Einwirkung einer aktivierenden Gruppe und anschließend einer Verbindung der Formel (F2) in Anwesen-

heit eines Katalysators

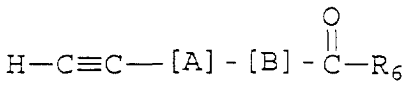

(F2)

unterzieht, um eine Verbindung der Formel (IIIb)

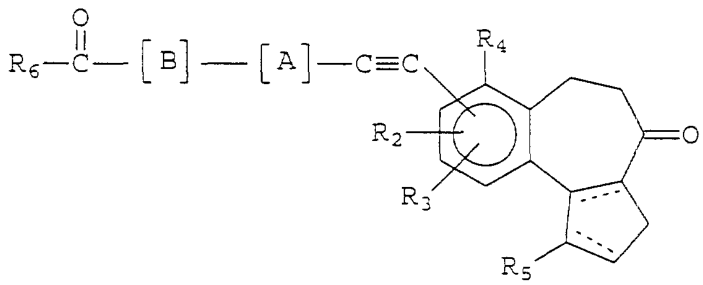

(IIIb)

zu erhalten, wobei man dann die Verbindungen der Formeln (IIIa) oder (IIIb) der Einwirkung einer Verbindung der Formel (F3)

$$H_2N\text{-}G \qquad (F3)$$

unterzieht, in der G wie in Anspruch 1 definiert ist, um die Verbindungen der Formeln (IVa) und (IVb) zu erhalten, die einigen Produkten der Formel (I) entsprechen

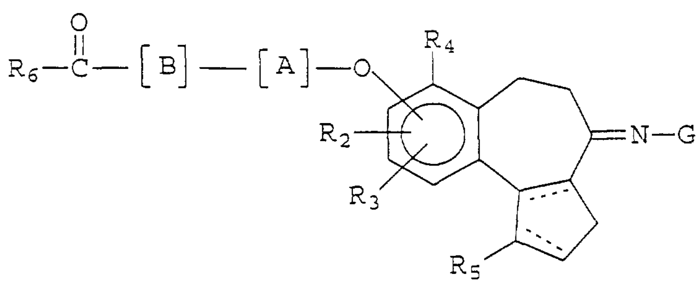

(IVa)

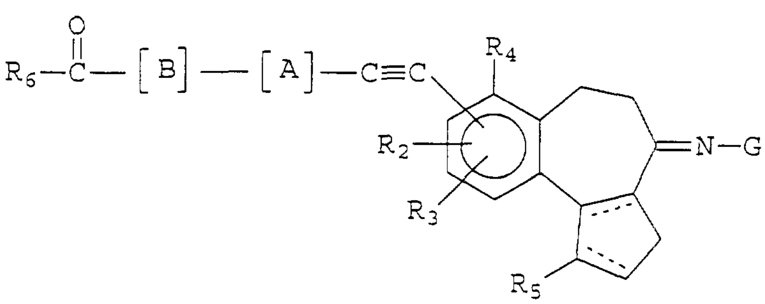

(IVb)

die man anschließend gegebenenfalls in einer geeigneten Reihenfolge einer oder mehreren der folgenden Reaktionen unterzieht:

- Einwirkung einer Base oder einer Säure, um den Ester zu spalten und die entsprechende Säure zu erhalten,
- Einwirkung eines geeigneten Reduktionsmittels zur partiellen oder vollständigen Reduktion der Unsättigungen,
- Einwirkung eines Hydratationsmittels der Dreifachbindung,
- Einwirkung eines Reagens zur Dealkylierung,
- Einwirkung eines Mittels zum Abspalten der Schutzgruppe von der Funktion NH-P in beta von CO-$R_6$, wenn [B] die Gruppe CH-NHP ist,
- Bildung der Gruppe NH-$SO_2$Rc, NH-$CO_2$Rc, NHCORc, NH-$SO_2$-NH-Rc, NH-CO-NHRc ausgehend vom entsprechenden Amin in beta von $COR_6$, um die entsprechenden Verbindungen der Formel (I) zu erhalten, die man dann gegebenenfalls der Einwirkung einer Säure oder einer Base unterzieht, um die entsprechenden Salze zu erhalten, oder der Einwirkung eines Veresterungsmittels unterwirft, um die entsprechenden Ester zu erhalten.

**15.** Verfahren zur Bildung der Verbindungen der Formel (I) wie in Anspruch 1 definiert, **dadurch gekennzeichnet, daß** man zuvor eine Verbindung der Formel (II) der Einwirkung einer Verbindung der Formel (F3) unterzieht, um eine Verbindung der Formel (IIIc)

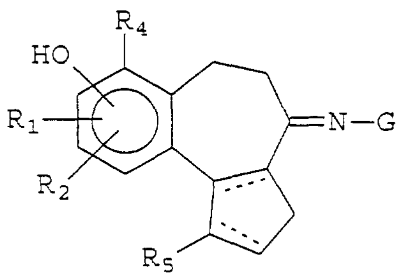

(IIIc)

zu erhalten, die man danach, gegebenenfalls nach dem Schutz von G, der Einwirkung einer Verbindung der Formeln (F1), (F'1) oder (F2) und anschließend gegebenenfalls einer Reaktion zum Abspalten der Schutzgruppe von G unterzieht, um die entsprechenden Verbindungen der Formeln (IVa) und (IVb) zu erhalten, die man dann gegebenenfalls den verschiedenen in Anspruch 14 definierten Reaktionen unterwirft, wobei die Verbindungen der Formeln (II), (F1), (F'1), (F2), (F3), (IVa) und (IVb) wie in Anspruch 14 definiert sind.

**16.** Verfahren zur Herstellung der Produkte der Formel (II) wie in Anspruch 14 definiert, worin $R_2$, $R_3$, $R_4$ und $R_5$ Wasserstoffatome sind und OH sich in Position 8, 9 oder 10 befindet, **dadurch gekennzeichnet, daß** man

(i) eine Verbindung der Formel (a)

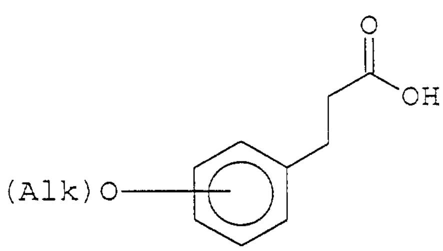

(a)

in der sich O-(Alk) in Position meta oder para von der Gruppe Alkylcarboxyl befindet und (Alk) wie in Anspruch 1 definiert ist, der Einwirkung eines Halogenierungsmittels unterzieht, um das entsprechende Acylhalogenid zu erhalten,

(ii) das man der Einwirkung eines Reagens der Formel (b)

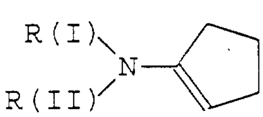

(b)

unterzieht, worin R(I) und R(II), gleich oder verschieden, eine Gruppe Alkyl mit 1 bis 6 Kohlenstoffatomen darstellen, oder R(I) und R(II) zusammen mit dem Stickstoffatom, an das sie gebunden sind, einen gesättigten oder ungesättigten Heterocyclus mit 5 oder 6 Ringgliedern bedeuten, der gegebenenfalls ein anderes Heteroatom umfaßt, ausgewählt unter O und N,
um eine Verbindung der Formel (c)

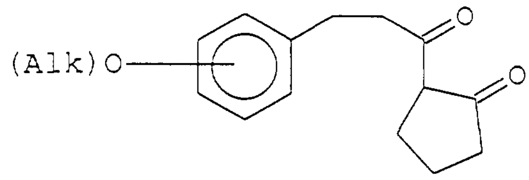

(c)

zu erhalten, die man
(iii) der Einwirkung eines Halogenierungsmittels unterwirft, um eine Verbindung der Formel (d)

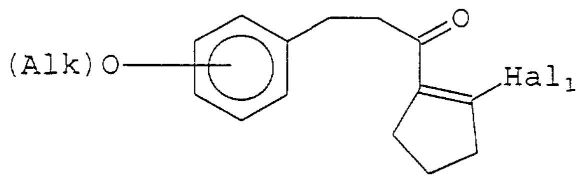

(d)

zu erhalten, in der $Hal_1$ ein Halogenatom ist, die man dann
(iv) der Einwirkung einer Lewis-Säure unterzieht, um eine Verbindung der Formel (e)

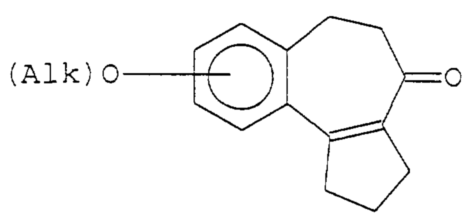

(e)

zu erhalten, die man
(v) einem Reagens zur Dealkylierung unterwirft, um das Produkt der Formel (IIF) zu erhalten, das dem erwarteten monosubstituierten Produkt der Formel (II) entspricht:

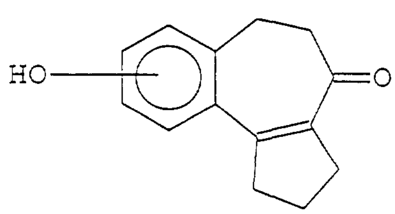

(IIF)

**17.** Verfahren zur Herstellung der Verbindungen der allgemeinen Formel (II) wie in Anspruch 14 definiert, worin $R_2$ entweder eine Gruppe OAlk oder eine Gruppe O-$(CH_2)_{0-3}$-Ar darstellt, $R_3$, $R_4$ und $R_5$ Wasserstoffatome sind und OH und $R_2$ sich in Position 8, 9 oder 10 befinden, **dadurch gekennzeichnet, daß** man die Verbindung der Formel (a')

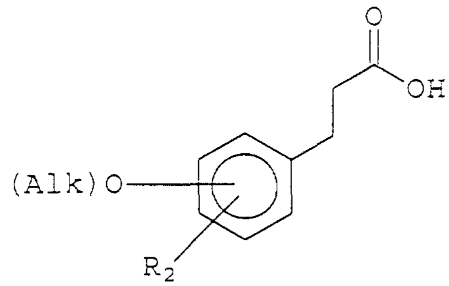

(a')

in der $R_2$ und O-(Alk) sich in Position meta oder para von der Gruppe Alkylcarboxyl befinden, nacheinander den Reaktionen (i), (ii), (iii), (iv) und (v) unterzieht, um das Produkt der Formel (IIG) zu erhalten, das dem erwarteten bisubstituierten Produkt der Formel (II) entspricht:

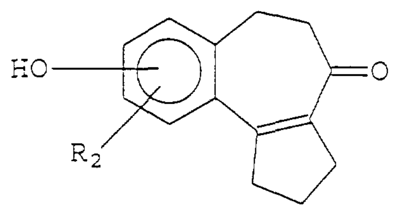

(IIG)

**18.** Verfahren zur Herstellung der Verbindungen der allgemeinen Formel (II) wie in Anspruch 14 definiert, worin $R_2$ und $R_3$ eine Gruppe O-(Alk) oder O-$(CH_2)_{0-3}$-Ar darstellen, $R_4$ und $R_5$ Wasserstoffatome sind und OH sich in Position 9 befindet, **dadurch gekennzeichnet, daß** man die Verbindung der Formel (IIA)

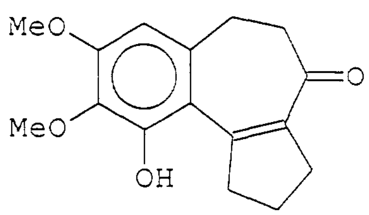

(IIA)

der Einwirkung eines Reagens zur Dealkylierung unterwirft, um die Verbindung der Formel (IIB)

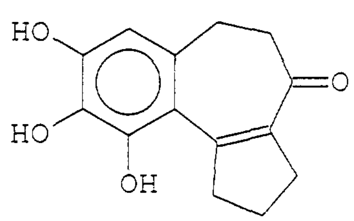

(IIB)

zu erhalten, die man dann

<u>entweder</u> der Einwirkung eines Reagens zum Schutz der Diole im basischen Medium unterzieht, um selektiv das Produkt der Formel (IIC)

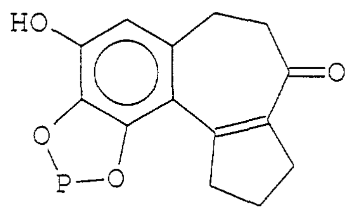

(IIC)

zu erhalten, in der P der Rest eines Schutzreagens für die Diole ist, das man anschließend nacheinander der Einwirkung eines Reagens zum Schutz des Phenols, eines Reagens zum Absealten der Schutzgruppen von den Diolen, eines Alkylierungsmittels und danach eines Reagens zum Abspalten der Schutzgruppe vom Phenol unterwirft, um die Verbindung der Formel (IID) zu erhalten, die dem trisubstituierten Produkt der Formel (II) entspricht, mit OH in Position 8:

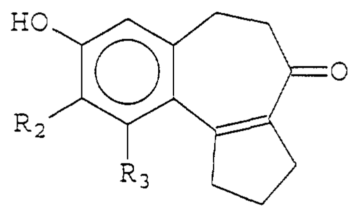

(IID)

oder nacheinander der Einwirkung eines Mittels zum Schutz des Phenols, eines Alkylierungsmittels und danach eines Mittels zum Abspalten der Schutzgruppe unterwirft, um die Verbindung der Formel (IIE) zu erhalten, die dem trisubstituierten Produkt der Formel (II) entspricht, mit OH in Position 9:

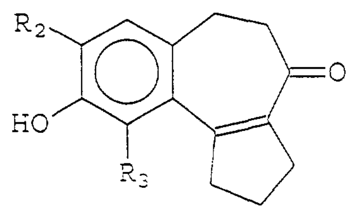

(IIE)

**19.** Als Arzneimittel die Verbindungen der Formel (I) wie in irgendeinem der Ansprüche 1 bis 12 definiert, sowie ihre pharmazeutisch akzeptablen Additionssalze und Ester.

**20.** Als Arzneimittel die Verbindungen der Formel (I) wie in Anspruch 13 definiert.

**21.** Pharmazeutische Zusammensetzungen, umfassend als Wirkstoff mindestens eines der in Anspruch 19 oder 20 definierten Arzneimittel.

**22.** Als neue Zwischenprodukte die Verbindungen der allgemeinen Formeln (IIIa), (IIIb), (IIIc) und (II) wie in einem der Ansprüche 14 bis 18 definiert, mit der Maßgabe, daß die Verbindungen der Formel (IIc) und die Verbindungen

- 2,3,5,6-Tetrahydro-9,10-dimethoxy-8-hydroxy-benz[e]-azulen-4(1H)-on und
- 2,3,5,6-Tetrahydro-8,9-dimethoxy-10-hydroxy-benz[e]-azulen-4(1H)-on

ausgeschlossen sind.

**Claims**

**1.** Compounds of general formula (I):

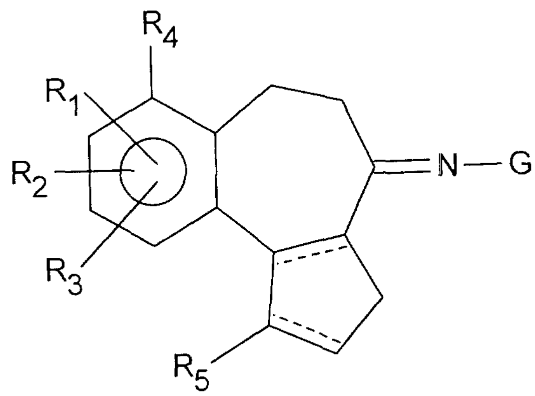

(I)

in which $R_1$ represents a $-C{\equiv}C-[A]-[B]-COR_6$, $-CH=CH-[A]-[B]-COR_6$, $-(CH_2)_2-[A]-[B]-COR_6$, $-O-[A]-[B]-COR_6$, $-CH_2CO-[A]-[B]-COR_6$ group, -[A]- representing

- either a divalent hydrocarbon radical derived from a linear or branched, saturated or unsaturated structure, comprising 1 to 12 carbon atoms and 1 to 6 heteroatoms chosen from oxygen, nitrogen or sulphur atoms,
- or a divalent radical derived from a linear or branched, saturated or unsaturated, acyclic hydrocarbon, comprising 1 to 12 carbon atoms,
  [B] representing a phenyl radical, a CH(Z) radical, or a single bond,
  Z represents a hydrogen atom, a
  $(D)_{0-6}$-NRaRb, $(D)_{0-6}$-NH-$SO_2$-Rc, $(D)_{0-6}$-NH-$CO_2$-Rc,
  $(D)_{0-6}$-NH-CO-Rc, $(D)_{0-6}$-NH-$SO_2$-NH-Rc, $(D)_{0-6}$-NH-CO-NH-Rc, $(D)_{0-6}$-$CO_2$-Rc, $(D)_{0-6}$-$SO_2$-Rc, $(D)_{0-6}$-CO-Rc or $(D)_{0-6}$-Rc group in which
  $(D)_{0-6}$ is a divalent radical derived from a linear or branched, saturated or unsaturated, acyclic hydrocarbon, comprising 0 to 6 carbon atoms,
  Ra, Rb and Rc represent a hydrogen atom, a $(CH_2)_{0-3}$-Ar radical in which Ar represents a carbocyclic aryl group containing 6 to 18 carbon atoms, a $(CH_2)_{0-3}$-Het radical in which Het represents a radical derived from an aromatic or nonaromatic, saturated or unsaturated heterocycle, comprising 1 to 9 carbon atoms and 1 to 5 heteroatoms chosen from oxygen, nitrogen or sulphur atoms, a $(CH_2)_{0-3}$-Alk radical in which Alk represents a radical derived from a non-aromatic, linear, branched or cyclic, saturated or unsaturated hydrocarbon, comprising 1 to 12 carbon atoms, the Het, Ar and Alk radicals can be non-substituted or substituted,
  or also, Ra and Rb together with the nitrogen atom to which they are linked represent an aromatic or non-aromatic, saturated or unsaturated nitrogenous heterocycle, optionally containing one or more heteroatoms chosen from oxygen, nitrogen or sulphur atoms, this radical can be substituted or non-substituted,
- $R_6$ represents a hydroxyl radical, an O-Alk, O-Ar, $NH_2$, NH-Alk, $N(Alk)_2$ radical or the remainder of an L or D amino acid, Alk and Ar being as defined previously and can be substituted or non-substituted,
- $R_2$ and $R_3$, identical or different, represent either a hydrogen atom, a hydroxyl radical, an O-Alk radical or an O-$(CH_2)_{0-3}$-Ar radical, Alk and Ar being as defined previously, or $R_2$ and $R_3$ together form an -O-$(CRdRe)_n$-O- type ring, n being an integer from 1 to 5, Rd and Re independently from one other represent a hydrogen atom, an alkyl radical containing 1 to 6 carbon atoms, or a phenyl radical,
- $R_4$ represents a hydrogen atom, a halogen atom, a hydroxyl amino, nitro, cyano, CF3, acyl or acyloxy containing 1 to 12 carbon atoms alkyl, alkynyl, alkynyl, alkylthio, alkoxy, alkylamino, dialkylamino, dialkylaminoalkyl, dialkylaminoalkyloxy group, in which the alkyl term contains 1 to 6 carbon atoms,
- $R_5$ represents a hydrogen atom, a hydroxyl radical, a halogen atom, an O-Alk radical or an O-$(CH_2)_{0-3}$-Ar radical, Alk and Ar being as defined previously,
- G represents,
  **either** a radical of formula G1

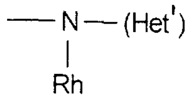

in which Rh is a hydrogen atom or an Alk group as defined previously and (Het') is a heterocycle of general formula:

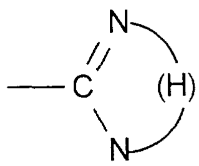

in which (H) forms, with the N=C-NH- unit, the remainder of an aromatic or non-aromatic, mono- or bicyclic, saturated or unsaturated heterocycle, comprising 1 to 9 carbon atoms and 2 to 5 heteroatoms chosen from oxygen, nitrogen and sulphur atoms, this radical can be substituted or non-substituted,

- **or** an NRaRb radical (G2 radical), Ra and Rb being as defined previously,
- **or** a (Het) radical (G3 radical) as defined previously,
- **or** an -NRh-C(=X)-NHRc radical (G4 radical), in which X is a sulphur, oxygen or NH atom, Rh and Rc are as defined previously,
- **or** an -NRh-$SO_2$Rc radical, (G5 radical), in which Rh and Rc are as defined previously,

the dotted lines represent an optional second bond, as well as the addition salts with acids and bases and esters,

$R_1$, $R_2$ and $R_3$ can be in position 8, 9 or 10 of the tricycle, the optional substituants of the (Alk), (Ar), (Het), (Het') or NRaRb radicals forming a heterocycle which can be a

- halogen
- alkyl, alkenyl, alkynyl containing 1 to 12 carbon atoms,
- oxo, cyano, nitro, formyl, carboxy and carboxyalkyl containing 1 to 6 carbon atoms, carboxamide,
- alkoxy containing 1 to 12 carbon atoms,
- alkylthio containing 1 to 12 carbon atoms,
- amino, alkylamino containing 1 to 12 carbon atoms, dialkylamino containing 2 to 24 carbon atoms,
- aminoalkyl containing 1 to 12 carbon atoms,
- dialkylaminoalkyl containing 3 to 25 carbon atoms,
- dialkylaminoalkyloxy containing 3 to 25 carbon atoms,
- optionally acylated hydroxyl containing 1 to 12 carbon atoms,
- acyl containing 1 to 12 carbon atoms or benzoyl optionally substituted by a chlorine, iodine or fluorine atom,
- carbocyclic or heterocyclic aryl or aralkyl optionally substituted by halogen, alkyl, alkoxy, alkylthio, aminoalkyl or dialkylamino indicated above.

**2.** Compounds of general formula (I) as defined in claim 1, corresponding to general formula (I'):

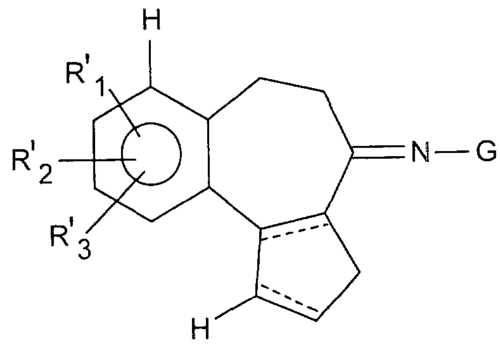

(I')

in which $R'_1$ represents a $-C{\equiv}C-[A']-[B']-COR'_6$, $-CH{=}CH-[A']-[B']-COR'_6$, $-(CH_2)_2-[A']-[B']-COR'_6$, $-O-[A']-[B']-COR'_6$, $-CH_2CO-[A']-[B']-COR'_6$ group, -[A']- representing a divalent alkylene, alkenylene or alkynylene radical containing 1 to 6 carbon atoms, [B'] representing a CH(Z') radical or a single bond, Z' represents a hydrogen atom, a $(CH_2)_{0-6}$-NRaRb, $(CH_2)_{0-6}$-NH-$SO_2$-Rc, $(CH_2)_{0-6}$-NH-$CO_2$-Rc, $(CH_2)_{0-6}$-NH-CO-Rc, $(CH_2)_{0-6}$-NH-$SO_2$-NH-Rc, $(CH_2)_{0-6}$-NH-CO-NH-Rc, $(CH_2)_{0-6}$-$CO_2$-Rc, $(CH_2)_{0-6}$-$SO_2$-Rc, $(CH_2)_{0-6}$-CO-Rc or $(CH_2)_{0-6}$-Rc group, Ra, Rb and Rc being as defined in claim 1, $R'_6$ represents an OH, amino or alkoxy radical containing 1 to 8 carbon atoms, optionally substituted by one or more radicals chosen from the hydroxy, amino, phenylalkylamino or dialkylamino radicals, $R'_2$ and $R'_3$ representing a hydrogen atom or a methoxy radical, and G is as defined in claim 1, the dotted lines represent an optional second bond, as well as the addition salts with acids and bases and esters.

3. Compounds of general formula (I) as defined in claim 1 or 2 in which $R_6$ represents an -OH, $-OCH_3$, $-OCH_2CH_3$, $-O-(CH_2)_2-OH$,

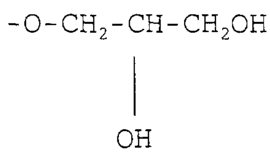

group, $-O-(CH_2)_2-NH_2$, $-O-(CH_2)_2-N-(CH_3)_2$, $-NH_2$ or $-O-(CH_2)$-phenyl group, as well as the addition salts with acids and bases and esters.

4. Compounds of general formula (I) as defined in claim 1, 2 or 3, in which $R_1$ represents an O-$(CH_2)_{0-6}$-CH(Z')-COOH or -$(CH_2)_{0-7}$-CH(Z')-COOH group, as well as the addition salts with acids and bases and esters.

5. Compounds of general formula (I) as defined in any one of claims 1 to 4, in which (Z') is a hydrogen atom, as well as the addition salts with acids and bases and esters.

6. Compounds of general formula (I) as defined in any one of claims 1 to 4, in which (Z') is the $(CH_2)_{0-6}$-NH-$CO_2$-Rc or $(CH_2)_{0-6}$-NH-Rb group, Rb and Rc being as defined in claim 1, as well as the addition salts with acids and bases and esters.

7. Compounds of general formula (I) as defined in claim 6, in which Rb and Rc are the $(CH_2)_{0-3}$-Ar or $(CH_2)_{0-3}$-Alk groups, Ar and Alk being as defined in claim 1 and can be substituted or non-substituted, as well as the addition salts with acids and bases and esters.

8. Compounds of general formula (I) as defined in one of claims 1 to 7, in which G is a G4 group of general formula -NH-C(=NH)-NHRc, Rc being as defined in claim 1, as well as the addition salts with acids and bases and esters.

**9.** Compounds of general formula (I) as defined in claim 8, in which Rc is a hydrogen atom, as well as the addition salts with acids and bases and esters.

**10.** Compounds of general formula (I) as defined in one of claims 1 to 7, in which G is an NH-(Het') group, (Het') being as defined in claim 1.

**11.** Compounds of general formula (I) as defined in claim 10, in which G represents the following heterocycles:

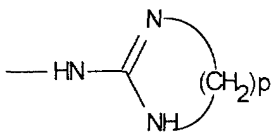

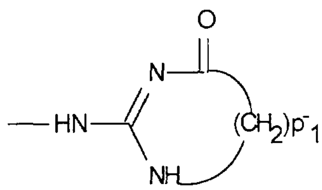

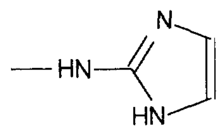

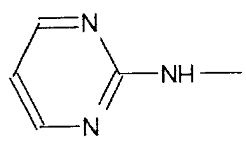

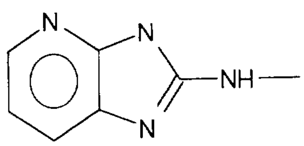

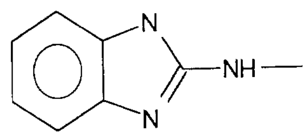

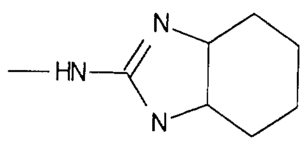

p being an integer equal to 2, 3 or 4, these heterocycles being substituted or non-substituted, as well as the addition salts with acids and bases and esters.

**12.** Compounds of general formula (I) as defined in claim 10 or 11, in which G is the

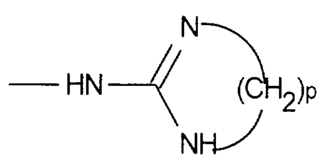

group, p being a integer equal to 2, 3 or 4, as well as the addition salts with acids and bases and esters.

**13.** Compounds of formula (I) as defined in claim 1 the names of which follow:

- 4-((4-((aminoiminomethyl)hydrazono)-9,10-dimethoxy-1, 2,3,4,5,6-hexahydro-8-benz(e)azulen-yl)oxy)-butanoic acid,
- 5-((4-((aminoiminomethyl)hydrazono)-9,10-dimethoxy-1, 2,3,4,5,6-hexahydro-8-benz(e)azulen-yl)oxy)-pentanoic acid,
- 5-((4-((aminoiminomethyl)hydrazono)-8,10-dimethoxy-1, 2,3,4,5,6-hexahydro-9-benz(e)azulen-yl)oxy)-pentanoic acid,
- 6-((4-((aminoiminomethyl)hydrazono)-9,10-dimethoxy-1, 2,3,4,5,6-hexahydro-8-benz(e)azulenyl)oxy)-hexanoic acid,

- 7-((4-((aminoiminomethyl)hydrazono)-9,10-dimethoxy-1, 2,3,4,5,6-hexahydro-8-benz(e)azulenyl)oxy)-heptanoic acid,
- 5-((9,10-dimethoxy-1,2,3,4,5,6-hexahydro-4-((4,5-dihydro-1H-imidazol-2-yl)hydrazono)-8-benz(e)azulenyl) oxy)-pentanoic acid,
- hydrochloride of ethyl 5-((4-((aminoiminomethyl)hydrazono)9,10-dimethoxy-1, 2,3,4,5,6-hexahydro-8-benz (e)azulenyl)oxy)pentanoate,
- 4-((4-((aminoiminomethyl)hydrazono)-8,9-dimethoxy-1, 2,3,4,5,6-hexahydro-10-benz(e)azulenyl)oxy)-butanoic acid,
- 5-((4-((aminoiminomethyl)hydrazono)-8,9-dimethoxy-1, 2,3,4,5,6-hexahydro-10-benz(e)azulenyl)oxy)-pentanoic acid,
- 5-((4-(((amino)carbonyl)hydrazono)-9,10-dimethoxy-1, 2,3,4,5,6-hexahydro-8-benz(e)azulenyl)oxy)-pentanoic acid,
- 5-((4-(((amino)thiocarbonyl)hydrazono)-9,10-dimethoxy-1,2,3,4,5,6-hexahydro-8-benz(e)azulenyl)oxy)-pentanoic acid,
- 4-((4-((aminoiminomethyl)hydrazono)-8,10-dimethoxy-1, 2,3,4,5,6-hexahydro-9-benz(e)azulen-yl)oxy)-butanoic acid,
- 6-((4-((4,5-dihydro-1H-imidazol-2-yl)hydrazono)-9,10-dimethoxy-1,2,3,4,5,6-hexahydro-8-benz(e)azulenyl) oxy)hexanoic acid,
- 5-((4-((aminoiminomethyl)hydrazono)-9,10-dimethoxy-1, 2,3,4,5,6-hexahydro-8-benz(e)azulenyl)oxy)-3,3-dimethyl-4-oxo-pentanoic acid,
- 5-((4-((4,5-dihydro-1H-imidazol-2--yl)hydrazono)-9,10-dimethoxy-1,2,3,4,5,6-hexahydro-8-benz(e)azulenyl) oxy)-3,3-dimethyl-4-oxo-pentanoic acid,
- hydrochloride of 5-((4-((aminoiminomethyl)hydrazono)-9,10-dimethoxy-1,2,3,4,5,6-hexahydro-8-benz(e)azulenyl)oxy)pentanoic acid,
- 4-((4-((4,5-dihydro-1H-imidazol-2-yl)hydrazono)-9,10-dimethoxy-1,2,3,4,5,6-hexahydro-8-benz(e)azulenyl) oxy)butanoic acid,
- 5-((8((aminoiminomethyl)hydrazono)-6,7,8,9,10,11-hexahydroazuleno(5,6-d)-1,3-benzodioxol-4-yl)oxy)-pentanoic acid,
- 5-((8((aminoiminomethyl)hydrazono)-2,2-diphenyl-6,7,8,9,10,11-hexahydro-azuleno(4,5-e)-(1,3)-benzodioxol-4-yl)oxy)-pentanoic acid,
- 4-((9,10-dimethoxy-4-((1,4,5,6-tetrahydro-2-pyrimidinyl)hydrazono)-1,2,3,4,5,6-hexahydro-8-benz(e)azulenyl) oxy)-butanoic acid,
- 2-((4-((4,5-dihydro-1H-imidazol-2-yl)hydrazono)-9,10-dimethoxy-1,2,3,4,5,6-hexahydro-8-benz(e)azulenyl) oxy)ethanoic acid,
- 3-((4-((4,5-dihydro-1H-imidazol-2-yl)hydrazono)-9,10-dimethoxy-1,2,3,4,5,6-hexahydro-8-benz(e)azulenyl) oxy)propanoic acid,
- 4-((4-((4,5-dihydro-1H-imidazol-2--yl)hydrazono)1,2,3,4,5,6-hexahydro-8-benz(e)azulenyl)oxy)-butanoic acid,
- 4-((4-((4,5-dihydro-1H-imidazol-2-yl)hydrazono)1,2,3,4,5,6-hexahydro-8-benz(e)azulenyl)oxy)-butanoic acid,
- O-[4[(4,5-dihydro-1H-imidazol-2-yl)hydrazono]-9,10-dimethoxy-1,2,3,4,5,6-hexahydro-8-benz[e]azulenyl]-N-[(phenylmethoxy)carbonyl]-DL-homoserine,
- O-[4[(4,5-dihydro-1H-imidazol-2-yl)hydrazono]-1,2,3,4,5,6-hexahydro-8-benz[e]azulenyl]-N-[(phenylmethoxy)carbonyl]-DL-homoserine,
- O-[4-[(1,2,3,4-tetrahydro 6-pyrimidinyl) hydrazono] 9,10-dimethoxy 1,2,3,4,5,6-hexahydro 8-benz[e]azulenyl] N-[(phenylmethoxy) carbonyl] DL-homoserine,
- (2,3-dihydroxypropylic) ester of 0-(9,10-dimethoxy 1,2,3,4,5,6,-hexahydro 4-[(1,4,5,6-tetrahydro 2-pyrimidinyl) hydrazono]-8-benz(e)azulenyl] N-[(phenylmethoxy) carbonyl] DL-homoserine,
- O-[4-[(4,5-dihydro 1H-imidazol-2-yl) hydrazono] 9,10-dimethoxy 1,2,3,4,5,6-hexahydro 8-benz(e)azulenyl] N-[(8-quinolinyl) sulphonyl] DL-homoserine,
- O-[4-[(4,5-dihydro 1H-imidazol-2-yl) hydrazono] 9,10-dimethoxy 1,2,3,4,5,6-hexahydro 8-benz(e)azulenyl] N-[[3-[4-(3-pyridinyl) 1H-imidazol-1-yl] propoxy] carbonyl] DL-homoserine monohydrochloride,
- 5-[[4-[(4,5-dihydro 4-oxo 1H-imidazol-2-yl) hydrazono] 9,10-dimethoxy 1,2,3,4,5,6-hexahydro 8-benz(e)azulenyl] oxy] pentanoic acid,
- O-[9,10-dimethoxy 1,2,3,4,5,6-hexahydro 4-[(4,5,6,7-tetrahydro 1H-1,3-diazepin-2-yl) hydrazono] 8-benz(e)-azulenyl] N-[(phenylmethoxy) carbonyl] DL-homoserine,
- O-[9,10-dimethoxy 1,2,3,4,5,6-hexahydro 4-[(3a,4,5,6,7,7a-hexahydro 1H-benzimidazol-2-yl) hydrazono] 8-benz(e)azulenyl] N-[(phenylmethoxy) carbonyl] DL-homoserine.

**14.** Process for the preparation of compounds of formula (I) as defined in claim 1 **characterized in that** the compound of formula (II):

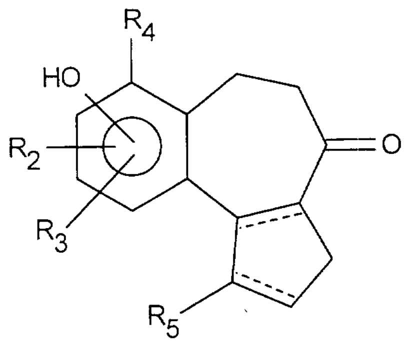

(II)

in which $R_2$, $R_3$, $R_4$ and $R_5$ are as defined in claim 1 with the exception of hydroxyl is subjected either to the action of a compound of formula (F1) in the presence of a base,

Hal-[A]-[B]-$COR_6$ (F1)

or of a compound of formula ($F'_1$) in the presence of a phosphine and diethyl azodicarboxylate:

OH-[A]-[B]-$COR_6$ ($F'_1$)

in which Hal is a halogen atom, [A], [B] and $R_6$ are as described previously, [B] can also represent the

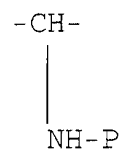

group, P being a protective group of the amine function, in order to obtain a compound of formula (IIIa):

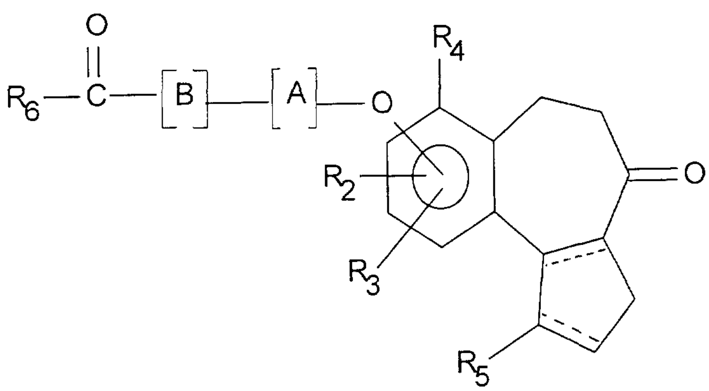

(IIIa)

or to the action of a activating group then a compound of formula (F2) in the presence of a catalyst:

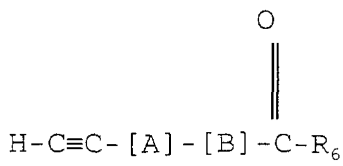

(F2)

in order to obtain a compound of formula (IIIb):

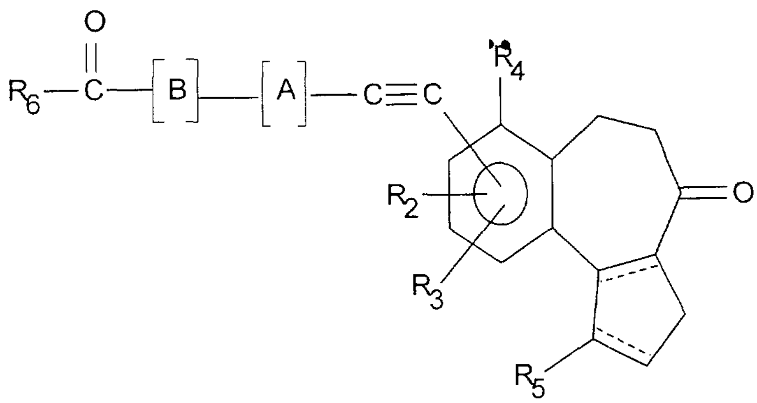

(IIIb)

which compounds of formula (IIIa) or (IIIb) are subjected to the action of a compound of formula (F3):

$$H_2N\text{-}G \qquad (F3)$$

in which G is as defined in claim 1, in order to obtain the compounds of formulae (IVa) and (IVb) corresponding to certain products of formula (I):

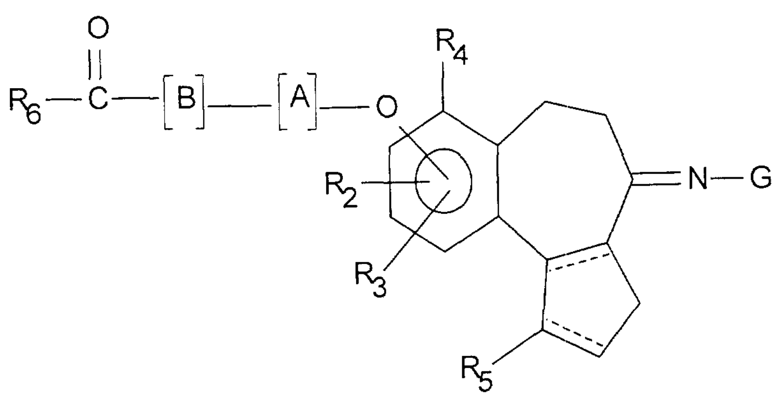

(IVa)

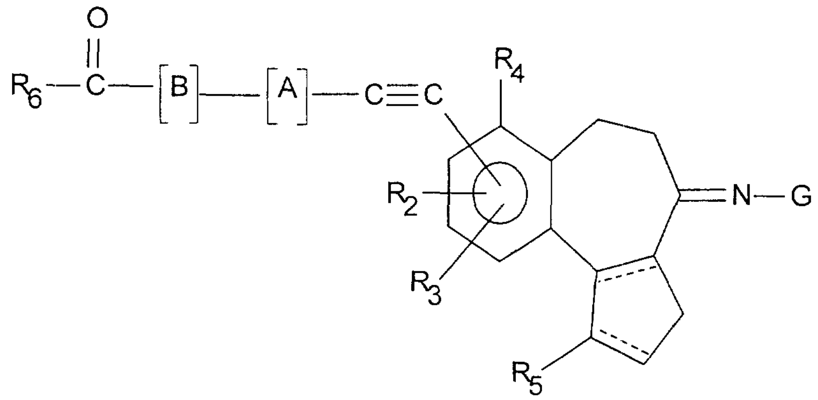

(IVb)

which are subjected, if appropriate, in a suitable order, to one or more of the following reactions:

- action of a base or an acid in order to cleave the ester and obtain the corresponding acid,
- action of a suitable reducing agent to partially or totally reduce the unsaturations,
- to the action of a hydration agent of the triple bond,
- to the action of a dealkylation reagent,
- to the action of a deprotection agent of the NH-P function in beta position of CO-$R_6$ when [B] represents the CH-NHP group,
- to the formation of the NH-$SO_2R_c$, NH-$CO_2R_c$, NHCO$R_c$, NH-$SO_2$-NH-$R_c$, NH-CO-NH$R_c$ group starting from the corresponding amine in beta position of CO$R_6$, in order to obtain the corresponding compounds of formula (I) which are subjected, if appropriate, to the action of an acid or a base in order to obtain the corresponding salts or to the action of an esterification agent in order to obtain the corresponding esters.

**15.** Process for the formation of the compounds of formula (I) as defined in claim 1, **characterized in that** a compound of formula (II) is subjected beforehand to the action of a compound of formula (F3) in order to obtain a compound of formula (IIIc):

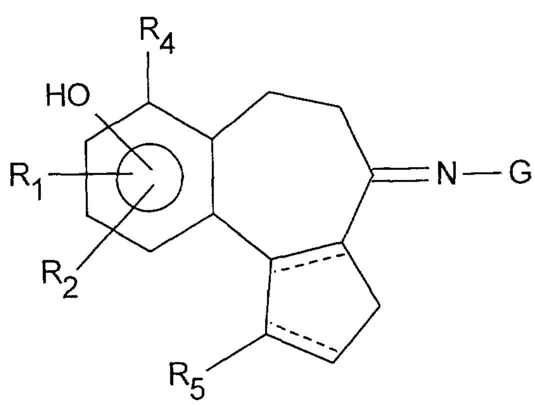

(IIIc)

which compound of formula (IIIc) after, if appropriate protection of G, is subjected to the action of a compound of formula (F1), (F'$_1$) or (F2) then, if appropriate, to a deprotection reaction of G, in order to obtain the corresponding compounds of formula (IVa) and (IVb) which are then subjected, if appropriate, to the different reactions as defined in claim 14, the compounds of formulae (II), (F1), (F'$_1$), (F2), (F3), (IVa) and (IVb) being as defined in claim 14.

**16.** Process for the preparation of the products of formula (II) as defined in claim 14, in which $R_2$, $R_3$, $R_4$ and $R_5$ are hydrogen atoms and OH is in position 8, 9 or 10,
**characterized in that**

(i) a compound of formula (a):

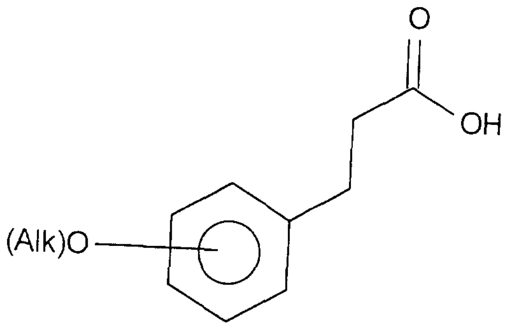

(a)

in which O-(Alk) is in meta or para position of the alkylcarboxylic group, (Alk) being as defined in claim 1, is subjected to the action of a halogenation agent in order to obtain the corresponding acyl halide,
(ii) which is subjected to the action of a reagent of formula (b) :

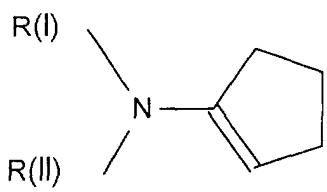

(b)

in which $R_{(I)}$ and $R_{(II)}$, identical or different, represent an alkyl group containing 1 to 6 carbon atoms, or $R_{(I)}$ and $R_{(II)}$ together with the nitrogen atom to which they are linked, represent a heterocycle with 5 or 6 saturated or unsatured members, optionally containing another heteroatom chosen from O and N,
in order to obtain a compound of formula (c):

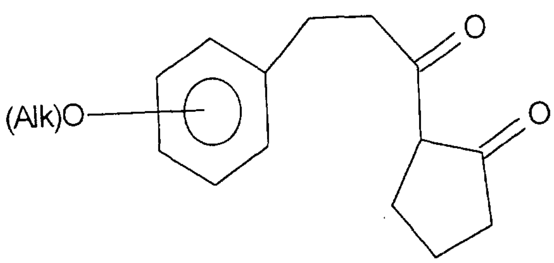

(c)

(iii) which is subjected to the action of a halogenation agent in order to obtain a compound of formula (d):

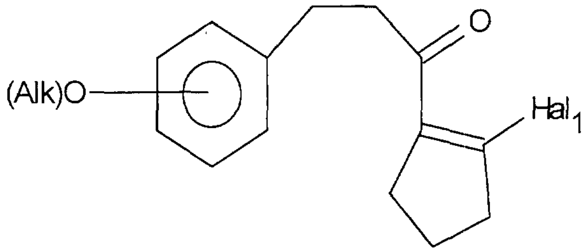

(d)

in which $Hal_1$ represents a halogen atom.

(iv) which is subjected to the action of a Lewis acid, in order to obtain a compound of formula (e):

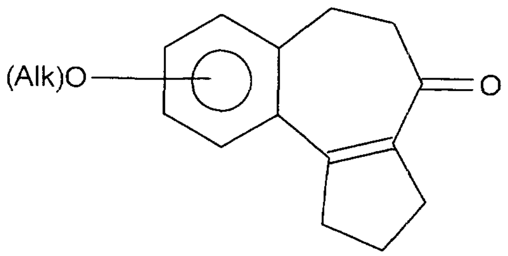

(e)

(v) which is subjected to a dealkylation reagent in order to obtain the product of formula (IIF) corresponding to the expected monosubstituted product of formula (II):

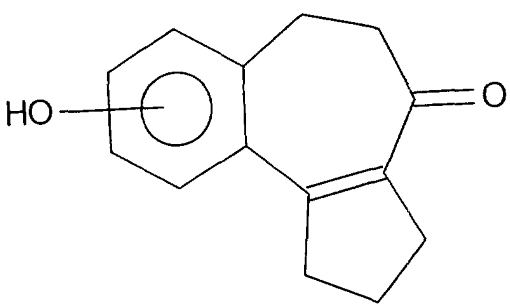

(IIF)

**17.** Process for the preparation of the compounds of general formula (II) as defined in claim 14 and in which $R_2$ is either an OAlk group, or an O-$(CH_2)_{0-3}$-Ar group and $R_3$, $R_4$ and $R_5$ are hydrogen atoms, OH and $R_2$ being in position 8, 9 or 10,
**characterized in that** the compound of general formula (a'):

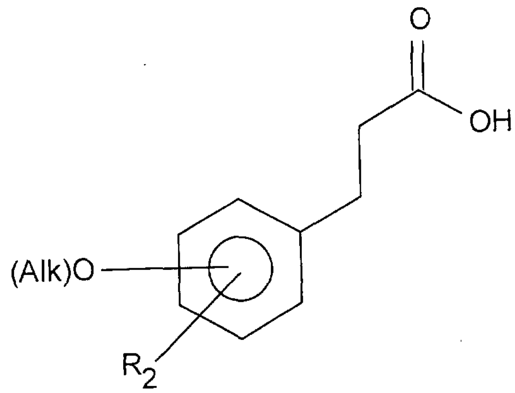

(a')

in which $R_2$ and O-(Alk) are in meta or para position of the alkylcarboxylic group, are subjected successively to reactions (i), (ii), (iii), (iv) and (v) in order to obtain the product of formula (IIG) corresponding to the expected bisubstituted product of formula (II):

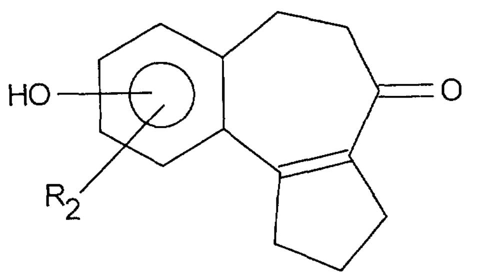

(IIG)

**18.** Process for the preparation of the compounds of general formula (II) as defined in claim 14 and in which $R_2$ and $R_3$ represent an O-(Alk) or O-$(CH_2)_{0-3}$-(Ar) group, $R_4$ and $R_5$ are hydrogen atoms, OH being in position 9 **characterized in that** the compound of formula (IIA):

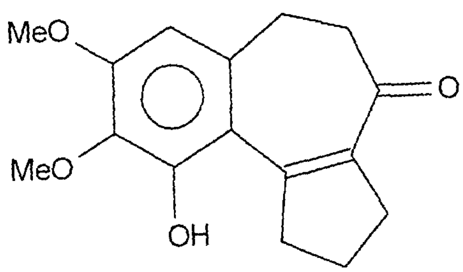

(IIA)

is subjected to the action of a dealkylation reagent, in order to obtain the compound of formula (IIB):

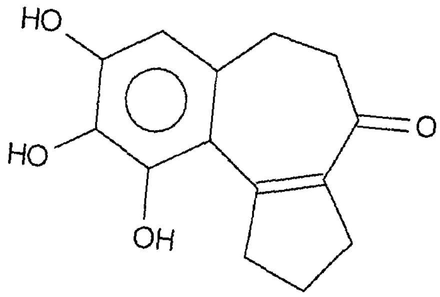

(IIB)

which compound of formula (IIB) is subjected:
**either** to the action of a diol protection reagent in a basic medium, in order to selectively obtain the product of formula (IIC):

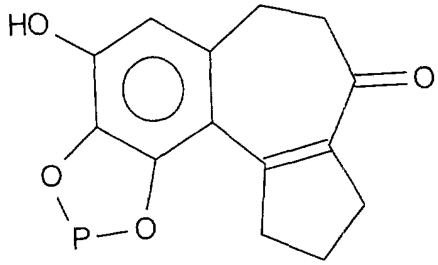

(IIC)

in which P represents the remainder of a diol protection reagent,
which is successively subjected to the action of a phenol protection reagent, a diol deprotection reagent, an alkylation agent then a phenol deprotection agent in order to obtain the compound of formula (IID) corresponding to the trisubstituted product of formula (II) with OH in position 8:

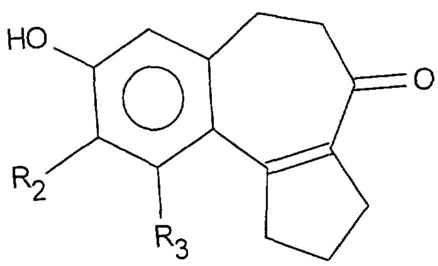

(IID)

or successively to the action of a phenol protection agent, an alkylation agent then a deprotection agent in order to obtain the compound of formula (IIE) corresponding to the trisubstituted product of formula (II) with OH in position 9

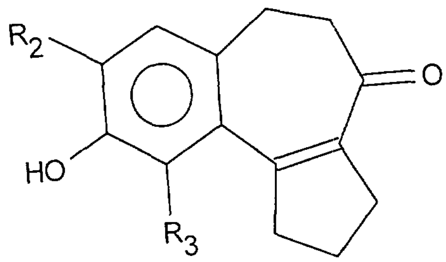

(IIE)

**19.** As a medicament, the compounds of formula (I) as defined in any one of claims 1 to 12, as well as their pharmaceutically acceptable addition salts and their esters.

**20.** As a medicament, the compounds of formula (I) as defined in claim 13.

**21.** The pharmaceutical compositions containing at least one of the medicaments defined in claim 19 or 20 as active ingredient.

**22.** As new intermediate products, the compounds of general formulae (IIIa), (IIIb), (IIIc) and (II) as defined in one of claims 14 to 18 it being understood that the compounds of formula (IIc) and the

- 2, 3, 5, 6-tetrahydro-9,10-dimethoxy-8-hydroxybenz[e]azulen-4(1H)-one,
- and the 2, 3, 5, 6-tetrahydro-8,9-dimethoxy-10-hydroxybenz[e]azulen-4(1H)-one
  compounds are excluded.